# EUROPEAN PATENT SPECIFICATION

(11) **EP 1 603 912 B1**
(45) Date of publication and mention of the grant of the patent: **18.07.2007**
(21) Application number: 04721899.5
(22) Date of filing: 19.03.2004
(51) Int. Cl.: C07D 471/10, C07D 311/24, C07D 405/14, C07D 401/12, C07D 401/14, A61K 31/438, A61P 3/04, A61P 15/00, A61P 25/22, A61P 25/24

(54) **SUBSTITUTED PIPERIDINE AND PIPERAZINE DERIVATIVES AS MELANOCORTIN-4 RECEPTOR MODULATORS**
SUBSTITUIERTE PIPERIDIN- UND PIPERAZIN-DERIVATE ALS MELANOCORTIN-4 REZEPTOR MODULATOREN
DERIVES A SUBSTITUTION PIPERIDINE ET PIPERAZINE AGISSANT COMME MODULATEURS DU RECEPTEUR DE LA MELANOCORTINE-4

(30) Priority: 20.03.2003 EP 03006254
(43) Date of publication of application: 14.12.2005
(73) Proprietor: Santhera Pharmaceuticals (Schweiz) AG, 4410 Liestal (CH)
(72) Inventor: SOEBERDT, Michael, 79618 Rheinfelden (DE); WEYERMANN, Philipp, CH-4450 Sissach (CH); VON SPRECHER, Andreas, CH-4104 Oberwil (CH)
(74) Representative: Grünecker, Kinkeldey, Stockmair & Schwanhäusser Anwaltssozietät
(86) International application number: PCT/EP2004/002907
(87) International publication number: WO 2004/083209

(56) References cited:
- WO-A-00/74679
- WO-A-01/70337
- WO-A-01/91752
- WO-A-99/64002
- WO-A-02/070511
- WO-A-03/009847

## Description

### Field of the Invention

The present invention relates to novel substituted piperidine and piperazine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these and other studies, it is evident that MC-1R is an important regulator of melanin production and coat color in animals and skin color in humans.

The MC-2R is expressed in the adrenal gland representing the ACTH receptor. The MC-2R is not a receptor for α -MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: a) the agouti (A^{vy}) mouse which ectopically expresses an antagonist of the MC-1R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats," Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study," J. Urol., 160: 389-393, 1998). Activation of melanocortin receptors of the brain appears to cause normal stimulation of sexual arousal. Evidence for the involvement of MC-R in male and/or female sexual dysfunction is detailed in WO/0074679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (Owen MJ and Nemeroff CB (1991) Physiology and pharmacology of corticotrophin releasing factor. Pharmacol Rev 43:425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl)ethyl]-4- [4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003)304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. et al. Role of the central melanocortin system in cachexia. Cancer Res. (2001) 61: 1432-1438).

WO03009847A and W003009850A describe phenylpiperazinyl-phenylalanine derivatives for the treatment of obesity. Most of the compounds in both patents contain a N-(2-piperidin-4-yl-phenyl)-alkyl, benzyl or aryl sulfonamide group and N-(2-piperazin-4-yl-phenyl)-alkyl, benzyl or aryl sulfonamide group, respectively. These compounds have in common the substituted phenylalanine moiety which is acylated with amino acids, carboxylic acids and sulfonyl chlorides. In some cases the phenylalanine moiety is also alkylated or uraes and urethanes are introduced. Biological data (e.g. binding IC₅₀ or functional activity) are not provided.

W002070511A describes phenylpiperazinyl-phenylalanine amides, phenylpiperidinyl-phenylalanine amides and cyclohexyl-phenylalanine amides as modulators of melanocortin receptors 1 and 4. The phenylalanine amino group is in the most cases acylated with a second amino acid. For amino acids with a basic side chain the amino group can be acylated. Biological data for the compounds are not given.

W002059108A describes melanocortin receptor agonists. The agonists consist of substituted phenylpiperazines which are first acylated with phenylalanines and then with amino acids. Biological data are not provided.

W00074679A describes substituted piperidines as melanocortin-4 receptor agonists. The piperidines are acylated with different substituted phenylalanines, e.g. D-p-chlorophenylalanine, which are subsequently acylated with other amino acids, in particular 1,2,3,4-tetrahydro-isoquinoline-3-carboxylic acid. Example 2 of this patent application binds to the human MC4-receptor with an IC₅₀ of 0.92 nM. The compound is acting as an agonist with an EC₅₀ of 2.1 nM (96% activation).

W09964002A describes spiroindane derivatives as melanocortin receptor agonists. The spiroindanes are acylated with phenylalanine, in particular p-chlorophenylalanine, which is then acylated with unsubstituted and substituted piperazine-2-carboxylic acid, morpholine-3-carboxylic acid and thiomorpholine-3-carboxylic acid, respectively. No biological data is given.

W00170337A describes spiroindane derivatives as melanocortin receptor agonists. The spiroindanes are acylated with phenylalanine, in particular p-chlorophenylalanine, which is then acylated with unsubstituted and substituted 1,2,3,4-tetrahydro-isoquinoline-3-carboxylic acid. No biological data is given.

W00191752A decribes melanocortin receptor agonists. Three examples are described consisting of 3a-benzyl-2-methyl-2,3a,4,5,6,7-hexahydro-pyrazolo[4,3-c]pyridin-3-one which is first acylated with Boc-D-4-chlorophenylalanine following a second acylation step using 1-amino-1,2,3,4-tetrahydro-naphthalene-2-carboxylic acid and 1,2,3,4-tetrahydro-isoquinoline-3-carboxylic acid, respectively. Biological data for the examples is not provided.

In view of the unresolved deficiencies in treatment of various diseases and disorders as discussed above, it is an object of the present invention to provide novel substituted piperidine and piperazine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to novel substituted piperidine and piperazine derivatives of structural formula (I), 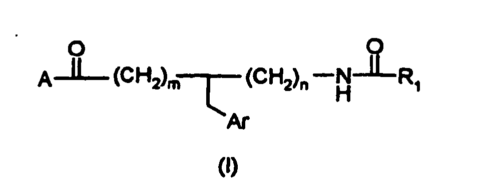 wherein the variables A, R₁, Ar, m and n have the meaning as defined below.

The disclosure also relates to novel substituted piperidine and piperazine derivatives of structural formula (II), 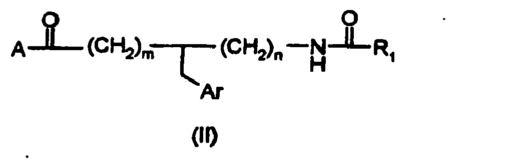 wherein the variables A, R₁, Ar, m and n have a different meaning than in structural formula (I) and are also defined below.

The piperidine and piperazine derivatives of structural formulas (I) and (II) are effective as melanocortin receptor modulators and are particularly effective as selective melanocortin-4 receptor (MC-4R) modulators. They are Therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to novel substituted piperidine and piperazine derivatives useful as melanocortin receptor modulators, in particular, selective MC-4R agonists and MC-4R antagonists.

The compounds of the present invention are represented by structural formula (I). 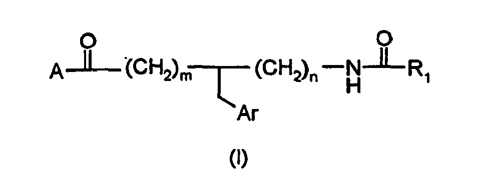 or a pharmaceutically acceptable salt or a solvate thereof, wherein
Ar is: aryl or heteroaryl which may both be substituted;
R₁ is: 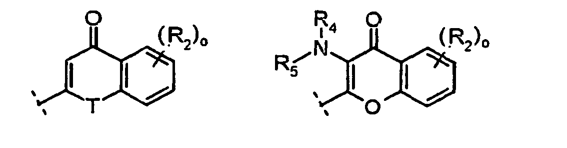
A is: 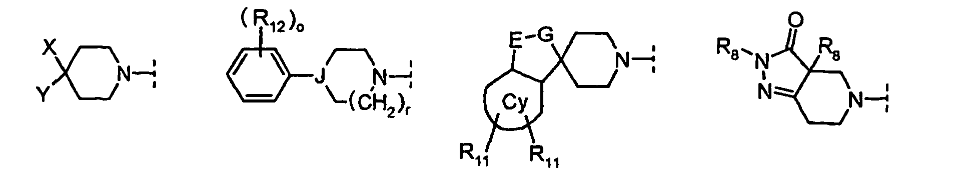
R₂ is independently:
   hydrogen,
   halo,
   alkyl,
   haloalkyl,
   hydroxy,
   alkoxy,
   S-alkyl,
   SO₂-alkyl,
   O-alkenyl,
   S-alkenyl,
   NR₁₄C(O)R₁₄,
   NR₁₄SO₂R₁₄,
   N(R₁₄)₂,
   (D)-cycloalkyl,
   (D)-aryl,
   (D)-heteroaryl,
   (D)-heterocyclyl (wherein heterocyclyl excludes a heterocyclyl containing a single nitrogen), and
   wherein aryl, heteroaryl, heterocyclyl, alkyl or cycloalkyl are substituted or unsubstituted, and two adjacent R₂ may form a 4- to 7-membered ring;
R₄ and R₅ are each independently:
   hydrogen,
   alkyl,
   (D)-cycloalkyl or
   R₄ and R₅ together with the nitrogen to which they are attached form a 5- to 8-membered ring,
   wherein alkyl and cycloalkyl are unsubstituted or substituted;
R₈ is independently:
   hydrogen,
   alkyl,
   (D)-aryl or
   (D)-cycloalkyl;
R₉ is independently:
   hydrogen,
   alkyl,
   (D)-aryl,
   (D)-heteroaryl or
   (D)-cycloalkyl;
R₁₀ is independently:
   R₉,
   (D)-heterocyclyl,
   (D)-N(Y)₂,
   (D)-NH-heteroaryl or
   (D)-NH-heterocyclyl,
   wherein aryl and heteroaryl, alkyl, D, cycloalkyl and heterocyclyl are substituted or unsubstituted, or
   two R₁₀ groups together with the atoms to which they are attached form a 5- to 8-membered mono- or bi-cyclic ring system;
R₁₁ is:
   hydrogen,
   halo,
   alkyl,
   alkoxy,
   C≡N,
   CF₃ or
   OCF₃;
R₁₂ is independently:
   hydrogen,
   hydroxy,
   cyano,
   nitro,
   halo,
   alkyl,
   alkoxy,
   haloalkyl,
   (D)-C(O)R₁₄,
   (D)-C(O)OR₁₄,
   (D)-C(O)SR₁₄,
   (D)-C(O)-heteroaryl,
   (D)-C(O)-heterocyclyl,
   (D)-C(O)N(R₁₄)₂,
   (D)-N(R₁₄)₂,
   (D)-NR₁₄COR₁₄,
   (D)-NR₁₄CON(R₁₄)₂,
   (D)-NR₁₄C(O)OR₁₄,
   (D)-NR₁₄C(R₁₄)=N(R₁₄),
   (D)-NR₁₄C(=NR₁₄)N(R₁₄)₂,
   (D)-NR₁₄SO₂R₁₄,
   (D)-NR₁₄SO₂N(R₁₄)2,
   (D)-NR₁₄(D)-heterocyclyl,
   (D)-NR₁₄(D)-heteroaryl.
   (D)-OR₁₄,
   OSO₂R₁₄,
   (D)[O]_{q}(cycloalkyl),
   (D)[O]_{q}(D)aryl,
   (D)[O]_{q}(D)-heteroaryl,
   (D)[O]_{q}(D)-heterocyclyl (wherein heterocyclyl excludes a heterocyclyl containing a single nitrogen when q=1),
   (D)-SR₁₄,
   (D)-SOR₁₄,
   (D)-SO₂R₁₄ or
   (D)-SO₂N(R₁₄)₂,
   wherein alkyl, alkoxy, cycloalkyl, aryl, heterocyclyl and heteroaryl are substituted or unsubstituted;
R₁₄ is independently:
   hydrogen,
   alkyl,
   haloalkyl,
   (D)-cycloalkyl,
   (D)-phenyl,
   (D)-naphthyl,
   (D)-heteroaryl,
   (D)-heterocyclyl (wherein heterocyclyl excludes a heterocyclyl containing a single nitrogen), and
   wherein phenyl, naphthyl, heteroaryl, heterocyclyl, alkyl or cycloalkyl is substituted or unsubstituted;
X is:
   alkyl,
   (D)-cycloallcyl,
   (D)-aryl,
   (D)-heteroaryl,
   (D)-heterocyclyl,
   (D)-C≡N,
   (D)-CON(R₉R₉),
   (D)-CO₂R₉,
   (D)-COR₉,
   (D)-NR₉C(O)R₉,
   (D)-NR₉CO₂R₉,
   (D)-NR₉C(O)N(R₉)₂,
   (D)-NR₉SO₂R₉,
   (D)-S(O)ₚR₉,
   (D)-SO₂N(R₉)(R₉),
   (D)-OR₉,
   (D)-OC(O)R₉,
   (D)-OC(O)OR₉,
   (D)-OC(O)N(R₉)₂,
   (D)-N(R₉)(R₉) or
   (D)-NR₉SO₂N(R₉)(R₉),
   wherein aryl, heteroaryl, alkyl, D, cycloalkyl and heterocyclyl are unsubstituted or substituted;
Y is:
   hydrogen,
   alkyl,
   (D)-cycloalkyl,
   (D)-aryl,
   (D)-heterocyclyl or
   (D)-heteroaryl,
   wherein aryl, heteroaryl, alkyl, D and cycloalkyl are unsubstituted or substituted;
Cy is benzene, pyridine or cyclohexane;
D is a bond or alkylen;
E is CHCO₂Y, CHC(O)N(Y)₂, NSO₂R₁₀, CHN(Y)COR₁₀, CHN(Y)SO₂R₁₀, CHCH₂OY or CHCH₂heteroaryl;
G is D, CH-alkyl, O, C=O or SO₂, with the proviso that when G is O, the ring atom E is carbon;
J is N or CH;
T is O or NR₄;
n is 0 - 2;
m is 0 - 2;
o is 0 - 3;
p is 0 - 2;
q is 0 or 1:
r is 1 or 2.

In the compounds of formula I, the variants have the following meanings:

Ar is as defined above, and is preferably aryl, more preferably phenyl or naphthyl. If aryl or heteroaryl are substituted, it is substituted with one to three, more preferably one or two, most preferably one, substituents. The substituents are preferably independently selected from the group consisting of cyano, nitro, perfluoroalkoxy, halo, alkyl, (D)-cycloalkyl, alkoxy and haloalkyl, more preferably perfluoroalkoxy, halo, alkyl, alkoxy or haloalkyl, even more preferably halo, alkyl, alkoxy and/or haloalkyl, in particular halo.

Most preferably, Ar is phenyl or naphthyl which both, preferably phenyl, may be substituted with one to three, in particular one, halo, e.g. Cl. The substitution can be in any position, preferably in the 4-position.

R₁ is as defined above preferably:

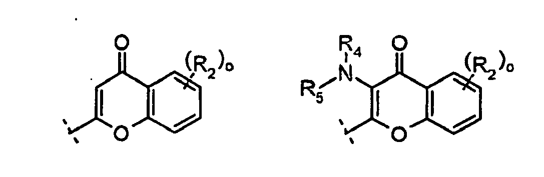

A is:

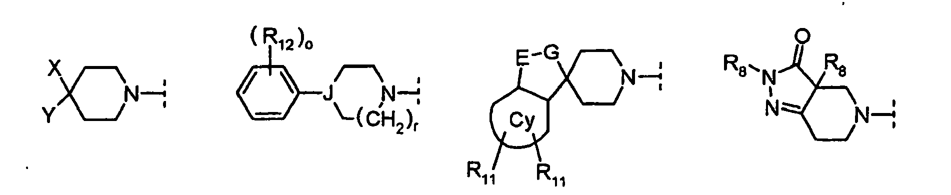

R₂ is as defined above. If aryl, heteroaryl, heterocyclyl, alkyl or cycloalkyl are substituted, they are preferably independently substituted with one to three, more preferably one substituent selected from the group consisting of oxo, halo, alkyl, N(R₆)₂, OR₆, SR₆ and/or CO₂R₆.

Preferably, R₂ is hydrogen, hydroxy, halo, alkyl, alkoxy, S-alkyl, SO₂-alkyl, O-alkenyl, S-alkenyl, haloalkyl or (D)-cycloalkyl, more preferably hydrogen, hydroxy, alkoxy, S-alkyl, SO₂-alkyl, O-alkenyl, S-alkenyl, halo or alkyl, e.g. methyl, ethyl, n-propyl, isopropyl. In one embodiment, R₂ is hydrogen, halo, alkyl, haloalkyl, alkoxy, (D)-cycloalkyl, (D)-aryl, (D)-heteroaryl, (D)-heterocyclyl (wherein heterocyclyl excludes a heterocyclyl containing a single nitrogen), and wherein aryl, heteroaryl, heterocyclyl, alkyl and cycloalkyl are substituted or unsubstituted; preferably hydrogen, hydroxy, halo, alkyl, alkoxy, haloalkyl or (D)-cycloalkyl, more preferably hydrogen, alkoxy, halo or alkyl.

R₄ and R₅ are each independently as defined above. When R₄ and R₅ form a ring, said ring may contain an additional heteroatom preferably selected from O, S and NR₆ in the ring. Moreover, if alkyl and cycloalkyl are substituted, they are preferably substituted with one to three, more preferably one or two groups independently selected from R₇ and oxo.

R₄ and R₅ are each independently preferably selected from the group consisting of hydrogen, alkyl or cycloalkyl; or R₄ and R₅ together with the nitrogen to which they are attached form a 5- to 7-membered ring. More preferably R₄ and R₅ are each independently selected from the group consisting of hydrogen or alkyl, or R₄ and R₅ together with the nitrogen to which they are attached form a 5- to 6-membered ring optionally containing an additional oxygen atom.

R₆ is independently hydrogen, alkyl, C(O) alkyl, (D)-aryl or (D)-cycloalkyl, preferably hydrogen, alkyl, C(O) alkyl, (D)-aryl or (D)-cycloalkyl, most preferably hydrogen.

R₇ is alkyl, (D)-aryl, (D)-cycloalkyl, (D)-heteroaryl, halo, OR₈, NHSO₂R₈, N(R₈)₂, C≡N, CO₂R₄, C(R₈)(R₈)N(R₈)₂, nitro, SO₂N(R₈)₂, S(O)ₚR₈, CF₃ or OCF₃, preferably hydrogen, alkyl, (D)-aryl or (D)-cycloalkyl, more preferably hydrogen.

R₈ is as defined above, preferably hydrogen, alkyl or (D)-aryl, more preferably alkyl or (D)-aryl.

R₉ is as defined above, preferably hydrogen, alkyl and (D)-cycloalkyl, more preferably alkyl, most preferably methyl, ethyl or tert-butyl.

R₁₀ is as defined above. If aryl and heteroaryl are substituted, they are preferably independently substituted with one to three groups selected from R₇. Moreover, if alkyl, D, cycloalkyl and heterocyclyl are substituted, they are preferably substituted with one to four groups independently selected from R₇ and oxo. If two R₁₀ groups together with the atoms to which they are attached form a 5- to 8-membered mono- or bi-cyclic ring system, said ring may contain an additional heteroatom preferably selected from O, S, NR₈, NBoc and NZ.

Preferably, R₁₀ is R₉.

R₁₁ is defined as above, preferably hydrogen, halo, alkyl, alkoxy or C≡N, more preferably hydrogen, halo or C₁ - C₄-alkyl, most preferably hydrogen.

R₁₂ is as defined above. Moreover, if alkyl, alkoxy, cycloalkyl, aryl, heterocyclyl and heteroaryl are substituted, they are preferably substituted with 1 to 5, more preferably 1 to 3, most preferably 1 or 2 substituents independently selected from R₁₃.

Preferably, R₁₂ is hydrogen, hydroxy, cyano, nitro, halo, alkyl, alkoxy, haloalkyl, (D)-C(O)-heterocyclyl, (D)-C(O)N(R₁₄)₂, (D)-N(R₁₄)₂, (D)-NR₁₄COR₁₄, (D)-NR₁₄CON(R₁₄)₂, (D)-NR₁₄C(O)OR₁₄, (D)-NR₁₄C(R₁₄)=N(R₁₄), (D)-NR₁₄C(=NR₁₄)N(R₁₄)₂, (D)-NR₁₄SO₂R₁₄ or (D)-NR₁₄SO₂N(R₁₄)₂, wherein alkyl or alkoxy are substituted or unsubstituted with one to five, preferably one to three, substituents selected from R₁₃. More preferably, R₁₂ is cyano, nitro, halo, alkyl, D)-C(O)-heterocyclyl, (D)-N(R₁₄)₂ (D)-NR₁₄COR₁₄, (D)-NR₁₄CON(R₁₄)₂, (D)-NR₁₄C(O)OR₁₄ or (D)-NR₁₄SO₂R₁₄. Most preferably, R₁₂ is (D)-C(O)-heterocyclyl, (D)-C(O)N(R₁₄)₂. Halo is preferably F, Cl or Br. R₁₂ can be on any position of the ring, preferably in the 1-position. In one embodiment, R₁₂ is hydrogen, hydroxy, cyano, nitro, halo, alkyl, alkoxy, haloalkyl, (D)-N(R₁₄)₂, (D)-NR₁₄COR₁₄, (D)-NR₁₄CON(R₁₄)₂, (D)-NR₁₄C(O)OR₁₄, (D)-NR₁₄C(R₁₄)=N(R₁₄), (D)-NR₁₄C(=NR₁₄)N(R₁₄)₂, (D)-NR₁₄SO₂R₁₄ or (D)-NR₁₄SO₂N(R₁₄)₂, wherein alkyl or alkoxy are substituted or unsubstituted with one to five, preferably one to three, substituents selected from R₁₃. More preferably, R₁₂ is cyano, nitro, halo, alkyl, (D)-N(R₁₄)₂ (D)-NR₁₄COR₁₄, (D)-NR₁₄CON(R₁₄)₂, (D)-NR₁₄C(O)OR₁₄ or (D)-NR₁₄SO₂R₁₄.

R₁₃ is independently hydrogen, halo, oxo, N(R₁₅)₂, alkyl, (D)-cycloalkyl, haloalkyl, alkoxy, heteroaryl, hydroxy or heterocyclyl, wherein heterocyclyl excludes a heterocyclyl containing a single nitrogen, phenyl, (D)-COR₁₄, (D)-C(O)OR₁₄, (D)-OR₁₄, (D)-OCOR₁₄, (D)-OCO₂R₁₄, (D)-SR₁₄, (D)-SOR₁₄ or (D)-SO₂R₁₄, wherein aryl, heteroaryl, heterocyclyl, alkyl or cycloalkyl is substituted or unsubstituted. If aryl, heteroaryl, heterocyclyl, alkyl or cycloalkyl are substituted, they are preferably substituted with one to three, preferably one or two, substituents selected from the group consisting of oxo, alkyl, N(R₁₅)₂, OR₁₅, SR₁₅ and/or CO₂R₁₅.

Preferably, R₁₃ is hydrogen, halo, alkyl, (D)-cycloalkyl, alkoxy or phenyl, more preferably R₁₃ is hydrogen, halo, alkyl, alkoxy or phenyl.

R₁₄ is as defined above. If aryl, heteroaryl, heterocyclyl, alkyl or cycloalkyl are substituted, they are preferably substituted with one to three, more preferably one or two, substituents selected from the group consisting of oxo, halo, alkyl, N(R₁₅)₂, OR₁₅, SR₁₅ and/or CO₂R₁₅.

Preferably, R₁₄ is hydrogen, haloalkyl, alkyl, (D)-cycloalkyl or phenyl, more preferably R₁₄ is hydrogen, haloalkyl, alkyl or phenyl.

R₁₅ is independently hydrogen, alkyl, C(O)alkyl, aryl or cycloalkyl, preferably hydrogen, alkyl or cycloalkyl, in particular, hydrogen.

X is as defined above. If aryl and heteroaryl are substituted, they are preferably substituted with one to three, more preferably one or two, groups selected from R₇. Moreover, if alkyl, D, cycloalkyl and heterocyclyl are substituted, they are preferably substituted with one to four groups independently selected from R₇ and oxo.

Preferably, X is alkyl, (D)-cycloalkyl, (D)-aryl, (D)-heteroaryl, (D)-heterocyclyl, (D)-NHC(O)R₉, (D)-CO₂R₉ or (D)-CON(R₉R₉), more preferably from alkyl, (D)-cycloalkyl, (D)-heterocyclyl, (D)-NHC(O)R₉ or (D)-CON(R₉R₉), most preferably from C₁ - C₄-alkyl, C₅ - C₇-cycloalkyl, (D)-CON(R₉R₉) and N-containing heterocyclyl, in particular triazolyl and tetrazolyl.

Y is as defined above. If aryl and heteroaryl are substituted, they are preferably substituted with one to three, more preferably one or two, groups selected from R₇. Moreoverif alkyl, D and cycloalkyl are substituted, they are preferably substituted with one to three groups selected from R₇ and oxo.

Preferably, Y is hydrogen, alkyl, (D)-cycloalkyl, (D)-aryl, (D)-heteroaryl or (D)-heterocyclyl, more preferably hydrogen, alkyl, (D)-cycloalkyl or (D)-heterocyclyl, most preferably hydrogen, C₁ - C₄-alkyl or C₅ - C₇-cycloalkyl, in particular cyclohexyl.

Cy is as defined above, preferably benzene or pyridine, more preferably benzene.

D is as defined above, preferably a bond or C₁- C₄-alkylene, more preferably a bond or CH₂. E is as defined above, preferably NSO₂R₁₀, CHN(Y)COR₁₀ or CHN(Y)SO₂R₁₀, more preferably NSO₂R₁₀.

G is as defined above, preferably D or CH-alkyl, more preferably D, in particular CH₂.
J is N or CH;
T is O or NR₄, preferably O; in one embodiment, T is NR₄;
n is 0, 1 or 2, preferably 0 or 1, more preferably 0;
m is 0, 1 or 2, preferably 0 or 1, more preferably 0;
o is 0, 1, 2 or 3, preferably 0, 1 or 2, more preferably 0 or 1;
p is 0, 1 or 2;
q is 0 or 1;
r is 1 or 2, preferably 1.

Compounds of the present disclosure are also represented by structural formula (II), 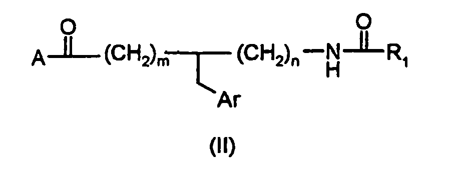 or a pharmaceutically acceptable salt or a solvate thereof, wherein
Ar is:
   aryl or heteroaryl, which may both be substituted;
R₁ is: 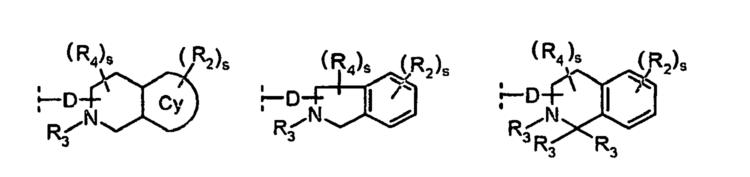 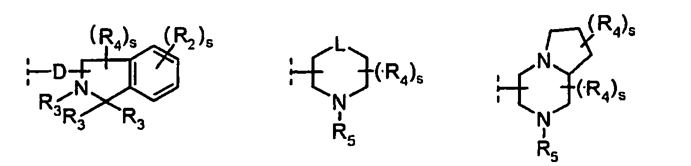 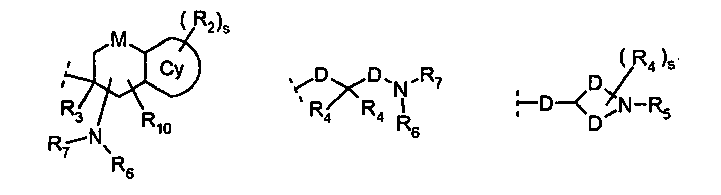
A is: 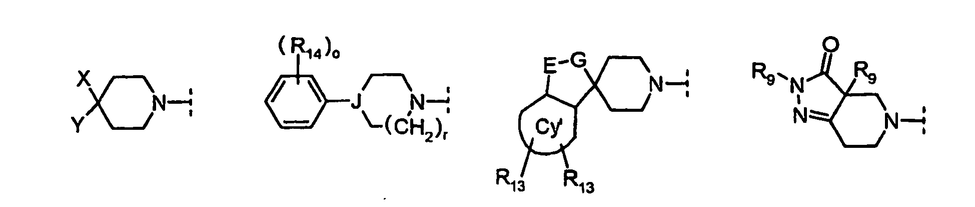
R₂ is independently:
   hydrogen,
   halo,
   alkyl,
   haloalkyl,
   alkoxy,
   (D)-cycloalkyl,
   (D)-aryl,
   (D)-heteroaryl,
   (D)-heterocyclyl (wherein heterocyclyl excludes a heterocyclyl containing a single nitrogen), and
   wherein aryl, heteroaryl, heterocyclyl, alkyl or cycloalkyl are substituted or unsubstituted.
R₃ is independently:
   hydrogen,
   alkyl,
   SO₂alkyl,
   SO₂aryl,
   C(O)alkyl,
   (D)-aryl or
   cycloalkyl;
R₄ is independently:
   hydrogen,
   alkyl,
   (D)-aryl,
   (D)-heteroaryl,
   (D)-N(R₆)₂,
   (D)-NR₆C(O)alkyl,
   (D)-NR₆SO₂alkyl,
   (D)-SO₂N(R₆)₂,
   (D)-(O)ᵥalkyl,
   (D)-(O)ᵥ(D)NR₆COR₆,
   (D)-(O)ᵥ(D)NR₇SO₂R₇,
   (D)-(O)ᵥ-heterocyclyl or
   (D)-(O)ᵥ(alkyl)-heterocyclyl;
R₅ is independently:
   hydrogen,
   alkyl,
   (D)-phenyl,
   C(O)alkyl,
   C(O)phenyl,
   SO₂-alkyl or
   SO₂-phenyl;
R₆ and R₇ are each independently:
   hydrogen,
   alkyl,
   cycloalkyl, or
   R₆ and R₇ together with the nitrogen to which they are attached form a 5- to 8-membered ring,
   wherein alkyl and cycloalkyl are unsubstituted orsubstituted;
R₉ is independently:
   hydrogen,
   alkyl,
   (D)-aryl or
   cycloalkyl;
R₁₀ is hydrogen or alkyl;
R₁₁ is independently:
   hydrogen,
   alkyl,
   (D)-aryl,
   (D)-heteroaryl or
   (D)-cycloalkyl;
R₁₂ is independently:
   R₁₁,
   (D)-heterocyclyl,
   (D)-N(Y)₂,
   (D)-NH-heteroaryl or
   (D)-NH-heterocyclyl,
   wherein aryl, heteroaryl, alkyl, D, cycloalkyl and heterocyclyl are unsubstituted or substituted, or
   two R₁₂ groups together with the atoms to which they are attached form a 5- to 8-membered mono- or bicyclic ring system;
R₁₃ is:
   hydrogen,
   halo,
   alkyl,
   alkoxy,
   C≡N,
   CF₃ or
   OCF₃;
R₁₄ is independently:
   hydrogen,
   hydroxy,
   cyano,
   nitro,
   halo,
   alkyl,
   alkoxy,
   haloalkyl,
   (D)-C(O)R₁₆,
   (D)-C(O)OR₁₆,
   (D)-C(O)SR₁₆,
   (D)-C(O)-heteroaryl,
   (D)-C(O)-heterocyclyl,
   (D)-C(O)N(R₁₆)₂,
   (D)-N(R₁₆)₂,
   (D)-NR₁₆COR₁₆,
   (D)-NR₁₆CON(R₁₆)₂,
   (D)-NR₁₆C(O)OR₁₆,
   (D)-NR₁₆C(R₁₆)=N(R₁₆),
   (D)-NR₁₆C(=NR₁₆)N(R₁₆)₂,
   (D)-NR₁₆SO₂R₁₆,
   (D)-NR₁₆SO₂N(R₁₆)₂,
   (D)-NR₁₆(D)-heterocyclyl,
   (D)-NR₁₆(D)-heteroaryl,
   (D)-OR₁₆,
   OSO₂R₁₆,
   (D)-[O]_{q}(cycloalkyl),
   (D)-[O]_{q}(D)aryl,
   (D)-[O]_{q}(D)-heteroaryl,
   (D)-[O]_{q}(D)-heterocyclyl (wherein heterocyclyl excludes a heterocyclyl containing a single nitrogen when q=1),
   (D)-SR₁₆,
   (D)-SOR₁₆,
   (D)-SO₂R₁₆ or
   (D)-SO₂N(R₁₆)₂,
   wherein alkyl, alkoxy, cycloalkyl, aryl, heterocyclyl and heteroaryl are substituted or unsubstituted;
R₁₆ is independently:
   hydrogen,
   alkyl,
   haloalkyl,
   (D)-cycloalkyl,
   (D)-aryl,
   (D)-heteroaryl,
   (D)-heterocyclyl (wherein heterocyclyl excludes a heterocyclyl containing a single nitrogen), and
   wherein aryl, heteroaryl, heterocyclyl, alkyl or cycloalkyl is substituted or unsubstituted;
Cy is:
   aryl,
   heteroaryl,
   heterocyclyl or
   carbocyclyl;
Xis:
   alkyl,
   (D)-cycloalkyl,
   (D)-aryl,
   (D)-heteroaryl,
   (D)-heterocyclyl,
   (D)-C≡N,
   (D)-CON(R₁₁R₁₁),
   (D)-CO₂R₁₁,
   (D)-COR₁₁,
   (D)-NR₁₁C(O)R₁₁,
   (D)-NR₁₁CO₂R₁₁,
   (D)-NR₁₁C(O)N(R₁₁)₂,
   (D)-NR₁₁SO₂R₁₁,
   (D)-S(O)ₚR₁₁,
   (D)-SO₂N(R₁₁)(R₁₁),
   (D)-OR₁₁,
   (D)-OC(O)R₁₁,
   (D)-OC(O)OR₁₁,
   (D)-OC(O)N(R₁₁)₂,
   (D)-N(R₁₁)(R₁₁) or
   (D)-NR₁₁SO₂N(R₁₁)(R₁₁),
   wherein aryl, heteroaryl, alkyl, D, cycloalkyl and heterocyclyl are unsubstituted or substituted;
V is:
   hydrogen,
   alkyl,
   (D)-cycloalkyl,
   (D)-aryl,
   (D)-heterocyclyl or
   (D)-heteroaryl,
   wherein aryl, heteroaryl, alkyl, D and cycloalkyl are unsubstituted or substituted;
Cy' is benzene, pyridine or cyclohexane;
D is a bond or alkylene;
E is CHCO₂Y, CHC(O)N(Y)₂, NSO₂R₁₀, CHN(Y)COR₁₂, CHN(Y)SO₂R₁₂, CHCH₂OY or CHCH₂heteroaryl;
G is D, CH-alkyl, O, C=O or SO₂, with the proviso that when G is O, the ring atom E is carbon;
J is N or CH;
L is O, S or NR₅;
M is bond, O, S(O)ᵤ, NR₅ or CH₂;
n is 0 - 2, unless m is 0, then n is 1 or 2;
m is 0 - 2, unless n is 0, then m is 1 or 2;
o is 0 - 3;
p is 0 - 2;
q is 0 or 1;
r is 1 or 2;
s is 0 - 5;
u is 0 - 2;
v is 0 or 1.

In preferred embodiments of formula II, the variants have the following meanings:

Ar is as defined above, and is preferably aryl, more preferably phenyl or naphthyl. If aryl or heteroaryl are substituted, it is preferably substituted with one to three, more preferably one or two, most preferably one, substituents. The substituents are preferably independently selected from the group consisting of: cyano, nitro, perfluoroalkoxy, halo, alkyl, (D)-cycloalkyl, alkoxy and haloalkyl, more preferably cyano, perfluoroalkoxy, halo, alkyl, (D)-cycloalkyl, alkoxy or haloalkyl, even more preferably halo, alkyl, alkoxy and/or haloalkyl, in particular halo.

Most preferably, Ar is phenyl or naphthyl which both, preferably phenyl, may be substituted with one to three, in particular one, halo, e.g. Cl. The substitution can be in any position, preferably in the 4-position.

R₁ is as defined above, preferably:

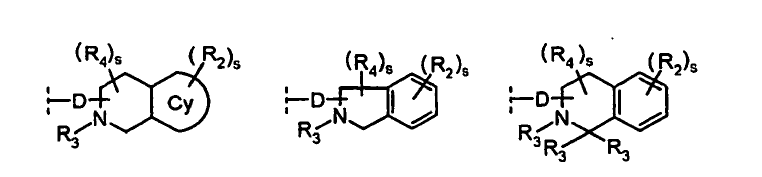

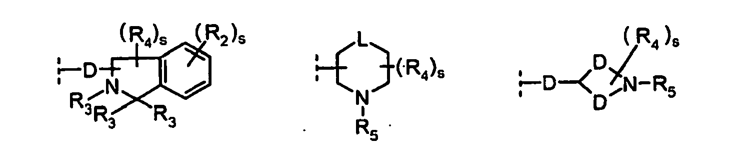

More preferably, R₁ is:

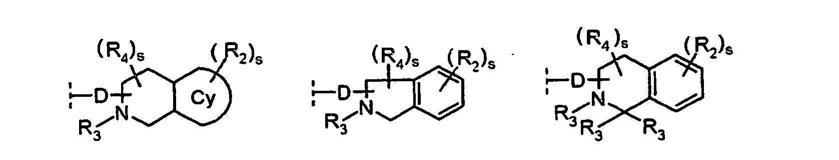

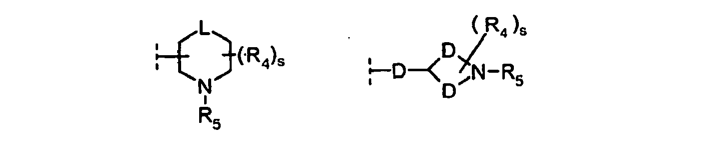

A is as defined above.

R₂ is as defined above. If aryl, heteroaryl, heterocyclyl, alkyl or cycloalkyl are substituted, they are preferably substituted with one to three substituents, more preferably one, selected from the group consisting of oxo, alkyl, N(R₃)₂, OR₃, SR₃ and/or CO₂R₃.

Preferably, R₂ is hydrogen, halo, alkyl, haloalkyl, alkoxy or (D)-cycloalkyl, more preferably hydrogen, halo or alkyl, e.g. methyl, ethyl, n-propyl, iso-propyl, most preferably hydrogen.

R₃ is as defined above, preferably hydrogen or alkyl, e.g. methyl, ethyl, n-propyl, iso-propyl, more preferably hydrogen.

R₄ is as defined above, preferably hydrogen, (D)-aryl, (D)-heteroaryl, (D)-N(R₆)₂, (D)-NR₆C(O)alkyl, (D)-NR₆SO₂alkyl or alkyl, e.g. methyl, ethyl, n-propyl, iso-propyl, more preferably hydrogen.

R₅ is as defined above, preferably hydrogen or alkyl, e.g. methyl, ethyl, n-propyl, iso-propyl, more preferably hydrogen.

R₆ and R₇ are each independently as defined above. When R₆ and R₇ form a ring, said ring may contain an additional heteroatom preferably selected from O, S and NR₃ in the ring. Moreover, if alkyl and cycloalkyl are substituted, they are preferably substituted with one to three, more preferably one or two, groups independently selected from R₈ and/or oxo. R₆ and R₇ are each independently preferably selected from the group consisting of hydrogen, alkyl or cycloalkyl, or R₆ and R₇ together with the nitrogen to which they are attached form a 5- to 7-membered ring. More preferably R₆ and R₇ are each independently selected from the group consisting of hydrogen or alkyl, or R₆ and R₇ together with the nitrogen to which they are attached form a 5- to 6-membered ring optionally containing an additional oxygen atom.

R₈ is alkyl, (D)-aryl, (D)-cycloalkyl, (D)-heteroaryl, halo, OR₉, NHSO₂R₉, N(R₉)₂, C≡N, CO₂R₆, C(R₉)(R₉)N(R₉)₂, nitro, SO₂N(R₉)₂, S(O)ᵤR₉, CF₃ or OCF₃. Preferably R₈ is alkyl, OR₉, (D)-aryl, (D)-cycloalkyl, (D)-heteroaryl or halo.

R₉ is as defined above, preferably hydrogen, (D)-aryl or alkyl, e.g. methyl, ethyl, n-propyl, iso-propyl, more preferably hydrogen or (D)-aryl.

R₁₀ is as defined above, preferably hydrogen or C₁ - C₄ alkyl as defined below, more preferaby hydrogen, methyl or ethyl, most preferably hydrogen or methyl.

R₁₁ is as defined above, preferably hydrogen or alkyl, more preferably hydrogen or C₁ - C₆ alkyl as defined below, in particular C₁ - C₅ alkyl, e.g. methyl, ethyl, n-propyl, iso-propyl, n-butyl, iso-butyl, tert-butyl and n-pentyl, most preferably hydrogen or tert-butyl.

R₁₂ is as defined above. If aryl and heteroaryl are substituted, they are preferably substituted with one to three, more preferably one or two, groups selected from R₇. Moreover, if alkyl, D, cycloalkyl and heterocyclyl are substituted, they are preferably substituted with one to four, more preferably one or two, groups independently selected from R₈ and/or oxo. Furthermore, if two R₁₂ groups together with the atoms to which they are attached form a 5- to 8-membered mono- or bi-cyclic ring system, said ring may contain an additional heteroatom preferably selected from O, S, NR₉, NBoc and NZ.

Preferably, R₁₂ is R₁₁.

R₁₃ is as defined above, preferably hydrogen, halo, alkyl, alkoxy and C=N, more preferably hydrogen, methyl or ethyl, most preferably hydrogen or methyl.

R₁₄ is as defined above. Moreover, if alkyl, alkoxy, cycloalkyl, aryl, heterocyclyl and heteroaryl are substituted, they are preferably substituted with 1 to 5, more preferably 1 to 3, most preferably 1 or 2, substituents independently selected from R₁₅.

Preferably, R₁₄ is hydrogen, hydroxy, cyano, nitro, halo, alkyl, alkoxy, haloalkyl, (D)-C(O)-heterocyclyl, (D)-C(O)N(R₁₆)₂, (D)-N(R₁₆)₂, (D)-NR₁₆COR₁₄, (D)-NR₁₄CON(R₁₄)₂, (D)-NR₁₄C(O)OR₁₆, (D)-NR₁₆C(R₁₆)=N(R₁₆), (D)-NR₁₆C(=NR₁₆)N(R₁₆)₂, (D)-NR₁₆SO₂R₁₆ or (D)-NR₁₆SO₂N(R₁₆)₂, wherein alkyl or alkoxy are substituted or unsubstituted with one to five, preferably one to three, substituents selected from R₁₅. More preferably, R₁₄ is cyano, nitro, halo, alkyl, (D)-C(O)-heterocyclyl, (D)-C(O)N(R₁₆)₂, (D)-N(R₁₆)₂, (D)-NR₁₆COR₁₆, (D)-NR₁₆CON(R₁₆)₂, (D)-NR₁₆C(O)OR₁₆ or (D)-NR₁₆SO₂R₁₆. Most preferably, R₁₆ is (D)-C(O)-heterocyclyl, (D)-C(O)N(R₁₆)₂ or NR₁₆COR₁₆. Halo is preferably F, Cl or Br. R₁₄ can be on any position of the ring, preferably in the 1-position. In one embodiment, R₁₄ is hydrogen, hydroxy, cyano, nitro, halo, alkyl, alkoxy, haloalkyl, (D)-N(R₁₆)₂, (D)-NR₁₆COR₁₄, (D)-NR₁₄CON(R₁₄)₂, (D)-NR₁₄C(O)OR₁₆, (D)-NR₁₆C(R₁₆)=N(R₁₆), (D)-NR₁₆C(=NR₁₆)N(R₁₆)₂, (D)-NR₁₆SO₂R₁₆ or (D)-NR₁₆SO₂N(R₁₆)₂, wherein alkyl or alkoxy are substituted or unsubstituted with one to five, preferably one to three, substituents selected from R₁₅. More preferably, R₁₄ is cyano, nitro, halo, alkyl, (D)-N(R₁₆)₂, (D)-NR₁₆COR₁₆, (D)-NR₁₆CON(R₁₆)₂, (D)-NR₁₆C(O)OR₁₆ or (D)-NR₁₆SO₂R₁₆.

R₁₅ is independently hydrogen, halo, oxo, N(R₃)₂, alkyl, (D)-cycloalkyl, alkoxy, haloalkyl, heteroaryl, hydroxy, heterocyclyl, wherein heterocyclyl excludes a heterocyclyl containing a single nitrogen, phenyl, (D)-COR₁₆, (D)-C(O)OR₁₆, (D)-OR₁₆, (D)-OCOR₁₆, (D)-OCO₂R₁₆, (D)-SR₁₆, (D)-SOR₁₆ or (D)-SO₂R₁₆, wherein aryl, heteroaryl, heterocyclyl, alkyl and cycloalkyl are substituted or unsubstituted. If aryl, heteroaryl, heterocyclyl, alkyl or cycloalkyl are substituted, they are preferably substituted with one to three, more preferably one or two, substituents selected from the group consisting of oxo, alkyl, N(R₁₆)₂, OR₁₆, SR₁₆ and/or CO₂R₁₆.

Preferably, R₁₅ is hydrogen, halo, alkyl, (D)-cycloalkyl, alkoxy or phenyl, more preferably R₁₅ is hydrogen, halo, alkyl, alkoxy or phenyl.

R₁₆ is as defined above. If aryl, heteroaryl, heterocyclyl, alkyl or cycloalkyl are substituted, they are preferably substituted with one to three, more preferably one or two, substituents selected from the group consisting of oxo, alkyl, N(R₃)₂, OR₃, SR₃ and/or CO₂R₃.

Preferably, R₁₆ is hydrogen, halo, alkyl, (D)-cycloalkyl, alkoxy or phenyl, more preferably R₁₆ is hydrogen, halo, alkyl, alkoxy or phenyl.

Cy is as defined above wherein heteroaryl and heterocyclyl are preferably 5- or 6-membered and carbocyclyl is preferably 5- or 7-membered. Preferably Cy is aryl, more preferably benzene.

X is as defined above. If aryl and heteroaryl are substituted, they are preferably substituted with one to three, more preferably one or two, groups selected from R₈. Moreover, if alkyl, D, cycloalkyl and heterocyclyl are substituted, they are preferably substituted with one to four groups independently selected from R₈ and/or oxo.

Preferably, X is alkyl, (D)-cycloalkyl, (D)-aryl, (D)-heteroaryl, (D)-heterocyclyl, (D)-NHC(O)R₁₁, (D)-CO₂R₁₁ or (D)-CON(R₁₁R₁₁), more preferably alkyl, (D)-cycloalkyl, (D)-heterocyclyl, (D)-NHC(O)R₁₁ or (D)-CON(R₁₁R₁₁), most preferably C₁ - C₄-alkyl, C₅ - C₇-cycloalkyl, (D)-CON(R₁₁R₁₁) and N-containing heterocyclyl, in particular triazolyl and tetrazolyl.

Y is as defined above. If aryl and heteroaryl are substituted, they are preferably substituted with one to three, more preferably one or two, groups selected from R₈. Moreover, if alkyl, D and cycloalkyl are substituted, they are preferably substituted with one to three groups selected from R₈ and/or oxo.

Preferably, Y is hydrogen, alkyl, (D)-cycloalkyl, (D)-aryl, (D)-heteroaryl or (D)-heterocyclyl, more preferably alkyl, (D)-cycloalkyl or (D)-heterocyclyl, most preferably hydrogen, C₁ - C₄-alkyl and C₅ - C₇-cycloalkyl, in particular cyclohexyl.

Cy' is as defined above, preferably benzene or pyridine, more preferably benzene.

D is as defined above, preferably a bond or C₁ - C₄ alkylene, more preferably a bond or CH₂.

E is as defined above, preferably NSO₂R₁₀, CHN(Y)COR₁₂ or CHN(Y)SO₂R₁₂, more preferably NSO₂R₁₀.

G is as defined above, preferably D or CH alkyl, more preferably D, in particular CH₂.
J is as defined above;
L is as defined above, preferably NR₅;
M is as defined above, preferably bond or CH₂;
n is 0, 1 or 2, preferably 0 or 1, unless m is 0, then n is 1;
m is 0, 1 or 2, preferably 0 or 1, unless n is 0, then m is 1, more preferably n+m=1;
o is 0, 1, 2 or 3, preferably 0, 1 or 2, more preferably 1;
p is 0 or 1, preferably 0;
q is 0 or 1, preferably 0;
r is 1 or 2, preferably 1;
s is 0, 1, 2, 3, 4 or 5, preferably 0, 1, 2 or 3, more preferably 0 or 1, most preferably 0;
u is 0, 1 or 2;
v is 0 or 1.

In the above and the following, the employed terms have the meaning as described below:

Aryl is an aromatic mono- or polycyclic moiety with 6 to 20 carbon atoms which is preferably selected from phenyl, biphenyl, naphthyl, tetrahydronaphthyl, fluorenyl, indenyl or phenanthrenyl, more preferably from phenyl or naphthyl.

Heteroaryl is an aromatic moiety having 6 to 20 carbon atoms with at least one heterocycle and is preferably selected from thienyl, benzothienyl, naphthothienyl, furanyl, benzofuranyl, chromenyl, indolyl, isoindolyl, indazolyl, quinolyl, isoquinolyl, phthalazinyl, quinoxalinyl, cinnolinyl or quinazolinyl, more preferably from thienyl, furanyl, benzothienyl, benzofuranyl or indolyl.

Heterocyclyl is a saturated, unsaturated or aromatic ring containing at least one heteroatom selected from O, N and/or S and 1 to 6 carbon atoms and is preferably selected from azetidin-2-one-1-yl, pyrrolidin-2-one-1-yl, piperid-2-one-1-yl and azepan-2-one-1-yl, thienyl, furyl, piperidinyl, pyranyl, pyrrolyl, imidazolyl, pyrazolyl, pyridyl, pyrazinyl, pyrimidinyl, pyridazinyl, isothiazolyl or isoxazyl, more preferably from pyridyl, piperidinyl, imidazolyl or pyrazinyl.

Carbocyclyl is a monocyclic or polycyclic ring system of 3 to 20 carbon atoms which may be saturated, unsaturated or aromatic.

Alkyl is straight chain or branched alkyl having preferably 1 to 8 carbon atoms, such as methyl, ethyl, n-propyl, isopropyl, n-butyl, isobutyl, tert-butyl, n-pentyl, isopentyl, neopentyl, hexyl or heptyl, more preferably 1 to 4 carbon atoms.

Cycloalkyl is an alkyl ring having preferably 3 to 8 carbon atoms, such as cyclopropyl, cyclobutyl, cyclopentyl, cyclohexyl, cycloheptyl or cyclooctyl, more preferably 3 to 6 carbon atoms.

Alkenyl is straight chain or branched alkenyl having preferably 2 to 8 carbon atoms such as vinyl, allyl, methallyl, buten-2-yl, buten-3-yl, penten-2-yl, penten-3-yl, penten-4-yl, 3-methyl-but-3-enyl, 2-methyl-but-3-enyl, 1-methyl-but-3-enyl, hexenyl or heptenyl, more preferably 2 to 4 atoms.

Alkoxy is O-alkyl wherein alkyl is as defined above and has preferably 1 to 4 carbon atoms, preferably 1 or 3 carbon atoms.

Halo or halogen is a halogen atom preferably selected from F, Cl, Br and I, preferably F, Cl and Br.

Haloalkyl is an alkyl moiety as defined above having preferably 1 to 4 carbon atoms, more preferably 1 or 2 carbon atoms, wherein at least one, preferably 1 to 3 hydrogen atoms, have been replaced by a halogen atom. Preferred examples are -CF₃, -CH₂CF₃ and -CF₂CF₃.

Therein, the alkylene moiety may be a straight chain or branched chain group. Said alkylene moiety preferably has 1 to 6 carbon atoms. Examples thereof include methylene, ethylene, n-propylene, n-butylene, n-pentylene, n-hexylene, iso-propylene, sec.-butylene, tert.-butylene, 1,1-dimethyl propylene, 1,2-dimethyl propylene, 2,2-dimethyl propylene, 1,1-dimethyl butylene, 1,2-dimethyl butylene, 1,3-dimethyl butylene, 2,2-dimethyl butylene, 2,3-dimethyl butylene, 3,3-dimethyl butylene, 1-ethyl butylene, 2-ethyl butylene, 3-ethyl butylene, 1-n-propyl propylene, 2-n-propyl propylene, 1-iso-propyl propylene, 2-iso-propyl propylene, 1-methyl pentylene, 2-methyl pentylene, 3-methyl pentylene and 4-methyl pentylene. More preferably, said alkylene moiety has 1 to 3 carbon atoms, such as methylene, ethylene, n-propylene and iso-propylene. Most preferred is methylene.

The compounds of structural formulas (I) and (II) are effective as melanocortin receptor modulators and are particularly effective as selective modulators of MC-4R. They are Thereforee useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

### Optical Isomers - Diastereomers - Geometric Isomers - Tautomers

Compounds of structural formulas (I) and (II) contain one or more asymmetric centers and can occur as racemates and racemic mixtures, single enantiomers, diastereomeric mixtures and individual diastereomers. The present invention is meant to comprehend all such isomeric forms of the compounds of structural formulas (I) and (II).

Some of the compounds described herein may exist as tautomers, such as keto-enol tautomers. The individual tautomers, as well as mixtures thereof, are encompassed within the compounds of structural formulas (I) and (II).

Compounds of structural formulas (I) and (II) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formulas (I) and (II) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, ptoluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formulas (I) and (II) are meant to also include the pharmaceutically acceptable salts.

### Utility

Compounds of formulas (I) and (II) are melanocortin receptor modulators and as such are useful in the treatment, control or prevention of diseases, disorders or conditions responsive to the activation or inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety, depression, obesity (by reducing appetite, increasing metabolic rate, reducing fat intake or reducing carbohydrate craving), diabetes mellitus (by enhancing glucose tolerance, decreasing insulin resistance), hypertension, hyperlipidemia, osteoarthritis, cancer, gall bladder disease, sleep apnea, depression, anxiety, compulsion, neuroses, insomnia/sleep disorder, substance abuse, pain, male and female sexual dysfunction (including impotence, loss of libido and erectile dysfunction), fever, inflammation, immune-modulation, rheumatoid arthritis, skin tanning, acne and other skin disorders, neuroprotective and cognitive and memory enhancement including the treatment of Alzheimer's disease.

Some compounds encompassed by formulas (I) and (II) show highly selective affinity for the melanocortin-4 receptor relative to MC-1R, MC-2R, MC-3R and MC-5R, which makes them especially useful in the prevention and treatment of cancer cachexia, muscle wasting, anorexia, anxiety, depression, obesity, as well as male and/or female sexual dysfunction, including erectile dysfunction. "Male sexual dysfunction" includes impotence, loss of libido and erectile dysfunction. "Female sexual dysfunction" can be seen as resulting from multiple components including dysfunction in desire, sexual arousal, sexual receptivity and orgasm.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formulas (I) and (II) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formulas I and (II) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compound of formulas (I) and (II) is preferably formulated into a dosage form prior to administration. Accordingly the present invention also includes a pharmaceutical composition comprising a compound of formulas (I) and (II) and a suitable pharmaceutical carrier.

The present pharmaceutical compositions are prepared by known procedures using well-known and readily available ingredients. In making the formulations of the present invention, the active ingredient (a compound of formulas (I) and (II)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formulas (I) and (II), the terms "A moiety", "B moiety" and "C moiety" are used below. This moiety concept is illustrated below:

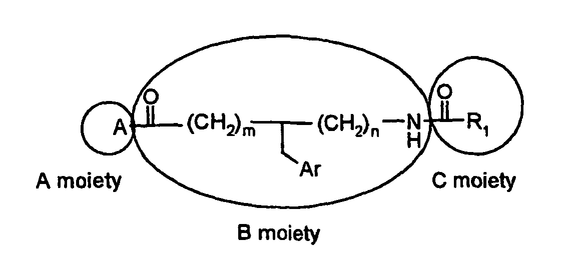

Preparation of the compounds of the present invention may be carried out via sequential or convergent synthetic routes. The skilled artisan will recognize that, in general, the three moieties of a compound of formulas (I) and (II) are connected via amide bonds. The skilled artist can, Thereforee, readily envision numerous routes and methods of connecting the three moieties via standard peptide coupling reaction conditions.

The phrase "standard peptide coupling reaction conditions" means coupling a carboxylic acid with an amine using an acid activating agent such as EDC, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene, et al., Protective Groups in Organic Synthesis, John Wiley & Sons, Inc., New York. NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The compounds of formulas (I) and (II), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formulas (I) and (II) may be obtained by stereospecific synthesis using optically pure starting materials or reagents of known configuration.

The compounds of formulas (I) and (II) of the present disclosure can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius. Mass spectra (MS) were measured by electron-spray ion-mass spectroscopy.

In the schemes, preparations and examples below, various reagent symbols and abbreviations have the following meanings:
- Boc: tert-butoxycarbonyl
- DCM: dichloromethane
- DIPEA: diisopropylethylamine
- DMAP: 4-dimethylaminopyridine
- DMF: N,N-dimethylformamide
- EDC: 1-(3-dimethylaminopropyl)-3-ethylcarbodiimide hydrochloride
- Et: ethyl
- EtOAc: ethyl acetate
- Fmoc: 9-fluorenylmethyl-carbamate
- HOAc: acetic acid
- HOAt: 1-hydroxy-7-azabenzotriazole
- HOBt: 1-hydroxybenzotriazole
- h: hour(s)
- NMM: N-methylmorpholine
- Phe: phenylalanine
- TFA: trifluoroacetic acid
- TEA: triethylamine
- THF: tetrahydrofurane
- Tic: 1,2,3,4-tetrahydroisoquinoline-3-carboxylic acid
- TMOF: trimethylorthoformate
- Z: benzyloxycarbonyl

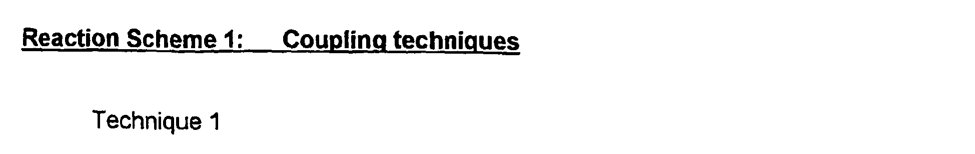

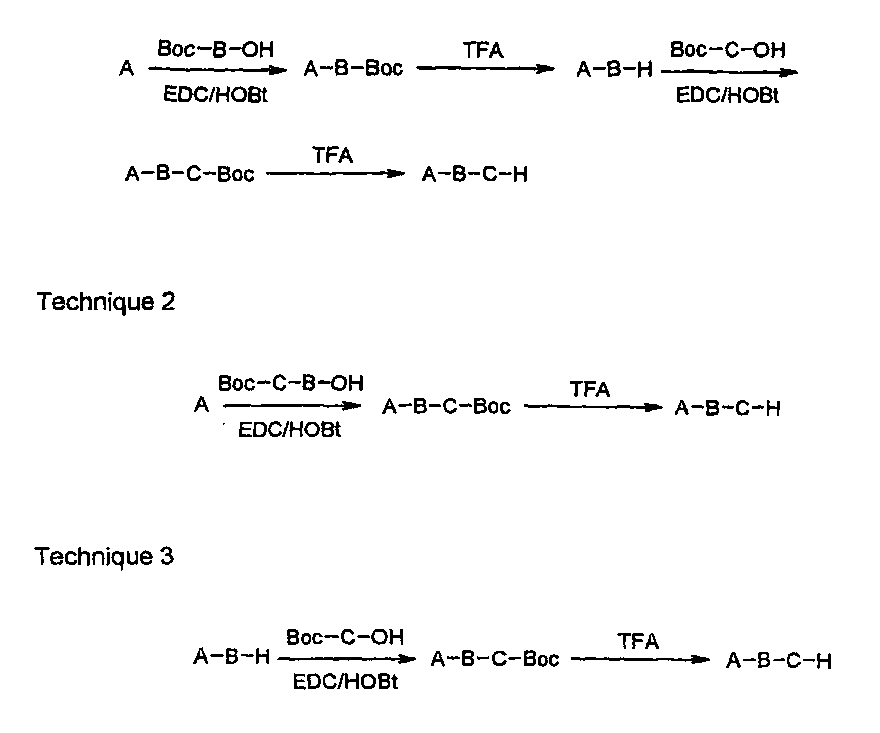

In coupling technique 1, an appropriate "A moiety" (e.g., 4-cyclohexyl-4-[1,2,4]triazol-1-yl-methyl-piperidine) is coupled to "B moiety" (e.g., L-Boc-p-Cl-Phe-OH) in the presence of EDC/HOBt followed by Boc deprotection. The coupled AB compound is then coupled to an appropriate "C moiety" followed by deprotection of Boc group and salt formation. Alternatively, when "C moiety" is not protected with Boc group, the final compound can be obtained without the deprotection step.

In coupling technique 2, an appropriate "AB moiety" is coupled to an appropriate "C moiety" in the presence of EDC/HOBt followed by deprotection of Boc group and salt formation. Alternatively, when "C moiety" is not protected with Boc group, the final compound can be obtained without the deprotection step.

In coupling technique 3, an appropriate "BC moiety" is coupled to an appropriate "A moiety" in the presence of EDC/HOBt followed by deprotection of Boc group and salt formation. Alternatively, when "C moiety" is not protected with Boc group, the final compound can be obtained without the deprotection step.

For coupling of A with Boc-B-OH, EDC/HOAt, EDC/HOBt or DCC/HOBt can be used.

Generally, the starting material of Boc-protected piperazine or piperidine (A moiety) can be deprotected in the presence of TFA/CH₂Cl₂, HCI/EtOAc, HCl/dioxane or HCl in MeOH/Et₂O with or without a cation scavenger, such as dimethyl sulfide (DMS) before being subjected to the coupling procedure. It can be free-based before being subjected to the coupling procedure or in some cases used as the salt.

A suitable solvent, such as CH₂Cl₂, DMF, THF or a mixture of the above solvents, can be used for the coupling procedure. A suitable base includes triethylamine (TEA), diisopropyethylamine (DIPEA), N-methymorpholine (NMM), collidine or 2,6-lutidine.

A base may not be needed when EDC/HOBt is used.

Generally after the reaction is completed, the reaction mixture can be diluted with an appropriate organic solvent, such as EtOAc, CH₂Cl₂ or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

Protecting groups such as Boc, Z, Fmoc and CF₃CO can be deprotected in the presence of H₂/Pd-C, TFA/DCM, HCI/EtOAc, HCl/dioxane, HCl in MeOH/Et₂O, NH₃/MeOH or TBAF, with or without a cation scavenger, such as thioanisole, ethane thiol and dimethyl sulfide (DMS). The deprotected amines can be used as the resulting salt or are free-based by dissolving in DCM and washing with aqueous bicarbonate or aqueous NaOH. The deprotected amines can also be free-based by ion exchange chromatography.

The "A moieties" can be prepared as described in the corresponding literature:
4-Cyclohexyl-4-[1,2,4]triazol-1-ylmethyl-piperidine (WO0074679), 4-cyclohexyl-piperidine-4-carboxylic acid tert-butylamide (WO0170708), 1,2-dihydro-1-methanesulfonylspiro-[3H-indole-3,4'-piperidine] (US005536716), 3a-benzyl-2-methyl-2,3a,4,5,6,7-hexahydro-pyrazolo[4,3-c]pyridin-3-one (WO9858949) and N-(2-piperazin-1-yl-benzyl)-methanesulfonamide, 1-(2-[1,2,4]triazol-1-ylmethyl-phenyl)-piperazine, N-(2-piperazin-1-yl-phenyl)-isobutyramide, N-(2-piperazin-1-yl-phenyl)-methanesulfonamide, (2-piperazin-1-yl-phenyl)-piperidin-1-yl-methanone, N,N-diethyl-2-piperazin-1-yl-benzamide (WO02059108).

The following "A moieties" were prepared in an analogues way:
N-(2-Piperazin-1-yl-phenyl)-benzamide, (4-methyl-piperazin-1-yl)-(2-piperazin-1-yl-phenyl)-methanone, morpholin-4-yl-(2-piperazin-1-yl-phenyl)-methanone, (2-piperazin-1-yl-phenyl)-pyrrolidin-1-yl-methanone, N-(2-methoxy-ethyl)-2-piperazin-1-yl-benzamide, N-(2-dimethylamino-ethyl)-2-piperazin-1-yl-benzamide, 2-piperazin-1-yl-N-(2,2,2-trifluoro-ethyl)-benzamide, N-cyclopropyl-2-piperazin-1-yl-benzamide, 4-methyl-N-(2-piperazin-1-yl-phenyl)-benzene-sulfonamide, 4-bromo-2-piperazin-1-yl-benzonitrile (WO02059108) and 4-cyclohexyl-piperidine-4-carboxylic acid methyl ester (WO0170708).

### Reaction Schemes for Preparation of "C moiety"

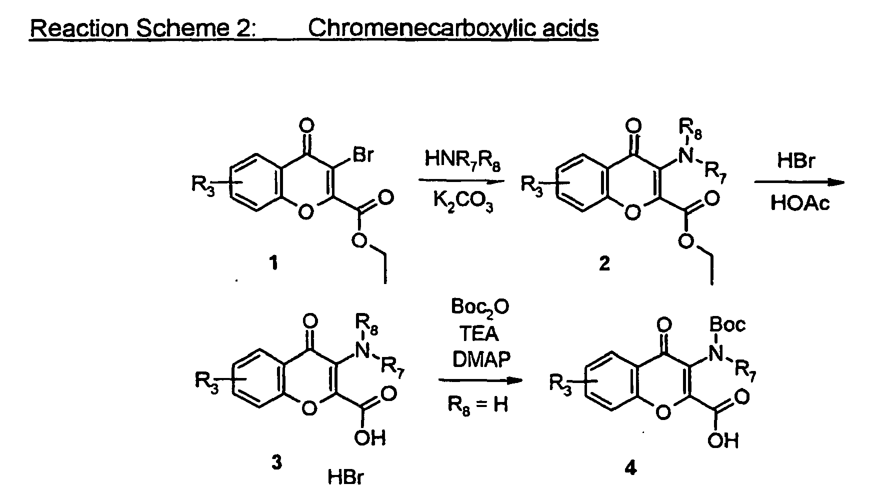

As shown in Reaction Scheme 2, ethyl 3-bromo-4-oxochromene-2-carboxylate **1** (*J. Chem. Soc. Perkin Trans. I* 1986, 1643-1649) can be reacted with amines, with or without a base such as K₂CO₃, in an appropriate solvent such as MeCN, to form products **2** which are subsequently treated with a reagent such as HBr/HOAc, to form carboxylic acids **3.** When R₈ is hydrogen, the free amine can be protected with a reagent such as Boc₂O in the presence of TEA and DMAP in an appropriate solvent.

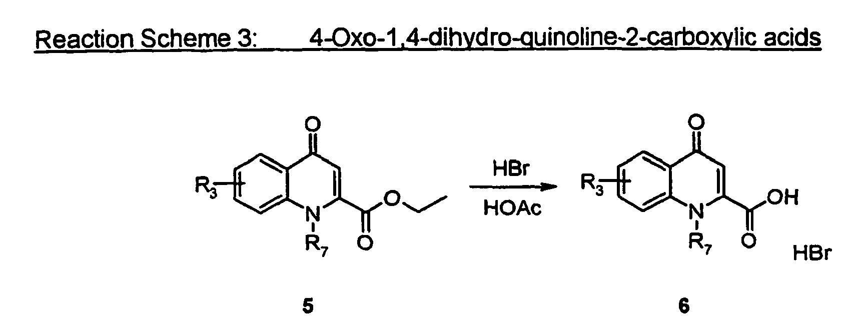

As shown in Reaction Scheme 3, ethyl 4-oxo-1,4-dihydro-quinoline-2-carboxylates **5** (Bioorg. Med. Chem. Lett. 2000, 10, 1487-1490) can be converted into the corresponding acids **6** by an appropriate reactant such as HBr/HOAc.

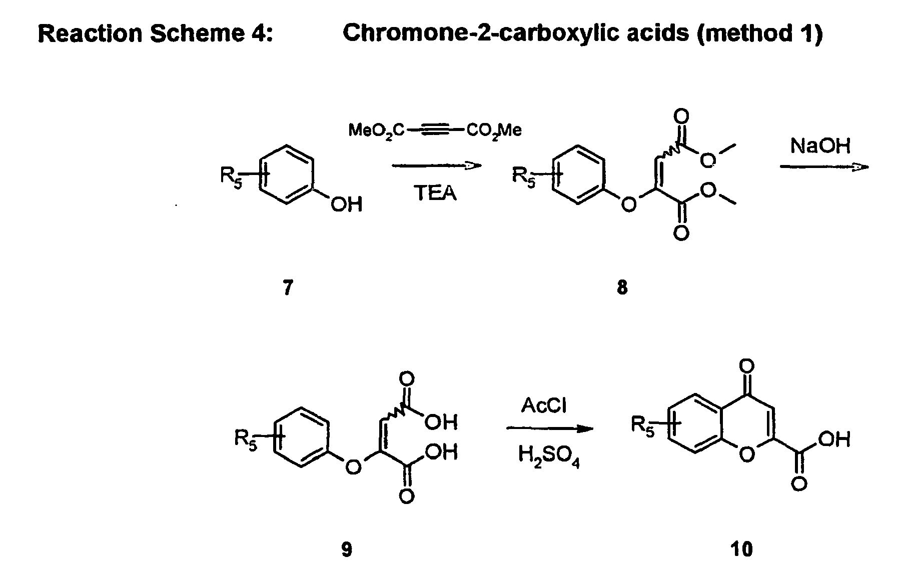

As shown in Reaction Scheme 4, substituted phenols **7** can be reacted with triethylamine followed by dimethyl acetylendicarboxylate in diethyl ether to yield compounds **8** (Aust. J. Chem. 1995, 48, 677-686). Saponification of the latter with aqueous sodium hydroxide leads to acids **9** which are subsequently cyclized to the chromone-2-carboxylic acids **10** using concentrated sulfuric acid in acetyl chloride.

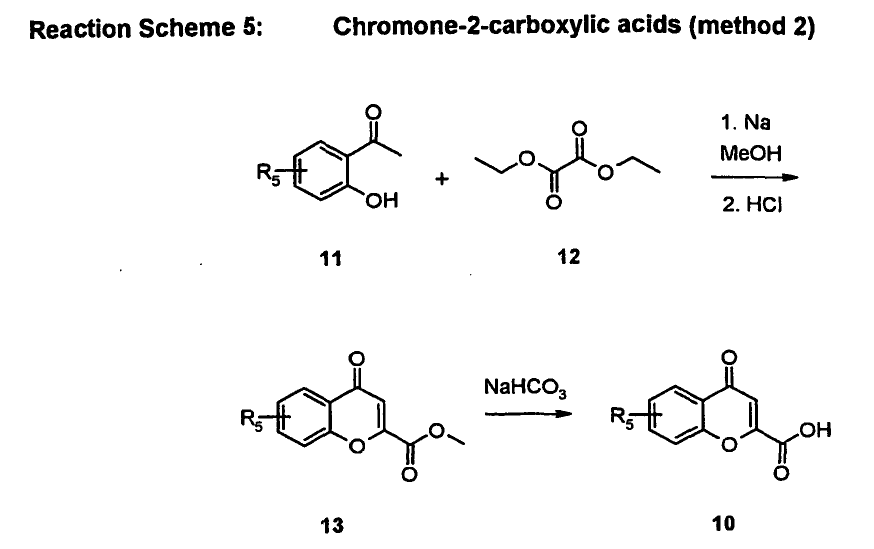

As shown in Reaction Scheme 5, 2'-hydroxyacetophenones **11** can be reacted with diethyl oxalate **12** in the presence of a base such as sodium methoxide in an appropriate solvent such as methanol or benzene followed by treatment with an acid such as hydrochloric acid to yield chromone-2-carboxylic acid esters **13** (J. Indian Chem. Soc. 1986, 63, 600-602). The esters can be cleaved using basic conditions such as sodium bicarbonate in water or acidic conditions such as polyphosphoric acid at an appropriate temperature to the corresponding acids **10.**

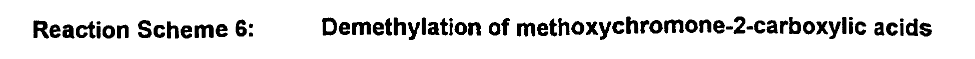

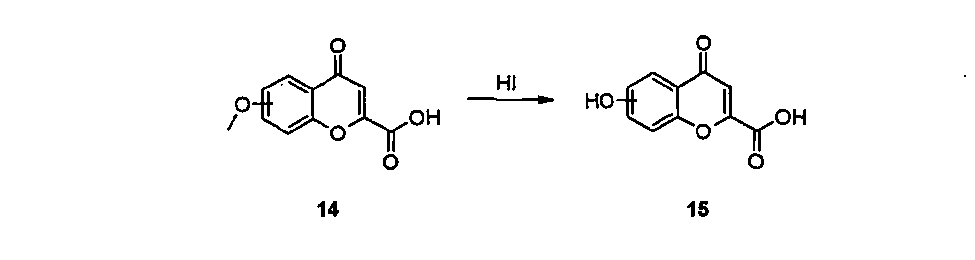

As shown in Reaction Scheme 6, methoxy-substituted chromone-2-carboxylic acids can be demethylated with reagents such as hydroiodic acid in an appropriate solvent such as glacial acetic acid to yield the corresponding hydroxy-substituted chromone-2-carboxylic acids.

5,7-Dihydroxychromone-2-carboxylic acid was prepared as described in the literature (OPPI Briefs 1991, 23, 390-392).

The following describes the detailed examples of the invention

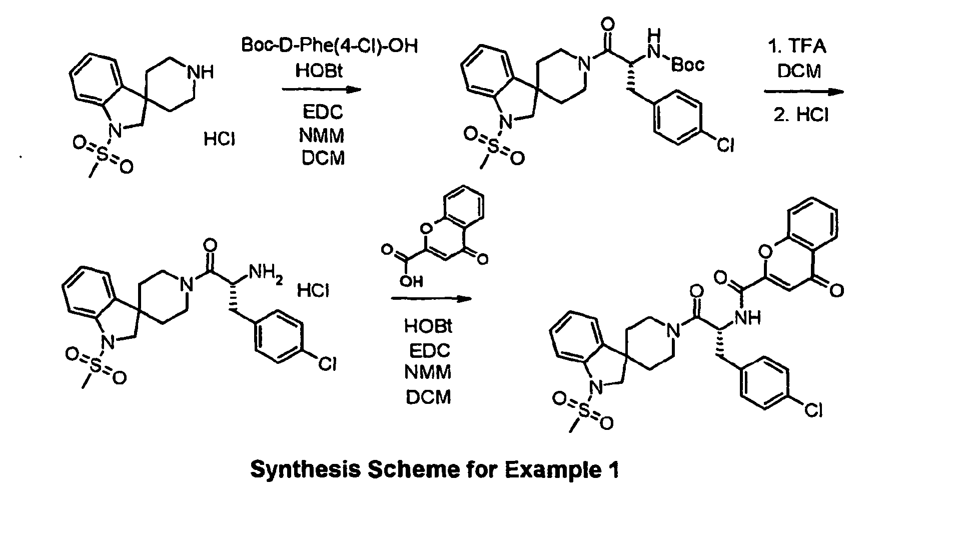

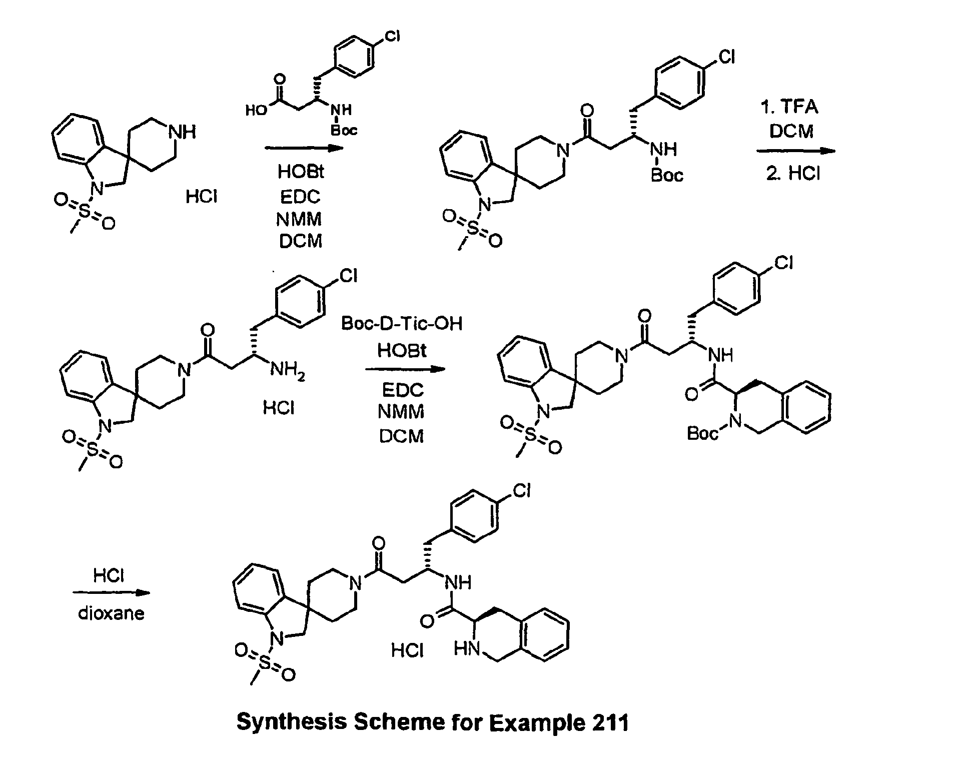

The following examples are provided to illustrate the invention and are not limiting the scope of the invention in any manner.

### Example 1:

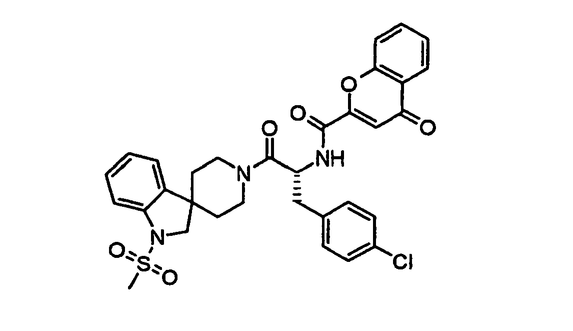

To chromone-2-carboxylic acid (17 mg) in DCM (2 ml) was added intermediate 1b) (39 mg), N-methylmorpholine (14 µl), HOBt (14 mg) and stirred for 20 min. EDC (23 mg) was added and stirring was continued for 1 h. An additional amount of N-methylmorpholine (8 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated to yield the product which was purified by column chromatography.
colorless solid
R_{f} = 0.52 (EtOAc); Mp. 137 - 147 °C.

The required intermediates can be synthesized in the following way:

### Intermediate 1a):

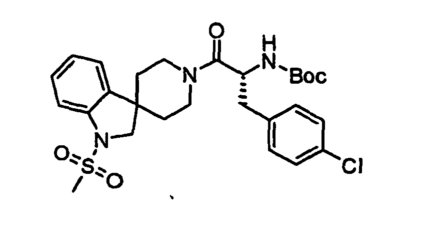

To Boc-D-4-chlorophenylaianine (82 mg) in DCM (5 ml) was added the amine hydrochloride (preparation in US005536716) (62 mg), N-methylmorpholine (42 µl), HOBt (48 mg) and stirred for 20 min. EDC (72 mg) was added and stirring was continued for 1 h. An additional amount of N-methylmorpholine (20 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated in vacuo. Purification by column chromatography yielded the title compound.

### Intermediate 1b):

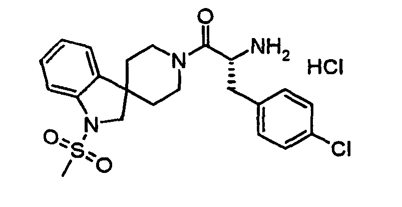

To the Boc-protected amine from 1a) (86 mg) in DCM (5 ml) was added TFA (1 ml) and stirred at room temperature for 90 min. Additional TFA (1 ml) was added and stirred for 10 min. The reaction mixture was diluted with DCM (10 ml) and carefully basified by pouring into 10% aqueous sodium carbonate solution (20 ml). The organic layer was separated and the aqueous layer was further extracted three times with DCM. The combined organics were washed with water and brine, dried over Na₂SO₄, concentrated to give a white solid.

For prolonged storage, the free base was converted into the corresponding hydrochloride. The free base was dissolved in DCM (5 ml) and app. 1 M HCl in ether (10 ml) was added. The precipitate was filtered and the residue was washed three times with ether and dried under reduced pressure to yield the desired compound.

The following examples can be prepared in a similar way:

### Example 2:

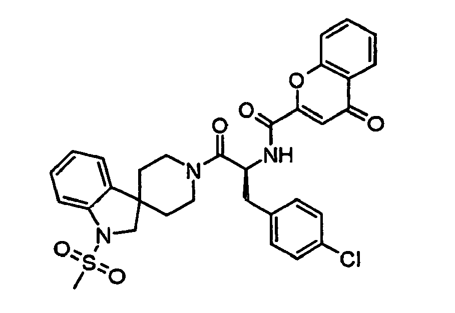

colorless solid
R_{f} = 0.52 (EtOAc); Mp. 139 - 160 °C.

### Example 3:

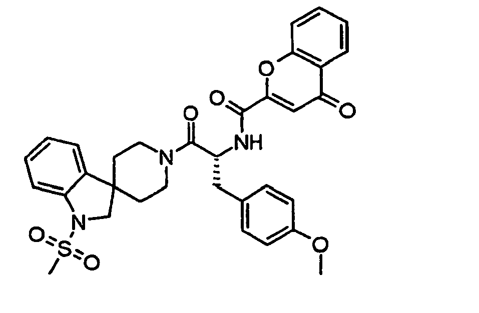

white solid
R_{f} = 0.56 (EtOAc); Mp. 203 - 204 °C.

### Example 4:

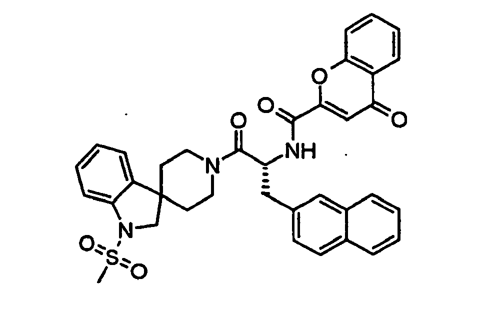

white solid
R_{f} = 0.75 (EtOAc/ethanol 3:1); Mp. 166 - 168 °C.

### Example 5:

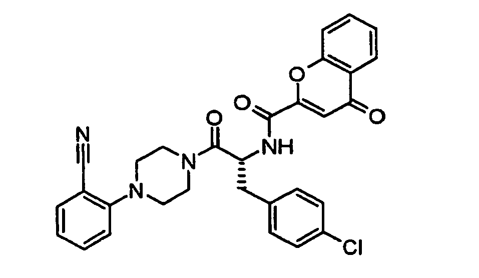

white solid
R_{f} = 0.61 (EtOAc); Mp. 94 °C.

### Example 6:

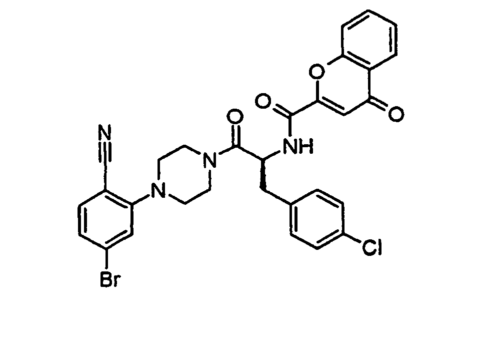

white solid
R_{f} = 0.71 (EtOAc); Mp. 147 - 156 °C.

### Example 7:

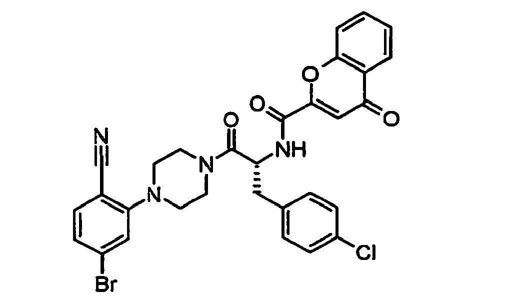

pale yellow solid
R_{f} = 0.61 (EtOAc); Mp. 119 - 126 °C.

### Example 8:

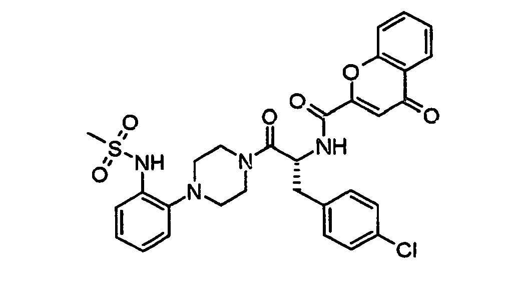

white solid
R_{f} = 0.62 (EtOAc); Mp. 136 - 140 °C.

### Example 9:

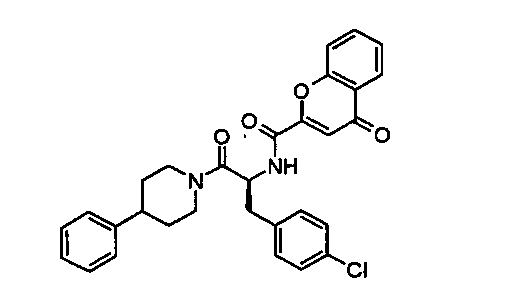

white solid
R_{f} = 0.77 (EtOAc); Mp. 157 - 161 °C.

### Example 10:

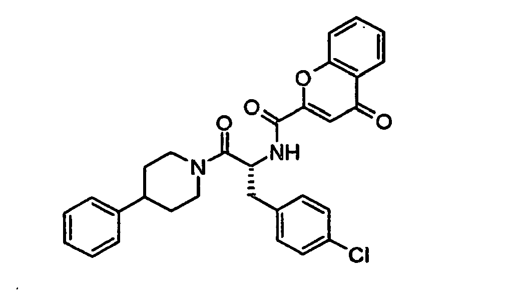

white solid
R_{f} = 0.77 (EtOAc); Mp. 160 - 167 °C.

### Example 11:

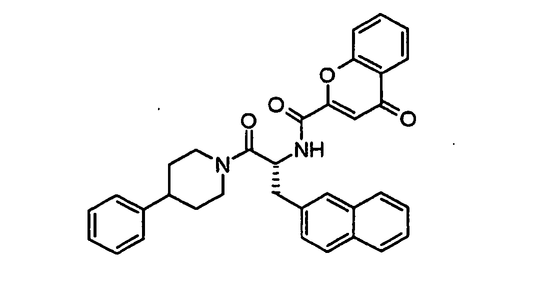

white solid
R_{f} = 0.74 (EtOAc); Mp. 140 - 149 °C.

### Example 12:

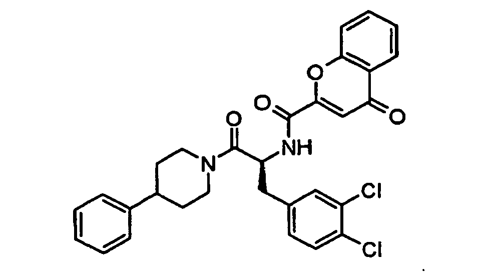

white solid
R_{f} = 0.74 (EtOAc); Mp. 128 - 135 °C.

### Example 13:

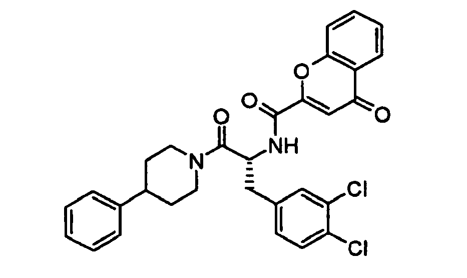

white solid
R_{f} = 0.74 (EtOAc); Mp. 107 - 118 °C.

### Example 14:

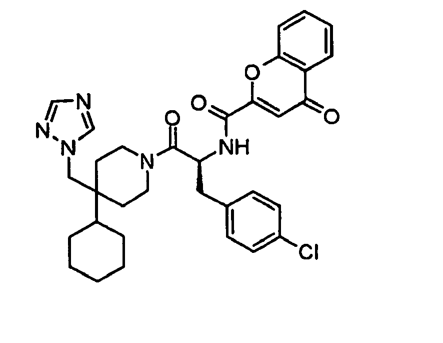

colorless glassy solid
R_{f} = 0.67 (EtOAc/ethanol 3:1); Mp. 126 - 135 °C.

### Example 15:

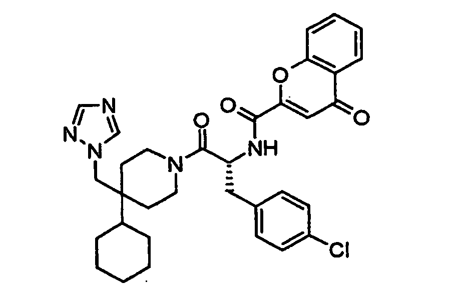

white solid
R_{f} = 0.67 (EtOAc/ethanol 3:1); Mp. 117 - 126 °C.

### Example 16:

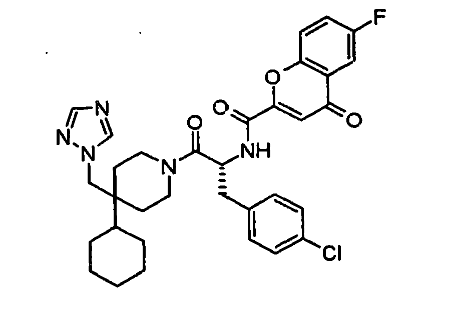

white solid
R_{f} = 0.77 (EtOAc/ethanol 3:1); Mp. 147 - 157 °C.

### Example 17:

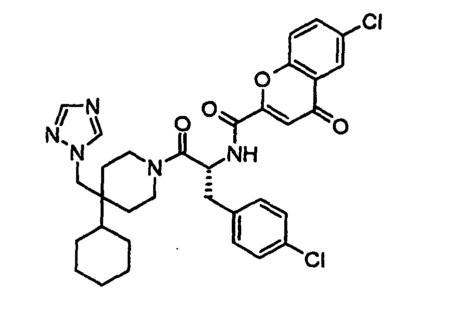

white solid
R_{f} = 0.65 (EtOAc/ethanol 3:1); Mp. 136 - 141 °C.

### Example 18:

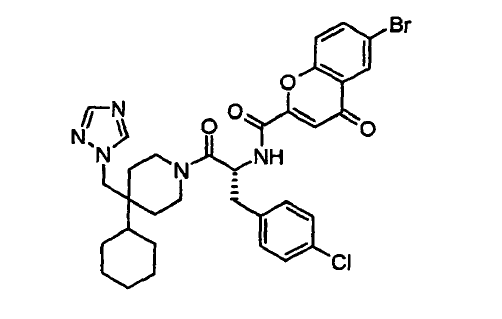

white solid
R_{f} = 0.70 (EtOAc/ethanol 3:1); Mp. 124 - 132 °C.

### Example 19:

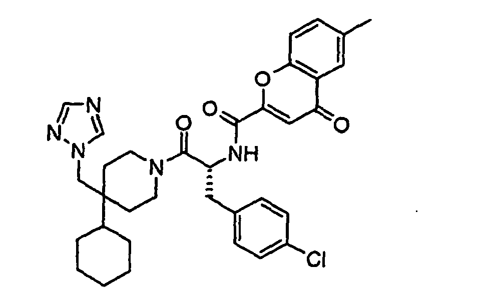

white solid
R_{f} = 0.75 (EtOAc/ethanol 3:1); Mp. 147 - 150 °C.

### Example 20:

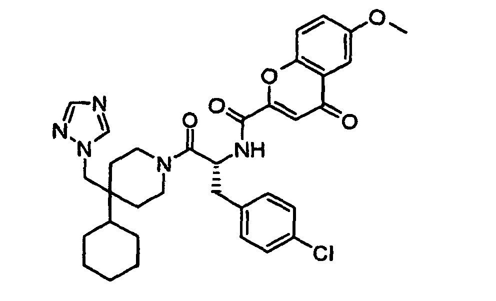

off-white solid
R_{f} = 0.58 (EtOAc/ethanol 3:1); Mp. 120 - 125 °C.

### Example 21:

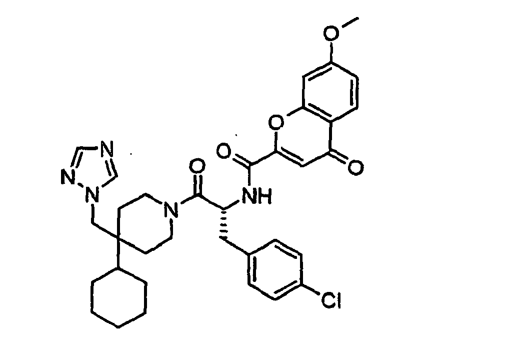

white solid
R_{f} = 0.56 (EtOAc/ethanol 3:1); Mp. 139 - 144 °C.

### Example 22:

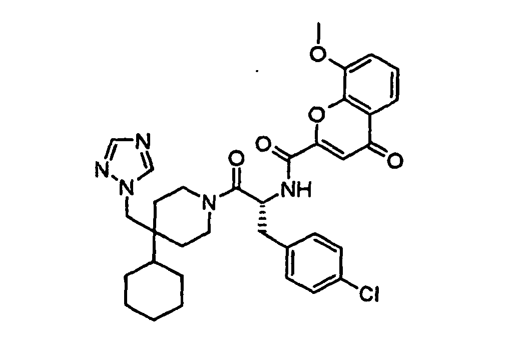

white solid
R_{f} = 0.54 (EtOAc/ethanol 3:1); Mp. 132 - 140 °C.

### Example 23:

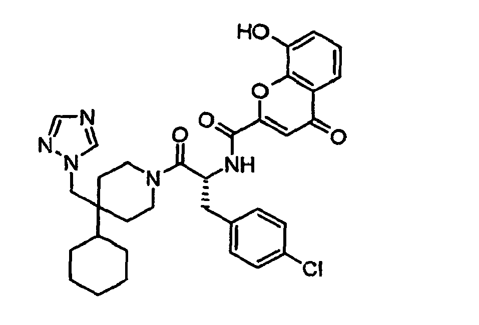

off-white solid
R_{f} = 0.56 (EtOAc/ethanol 3:1) ; Mp. 154 - 162 °C.

### Example 24:

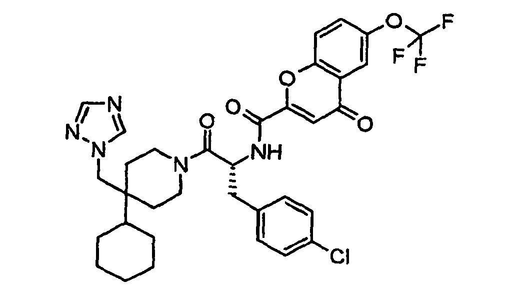

off-white solid
R_{f} = 0.69 (EtOAc/ethanol 3:1); Mp. 117 - 130 °C.

### Example 25:

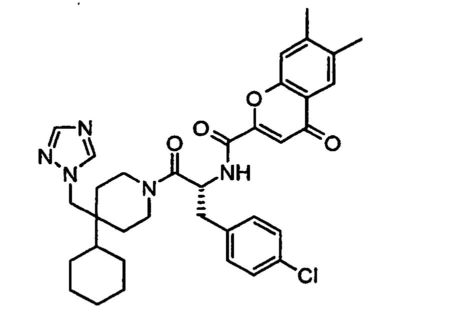

white solid
R_{f} = 0.64 (EtOAc/ethanol 3:1); Mp. 137 - 142 °C.

### Example 26:

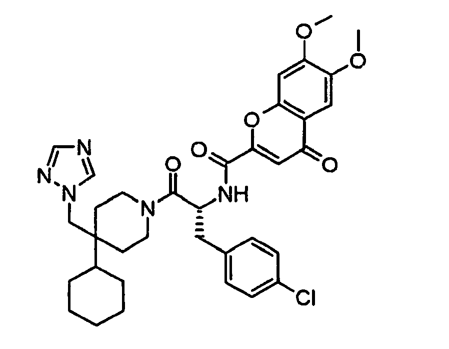

white solid
R_{f} = 0.53 (EtOAc/ethanol 3:1); Mp. 144 - 157 °C.

### Example 27:

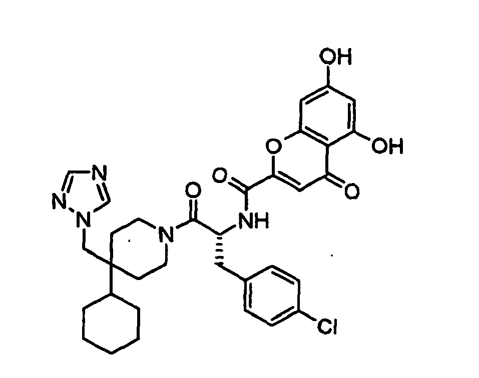

beige solid
R_{f} = 0.66 (EtOAc/ethanol 3:1); Mp. 166 - 173 °C.

### Example 28:

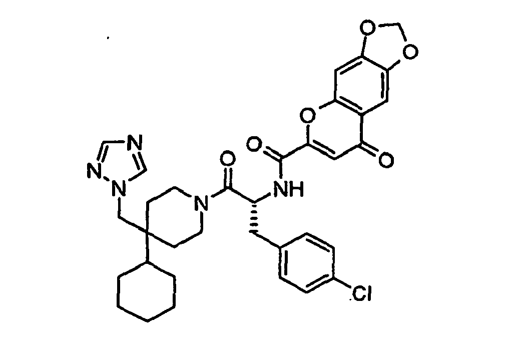

white solid
R_{f} = 0.60 (EtOAc/ethanol 3:1); Mp. 142 - 146 °C.

### Example 29:

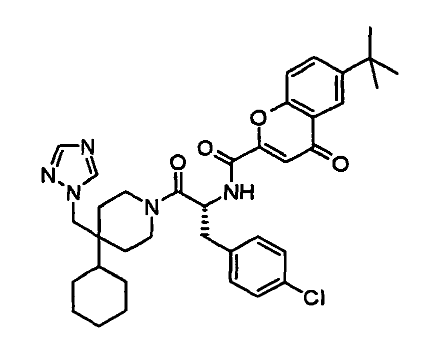

white solid
R_{f} = 0.65 (EtOAc/ethanol 3:1); Mp. 130 - 141 °C.

### Example 30:

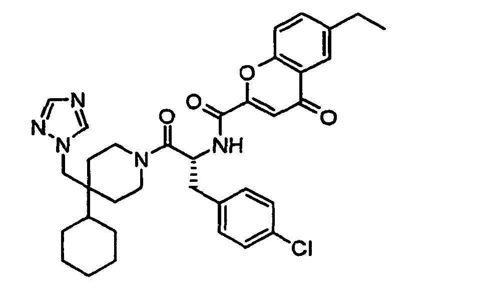

white solid
R_{f} = 0.76 (EtOAc/ethanol 3:1); Mp. 116 - 124 °C.

### Example 31:

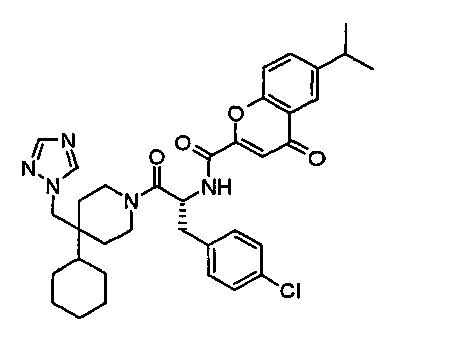

white solid
R_{f} = 0.69 (EtOAc/ethanol 3:1); Mp. 119 - 126 °C.

### Example 32:

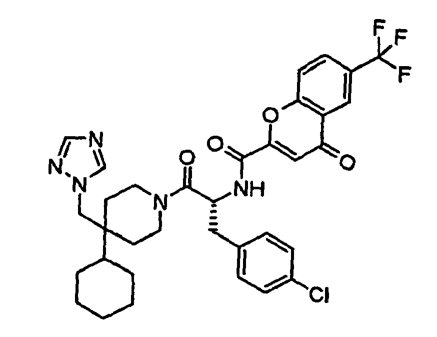

### Example 33:

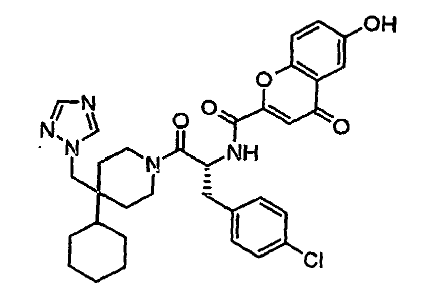

### Example 34:

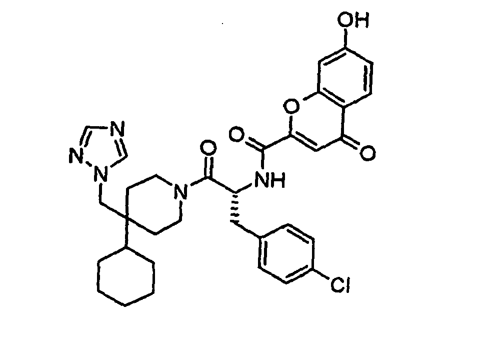

### Example 35:

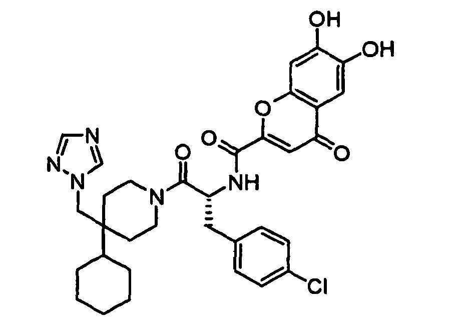

### Example 36:

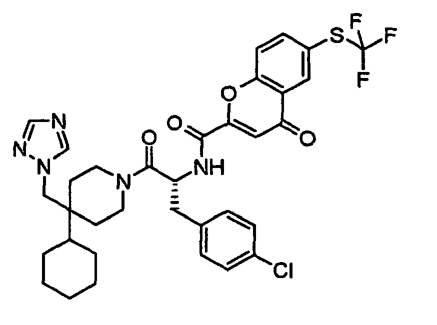

white solid
R_{f} = 0.83 (EtOAc/ethanol 3:1); Mp. 122 - 130 °C.

### Example 37:

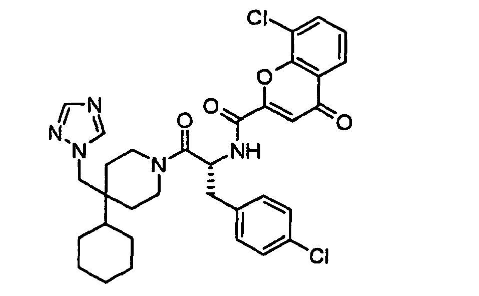

off-white solid
R_{f} = 0.77 (EtOAc/ethanol 3:1); Mp. 123 - 134 °C.

### Example 38:

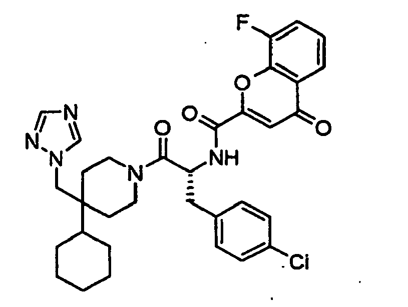

white solid
R_{f} = 0.78 (EtOAc/ethanol 3:1); Mp. 123 - 132 °C.

### Example 39:

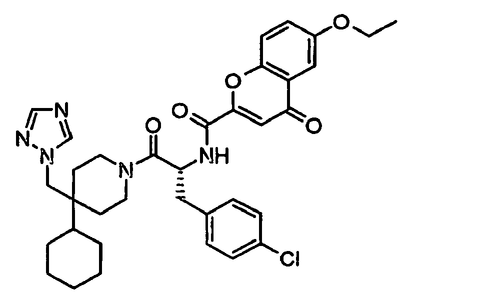

### Example 40:

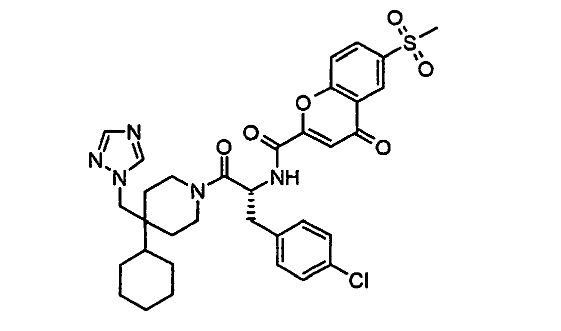

### Example 41:

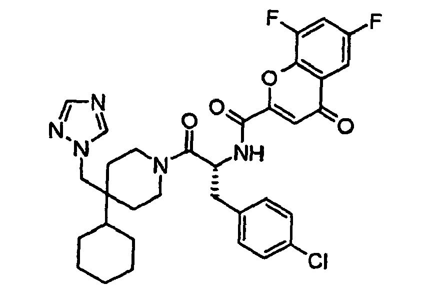

### Example 42:

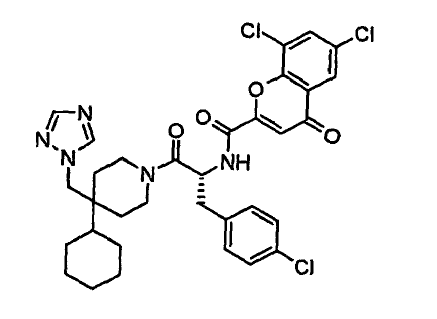

### Example 43:

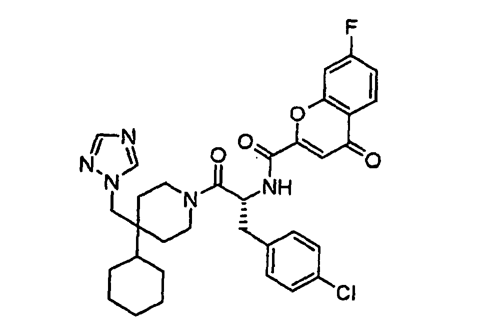

### Example 44:

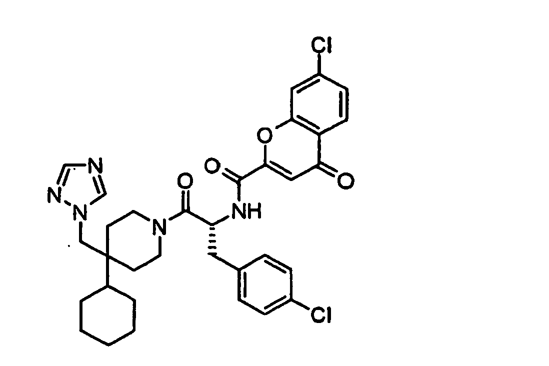

### Example 45:

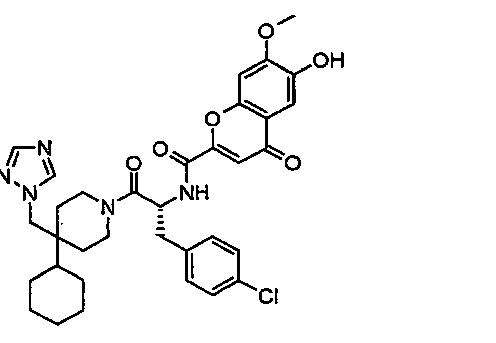

### Example 46:

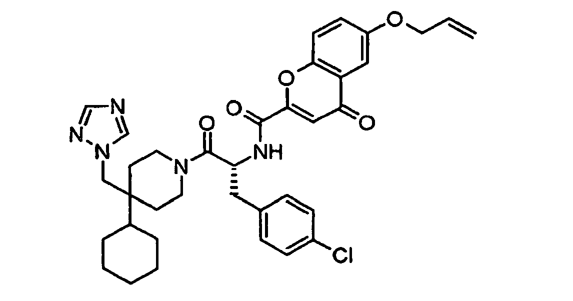

### Example 47:

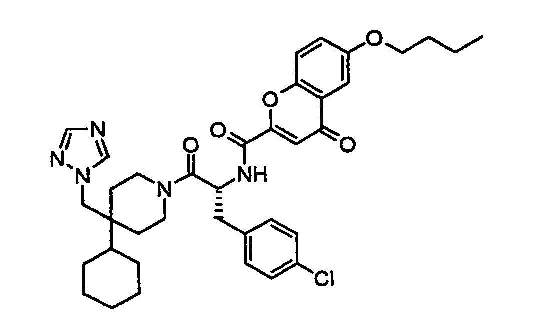

### Example 48:

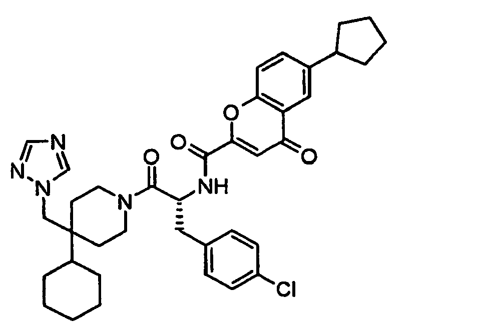

### Example 49:

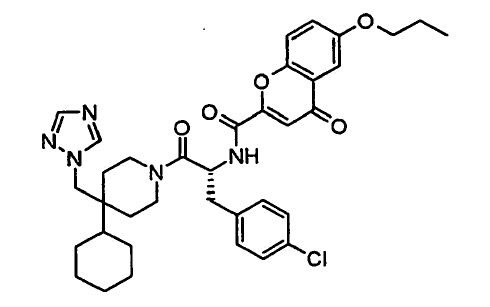

### Example 50:

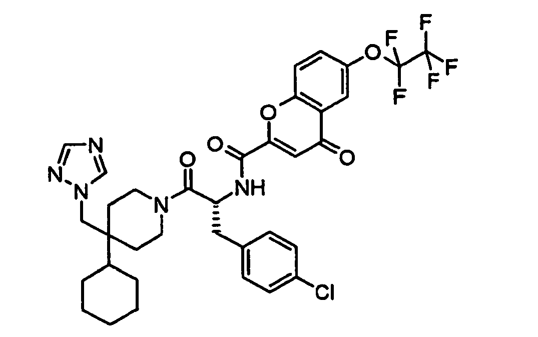

### Example 51:

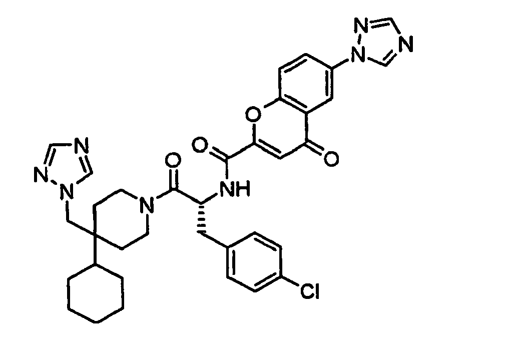

### Example 52:

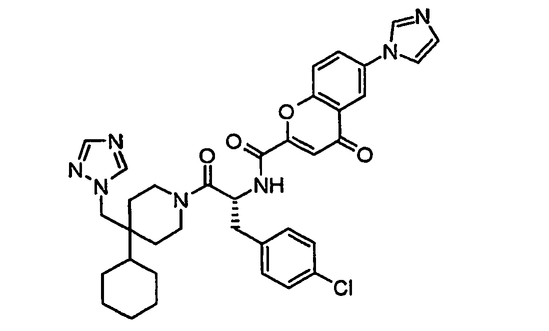

### Example 53:

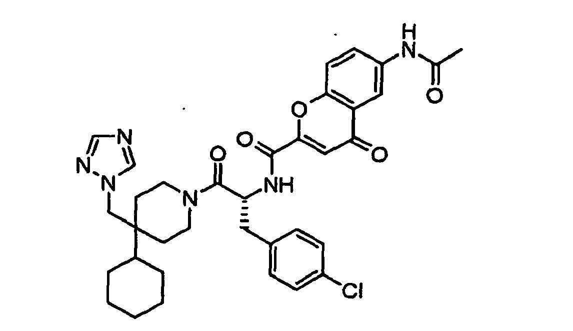

### Example 54:

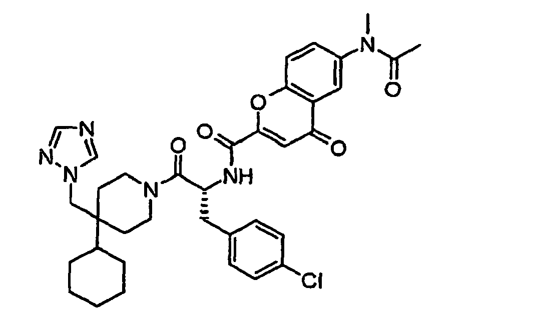

### Example 55:

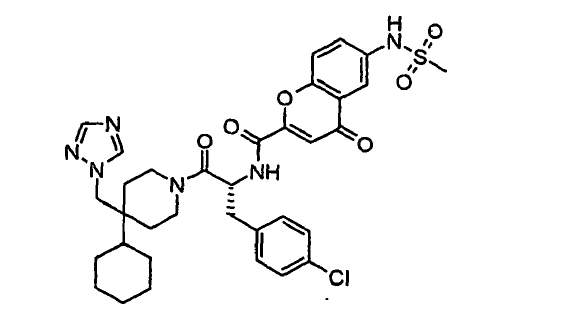

### Example 56:

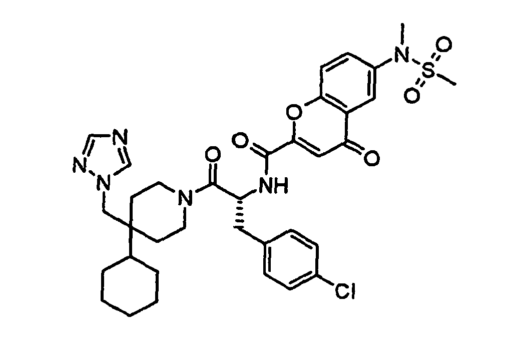

### Example 57:

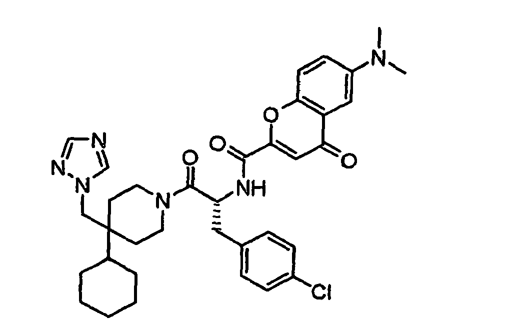

### Example 58:

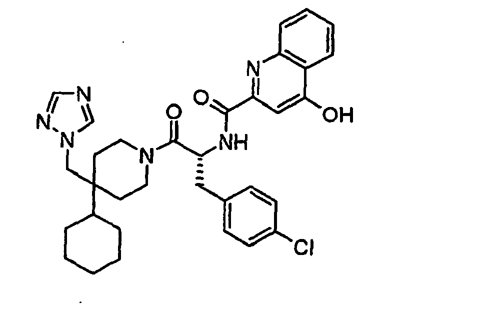

white solid
R_{f} = 0.62 (EtOAc/ethanol 3:1); Mp. 154 - 162 °C.

### Example 59:

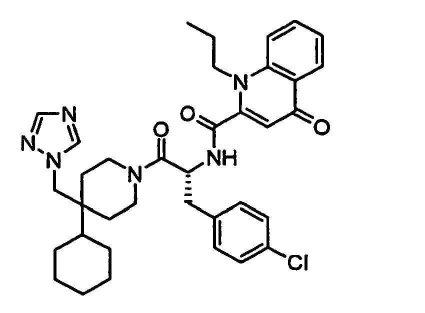

white solid
R_{f} = 0.66 (EtOAc/ethanol 3:1); Mp. 143 - 150 °C.

### Example 60:

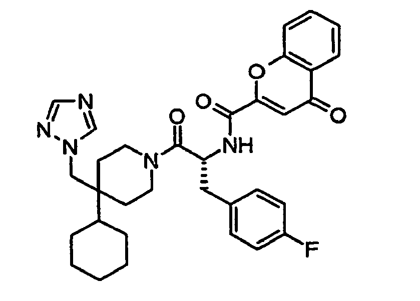

white solid
R_{f} = 0.68 (EtOAc/ethanol 3:1); Mp. 118 - 124 °C.

### Example 61:

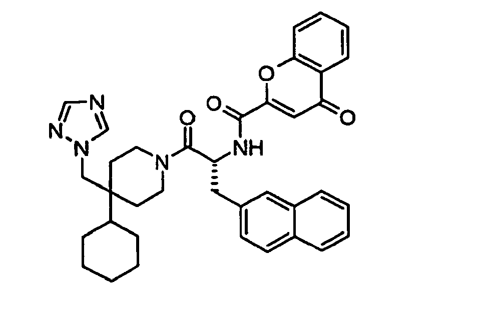

white solid
R_{f} = 0.66 (EtOAc/ethanol 3:1); Mp. 132 - 142 °C.

### Example 62:

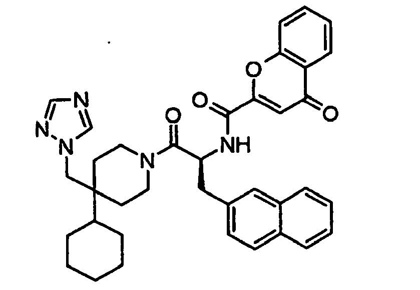

white solid
R_{f} = 0.66 (EtOAc/ethanol 3:1); Mp. 132 - 142 °C.

### Example 63:

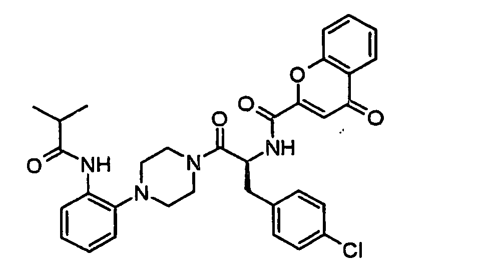

white solid
R_{f} = 0. 54 (EtOAc); Mp. 140 - 163 °C.

### Example 64:

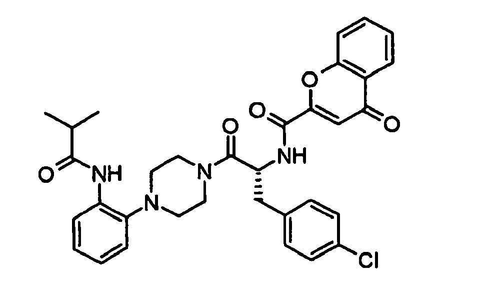

white solid
R_{f} = 0. 54 (EtOAc); Mp. 149 - 158 °C.

### Example 65:

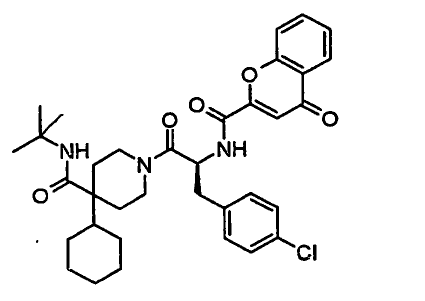

white solid
R_{f} = 0.58 (EtOAc); Mp. 136 - 148 °C.

### Example 66:

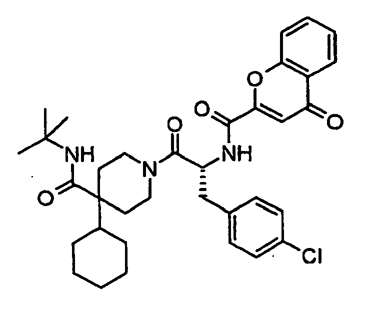

white solid
R_{f} = 0.58 (EtOAc); Mp. 139 - 151 °C.

### Example 67:

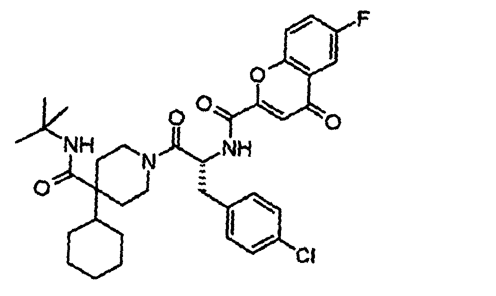

white solid
R_{f} = 0.83 (EtOAc); Mp. 139 - 146 °C.

### Example 68:

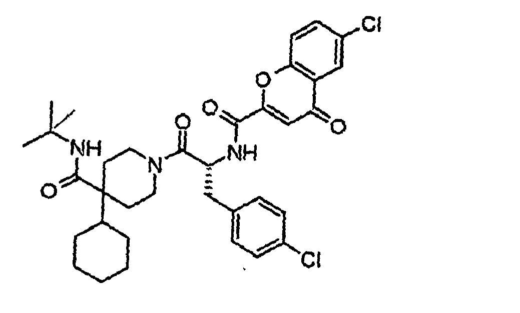

white solid
R_{f} = 0.71 (EtOAc); Mp. 170 - 177 °C.

### Example 69:

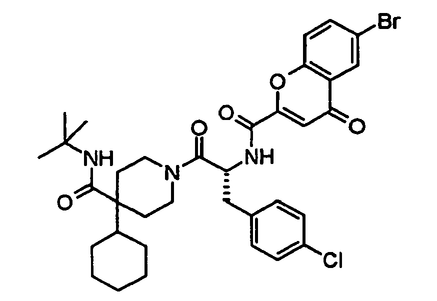

white solid
R_{f} = 0.82 (EtOAc); Mp. 157 - 166 °C.

### Example 70:

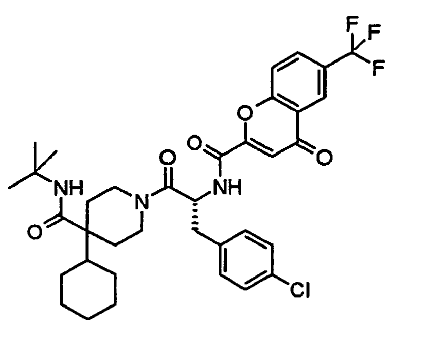

white solid
R_{f} = 0.74 (EtOAc); Mp. 127 - 131 °C.

### Example 71:

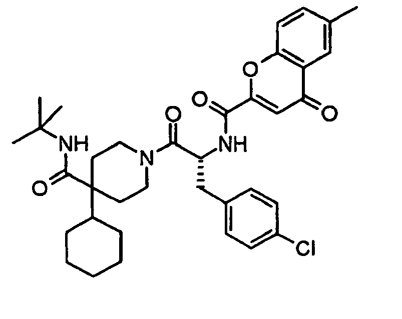

white solid
R_{f} = 0.72 (EtOAc); Mp. 141 - 148 °C.

### Example 72:

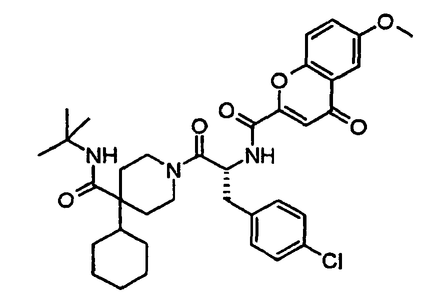

white solid
R_{f} = 0.63 (EtOAc); Mp. 137 - 140 °C.

### Example 73:

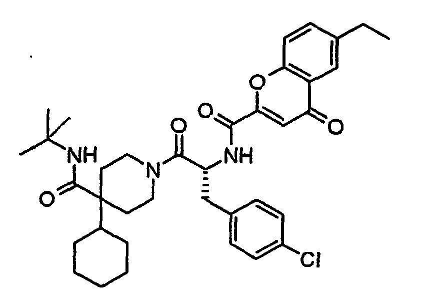

### Example 74:

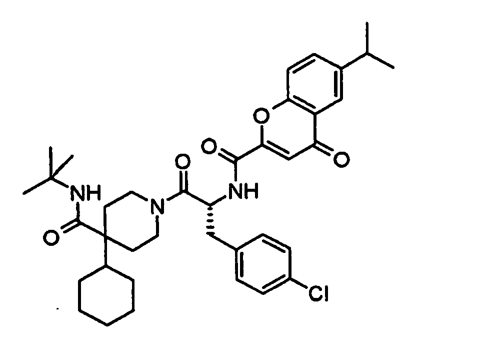

### Example 75:

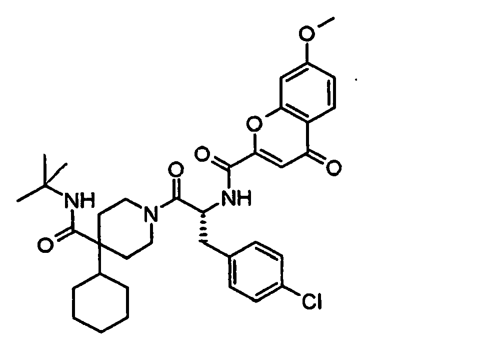

### Example 76:

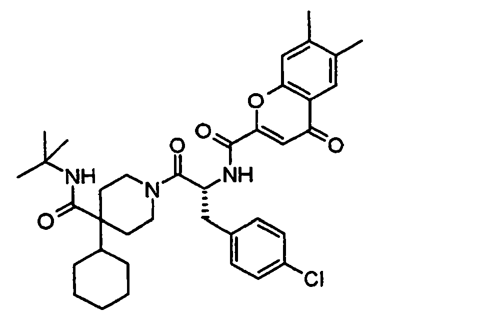

### Example 77:

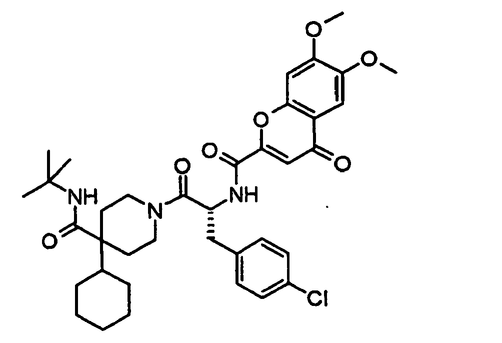

### Example 78:

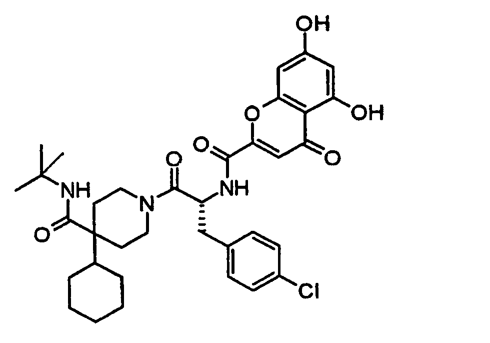

### Example 79:

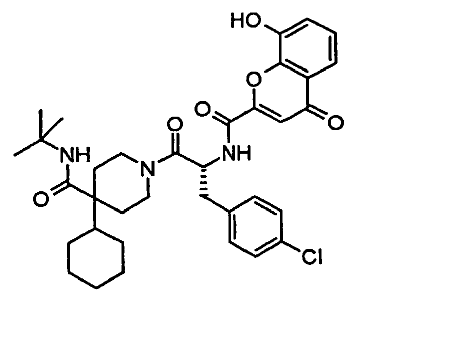

### Example 80:

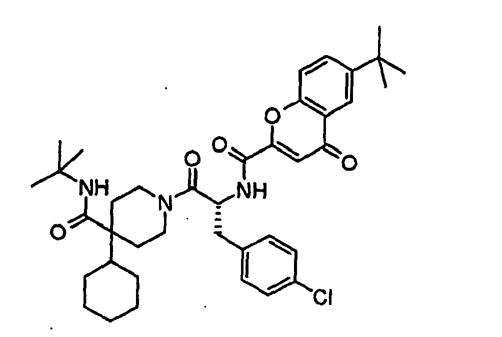

### Example 81:

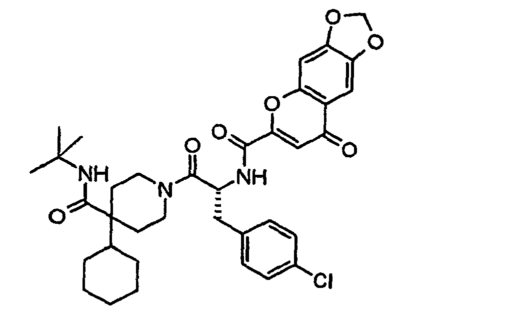

### Example 82:

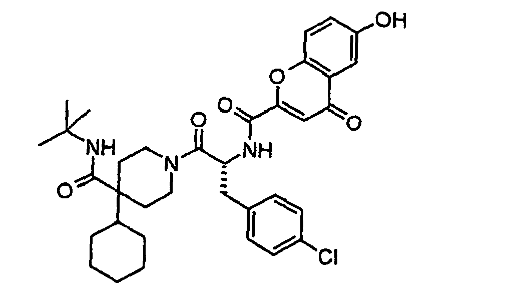

### Example 83:

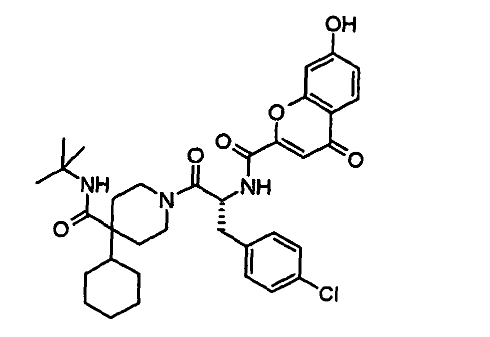

### Example 84:

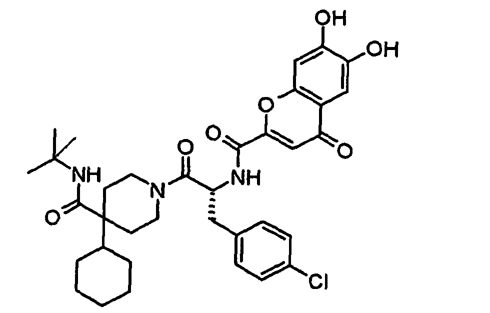

### Example 85:

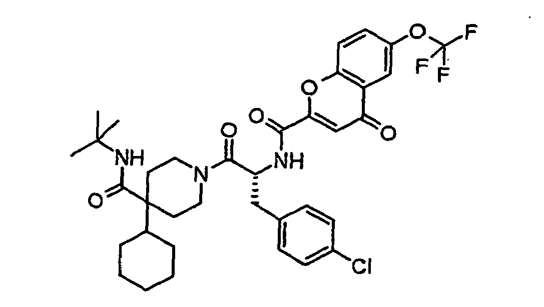

### Example 86:

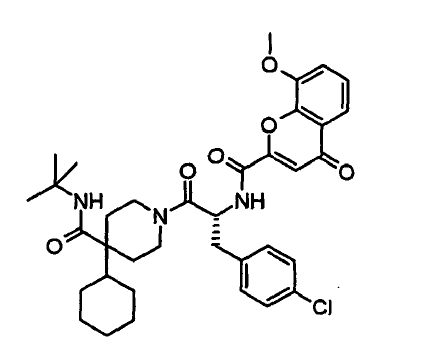

### Example 87:

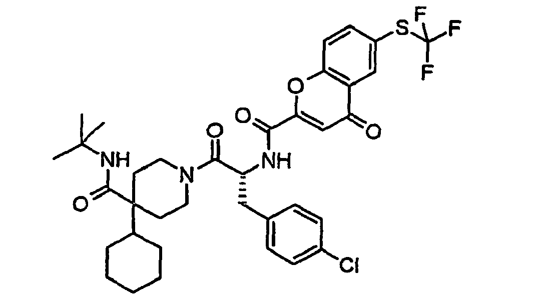

### Example 88:

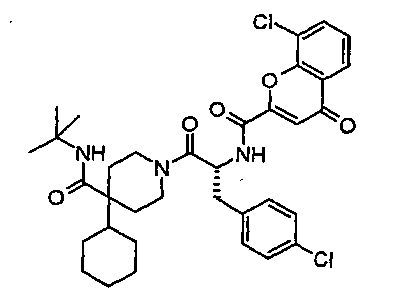

### Example 89:

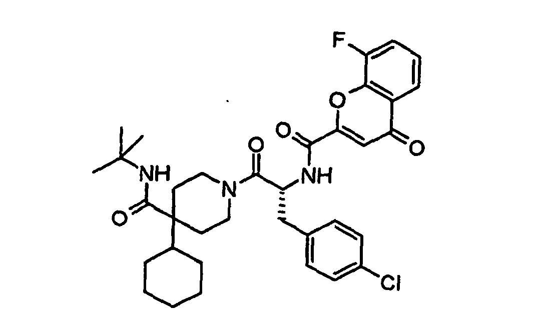

### Example 90:

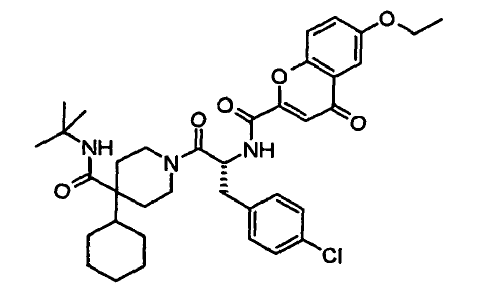

### Example 91:

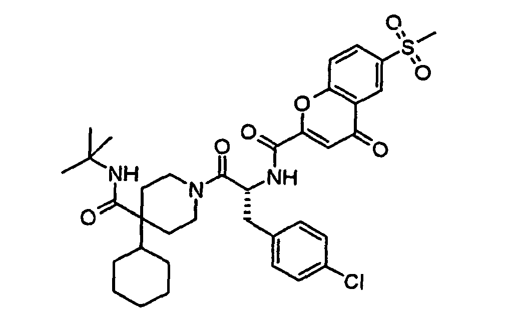

### Example 92:

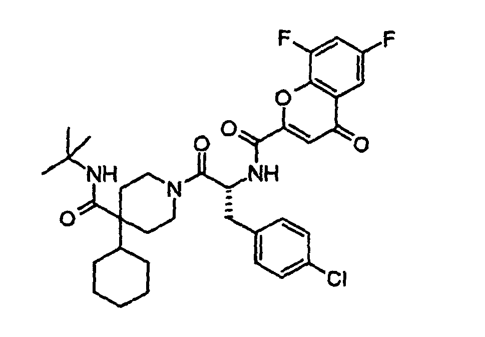

### Example 93:

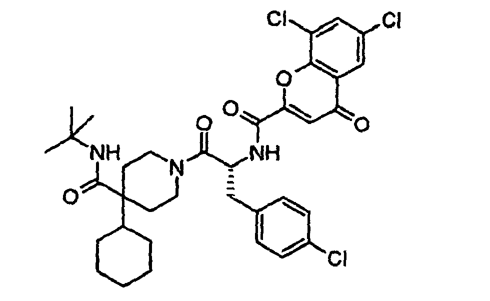

### Example 94:

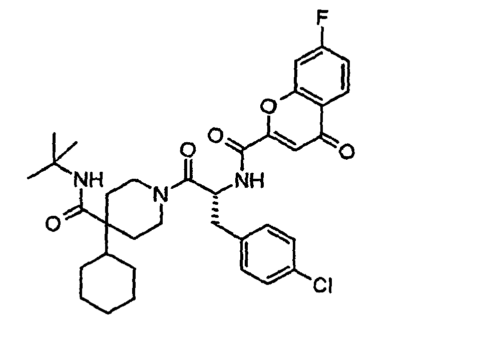

### Example 95:

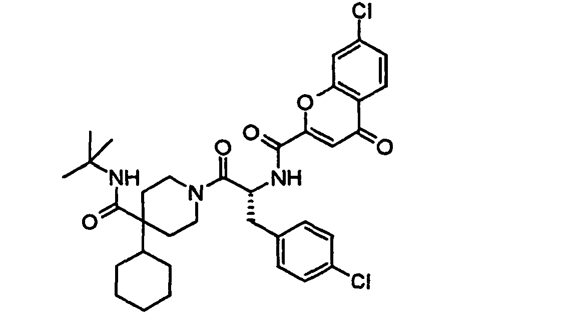

### Example 96:

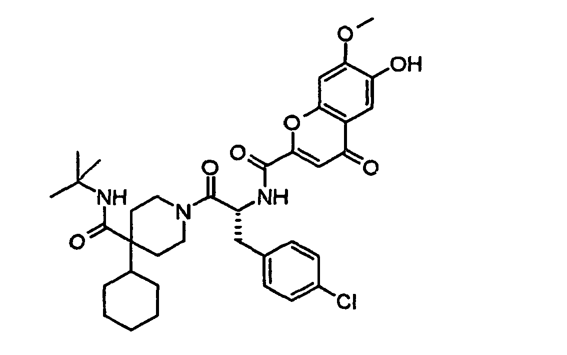

### Example 97:

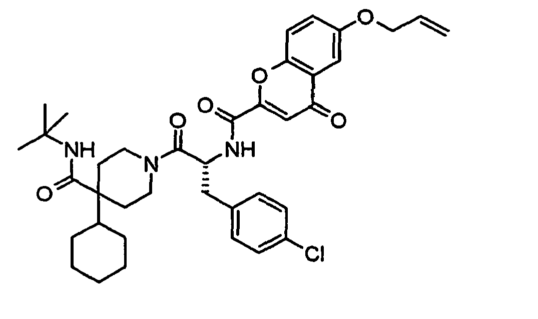

### Example 98:

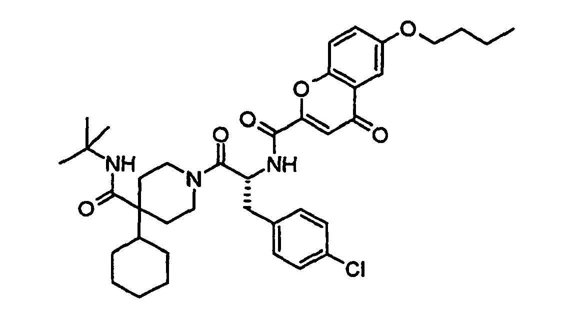

### Example 99:

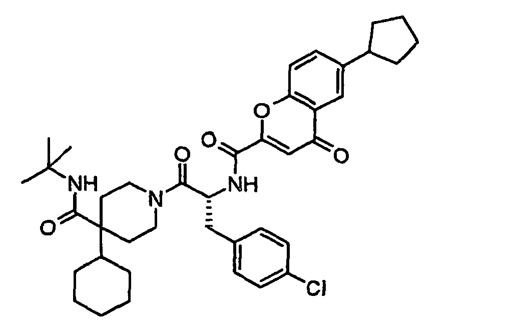

### Example 100:

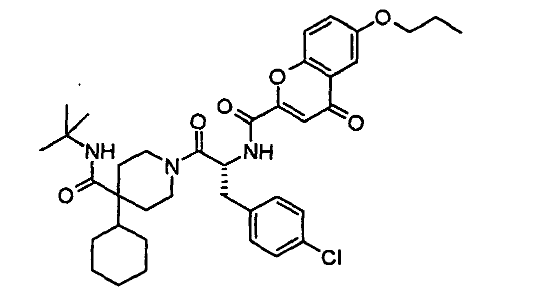

### Example 101:

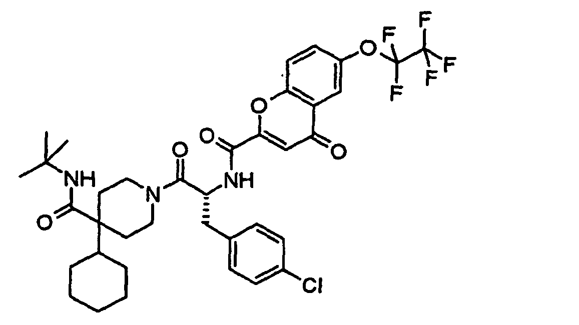

### Example 102:

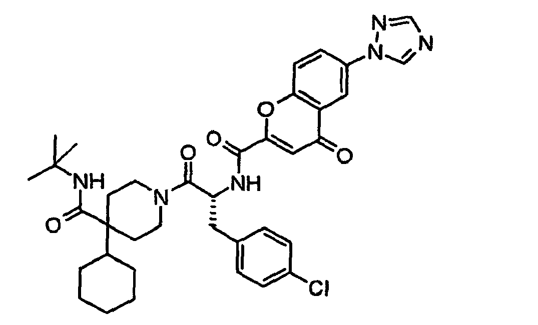

### Example 103:

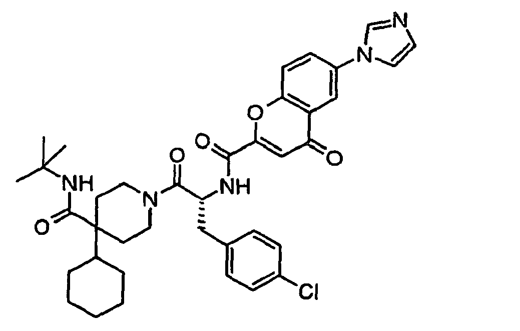

### Example 104:

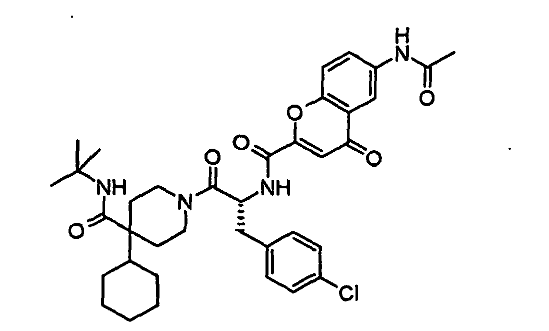

### Example 105:

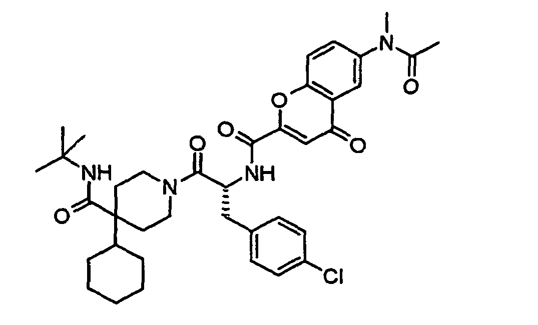

### Example 106:

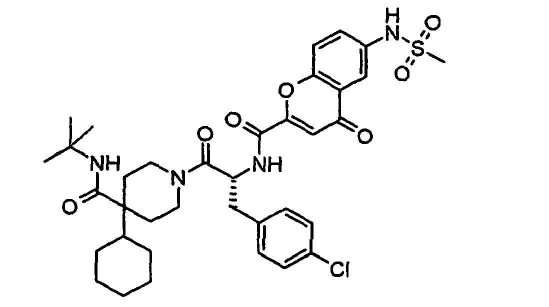

### Example 107:

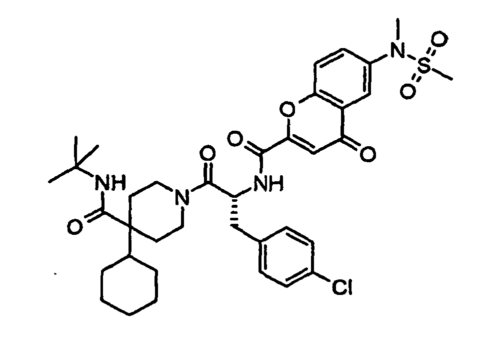

### Example 108:

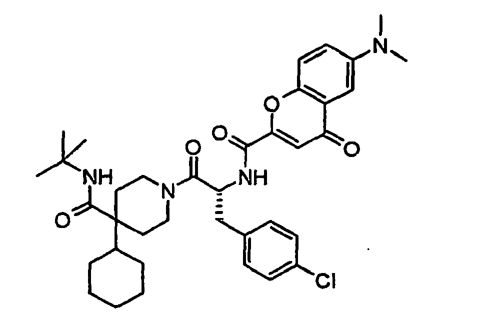

### Example 109:

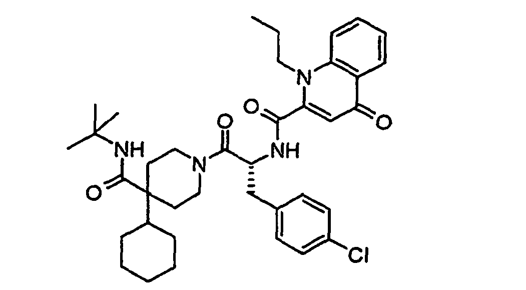

yellow solid
R_{f} = 0.79 (EtOAc).

### Example 110:

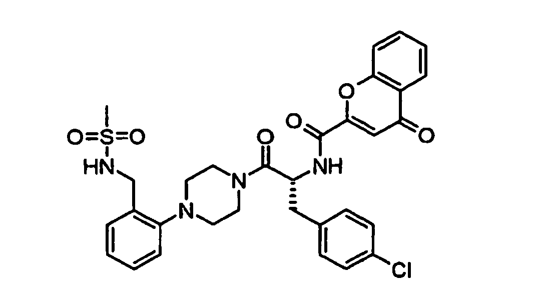

white solid
R_{f} = 0.63 (EtOAc); Mp. 160 - 174 °C.

### Example 111:

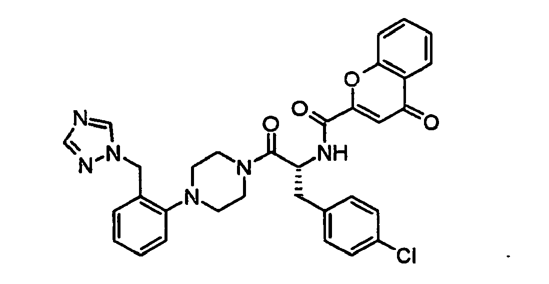

white solid
R_{f} = 0.72 (EtOAc/ethanol 3:1); Mp. 113 - 131 °C.

### Example 112:

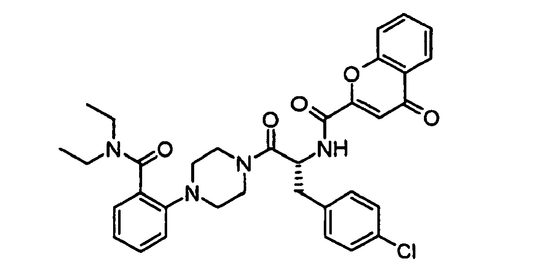

white solid
R_{f} = 0.85 (EtOAc); Mp. 131 - 139 °C.

### Example 113:

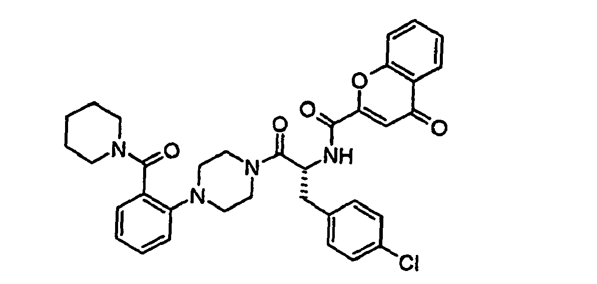

white solid
R_{f} = 0.77 (EtOAc/ethanol 3: 1); Mp. 135 - 141 °C.

### Example 114:

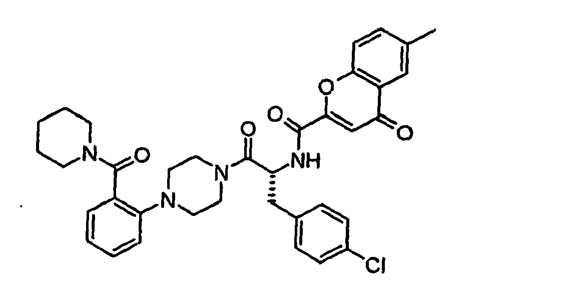

white solid
R_{f} = 0.32 (EtOAc); Mp. 141 - 147 °C.

### Example 115:

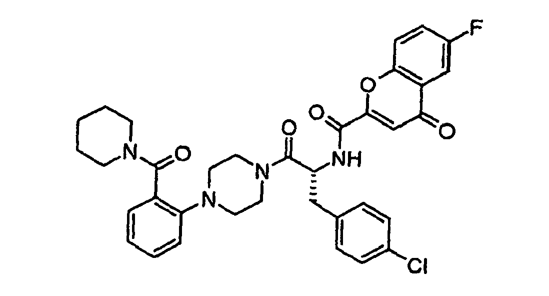

white solid
R_{f} = 0.30 (EtOAc); Mp. 129 - 145 °C.

### Example 116:

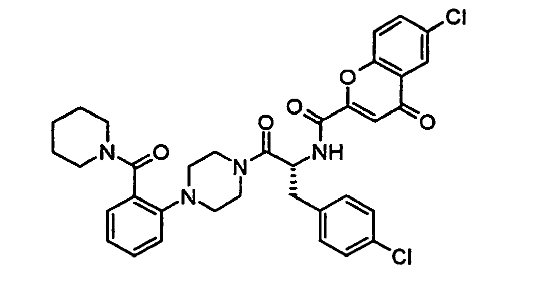

white solid
R_{f} = 0.39 (EtOAc); Mp. 210 - 215 °C.

### Example 117:

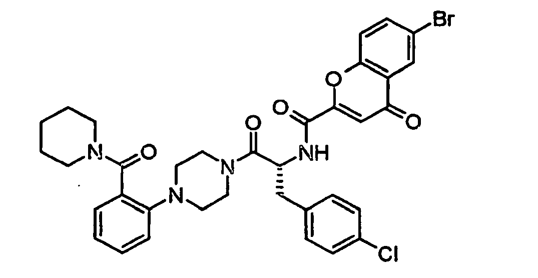

white solid
R_{f} = 0.44 (EtOAc/ethanol 3:1); Mp. 148 - 154 °C.

### Example 118:

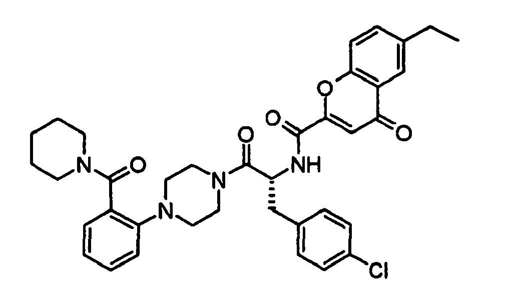

### Example 119:

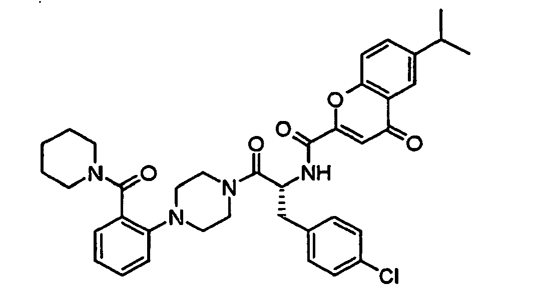

### Example 120:

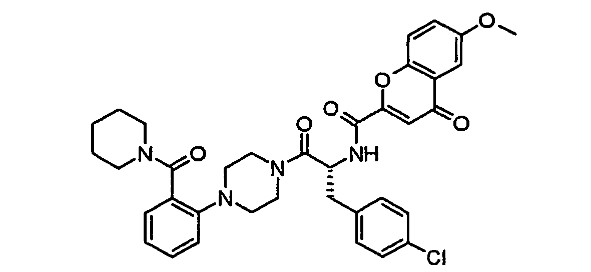

white solid
R_{f} = 0.31 (EtOAc); Mp. 141 - 145 °C.

### Example 121:

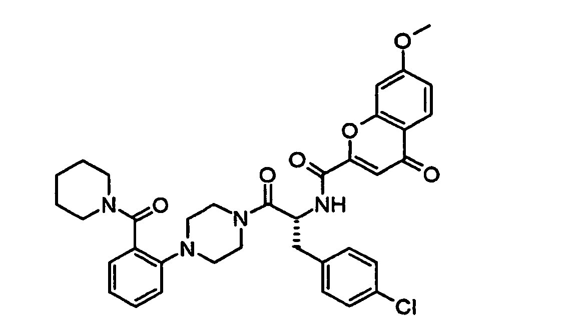

white solid
R_{f} = 0.22 (EtOAc); Mp. 150 - 153 °C.

### Example 122:

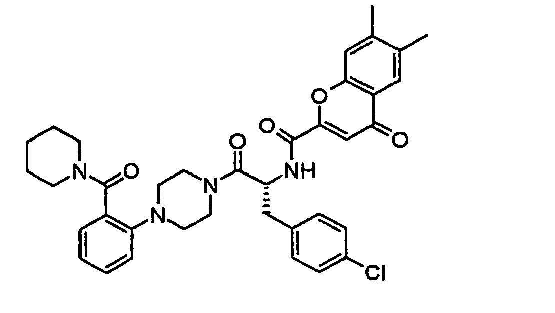

white solid
R_{f} = 0.36 (EtOAc); Mp. 134 - 152 °C.

### Example 123:

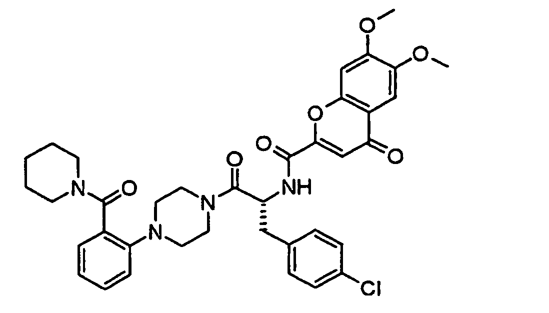

white solid
R_{f} = 0.11 (EtOAc); Mp. 140 - 152 °C.

### Example 124:

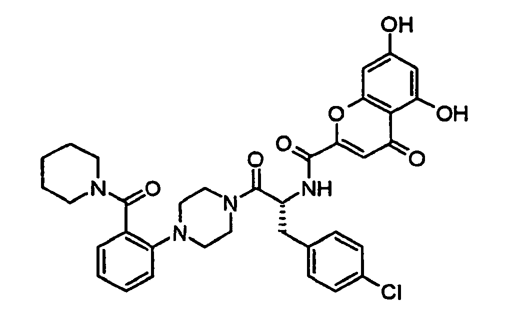

beige solid
R_{f} = 0.30 (EtOAc); Mp. 163 - 171 °C.

### Example 125:

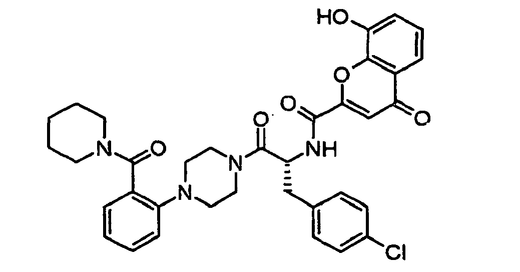

### Example 126:

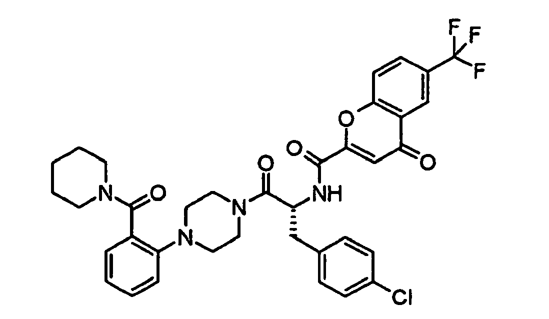

white solid
R_{f} = 0.44 (EtOAc); Mp. 139 - 144 °C.

### Example 127:

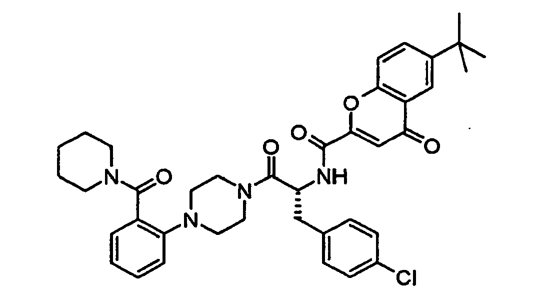

### Example 128:

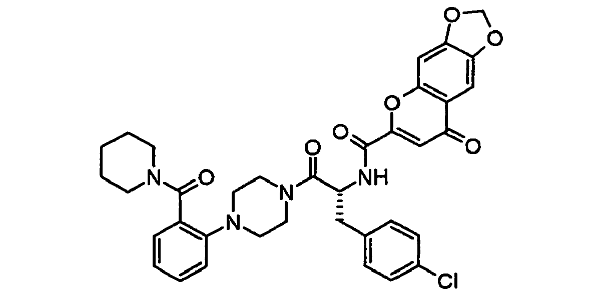

### Example 129:

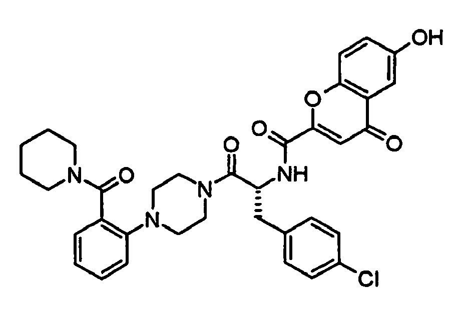

### Example 130:

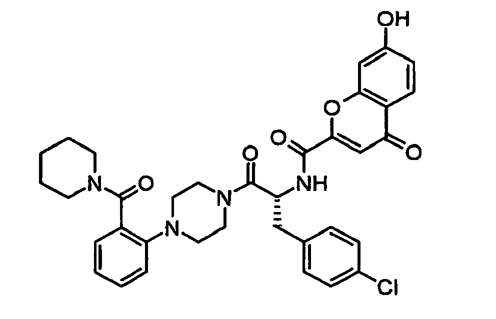

### Example 131:

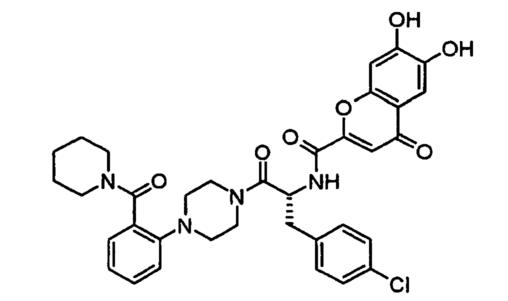

### Example 132:

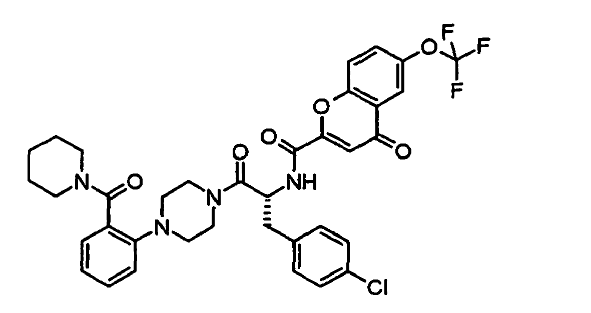

white solid
R_{f} = 0.47 (EtOAc); Mp. 130 - 135 °C.

### Example 133:

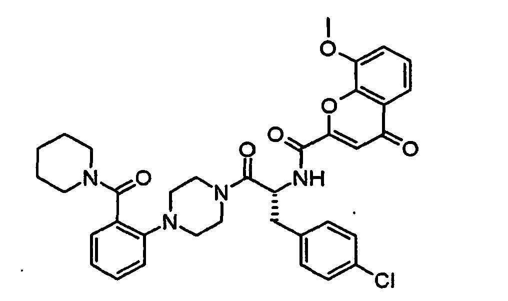

white solid
R_{f} = 0.18 (EtOAc); Mp. 143 - 150 °C.

### Example 134:

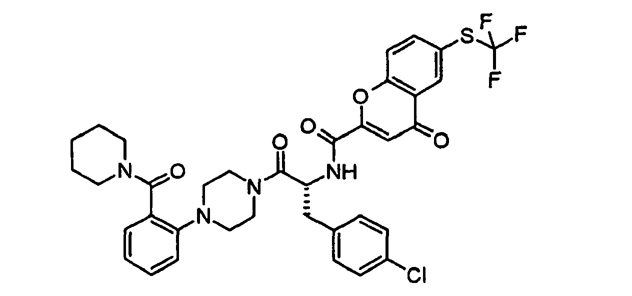

### Example 135:

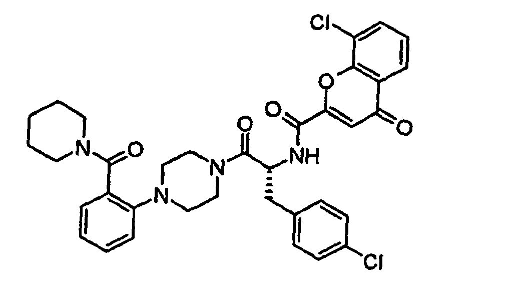

### Example 136:

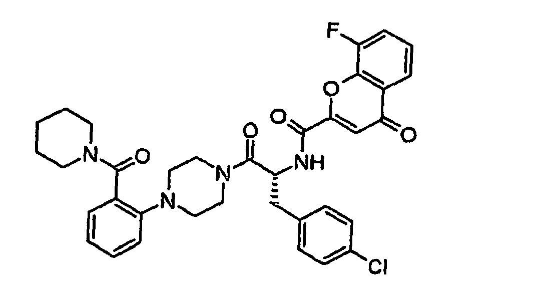

### Example 137:

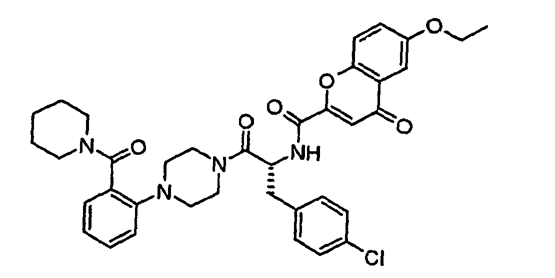

### Example 138:

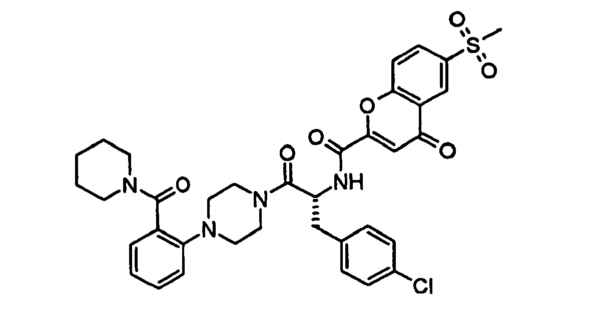

### Example 139:

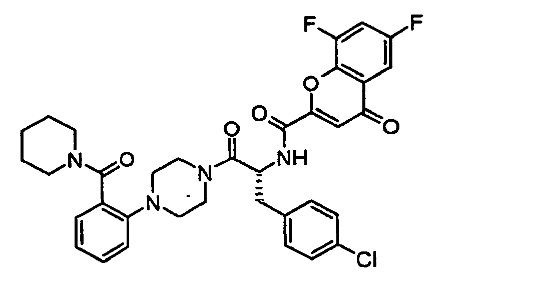

### Example 140:

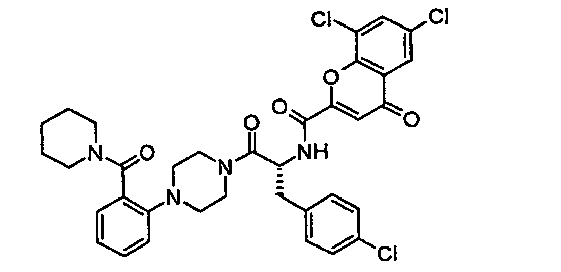

### Example 141:

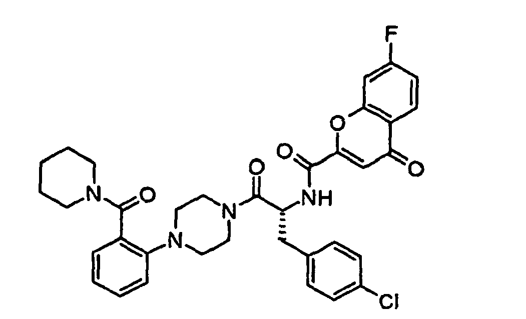

### Example 142:

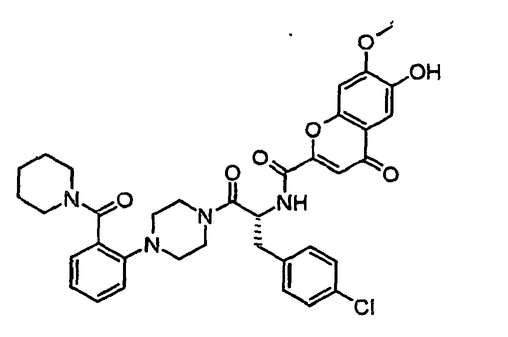

### Example 143:

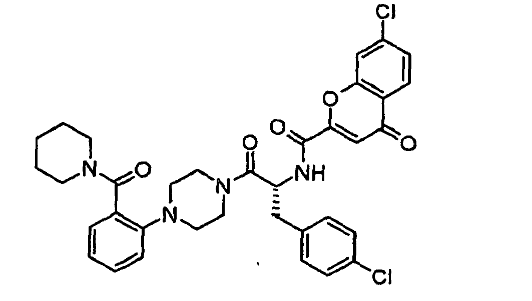

### Example 144:

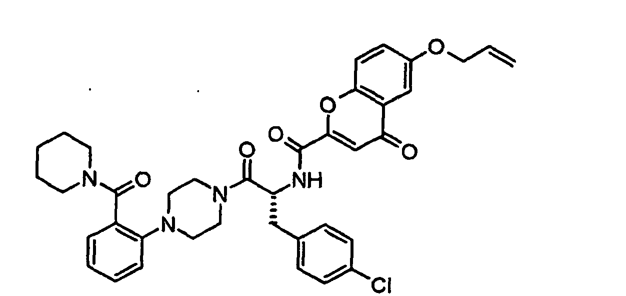

### Example 145:

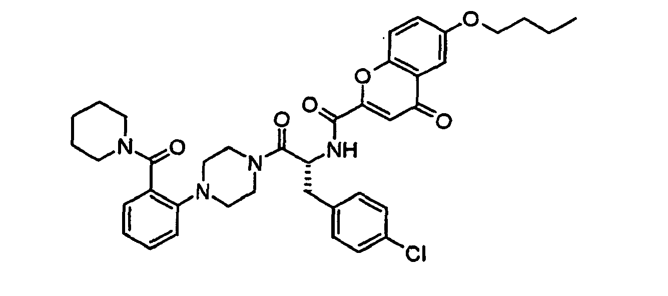

### Example 146:

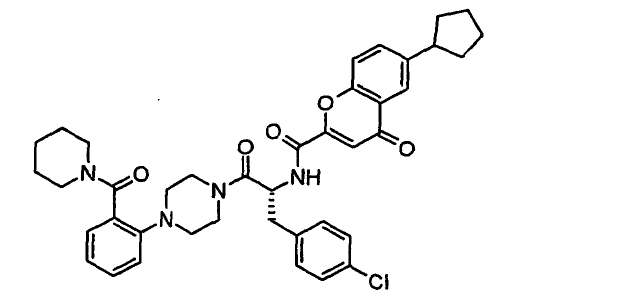

### Example 147:

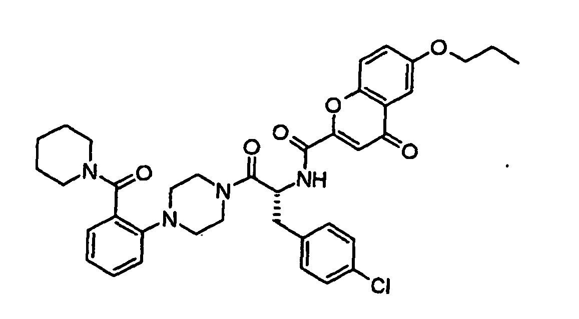

### Example 148:

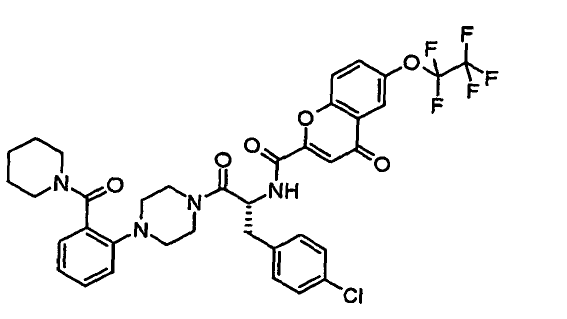

### Example 149:

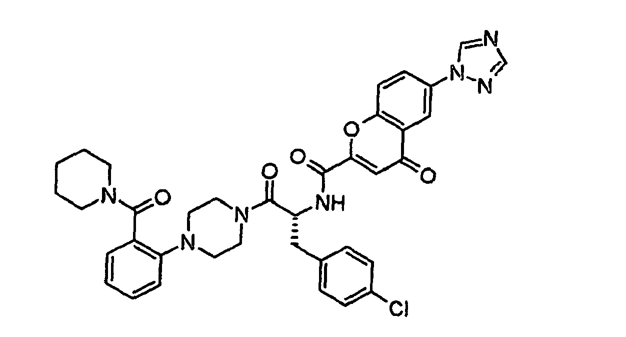

### Example 150:

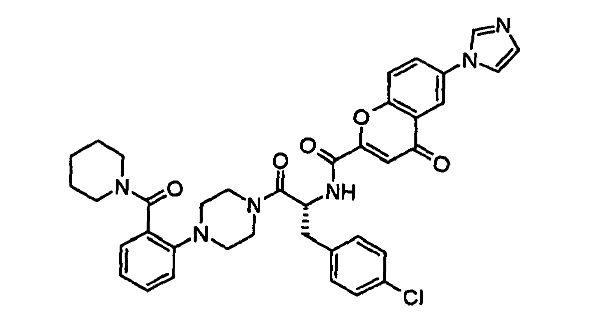

### Example 151:

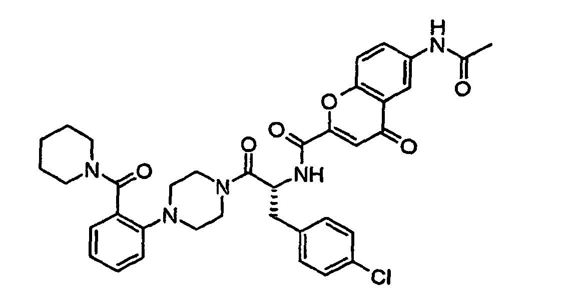

### Example 152:

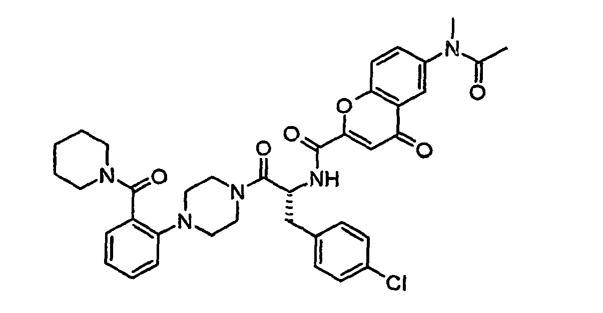

### Example 153:

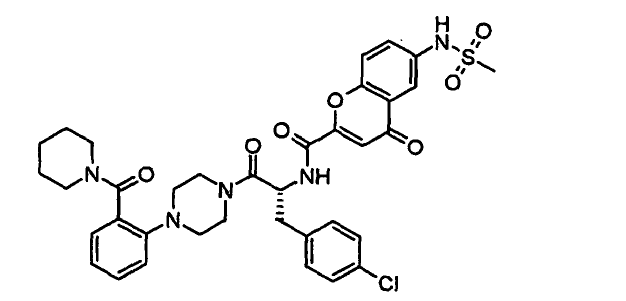

### Example 154:

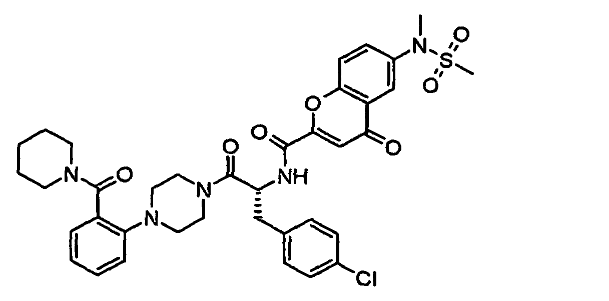

### Example 155:

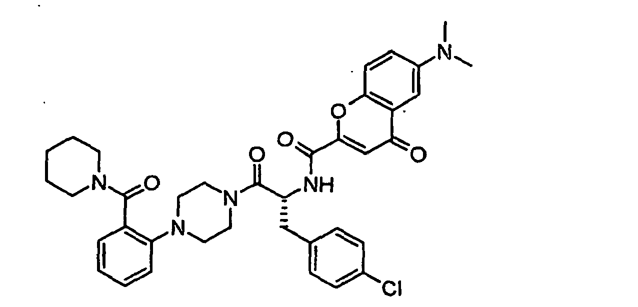

### Example 156:

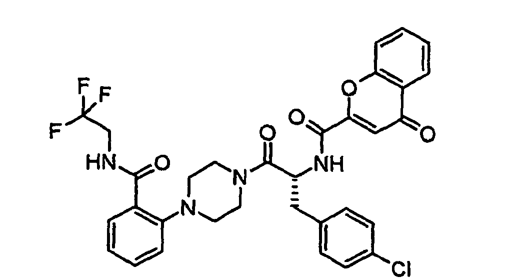

white solid
R_{f} = 0.51 (EtOAc); Mp. 125 - 136 °C.

### Example 157:

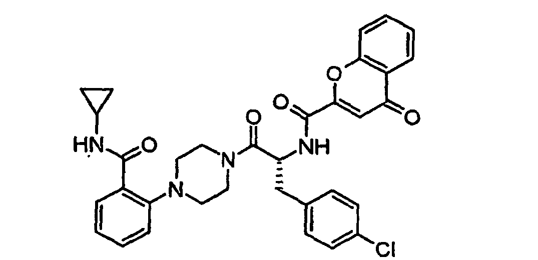

white solid
R_{f} = 0.45 (EtOAc); Mp. 170 - 188 °C.

### Example 158:

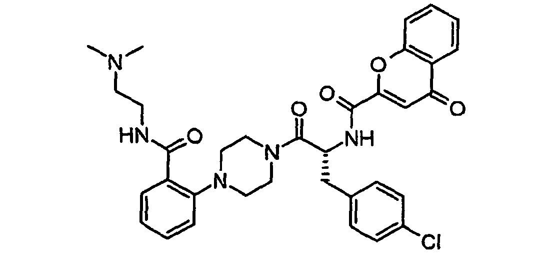

white solid
R_{f} = 0.06 (EtOAc/ethanol 3:1); Mp. 160 - 165 °C.

### Example 159:

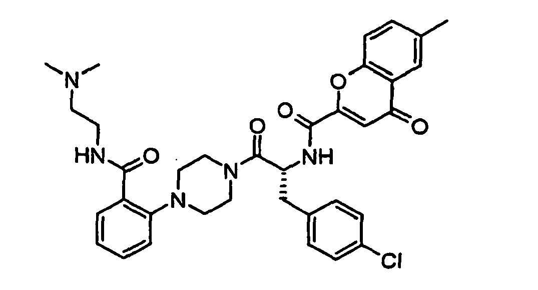

### Example 160:

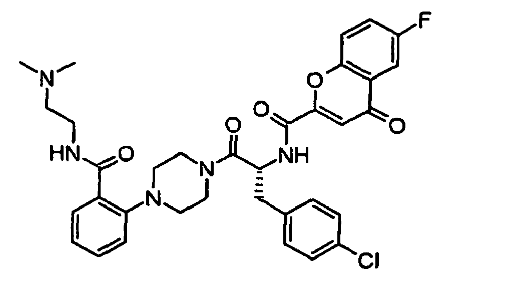

### Example 161:

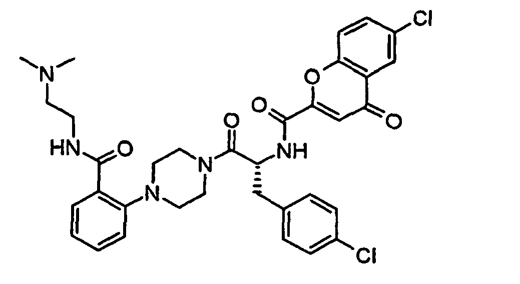

### Example 162:

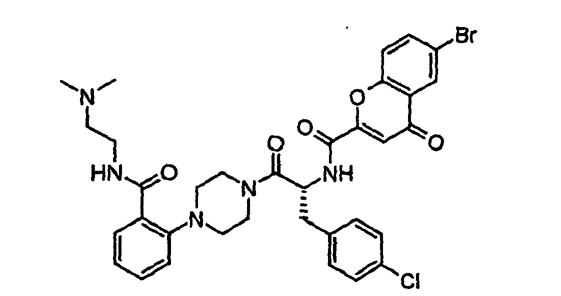

### Example 163:

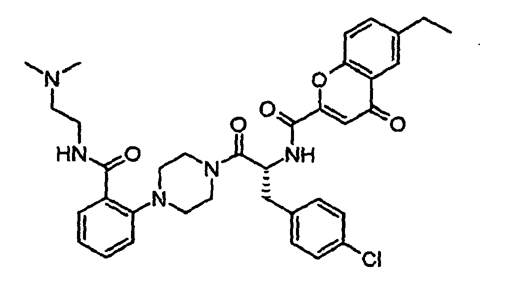

### Example 164:

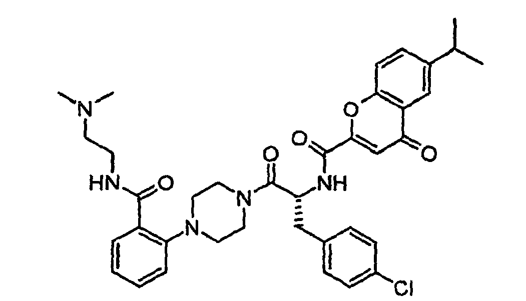

### Example 165:

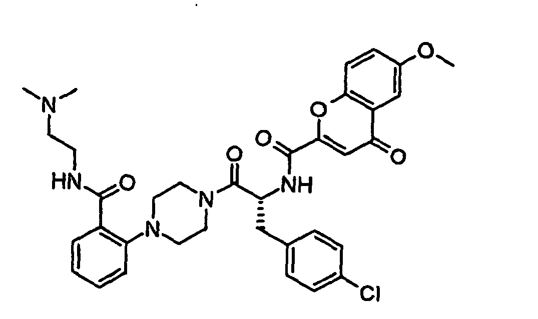

### Example 166:

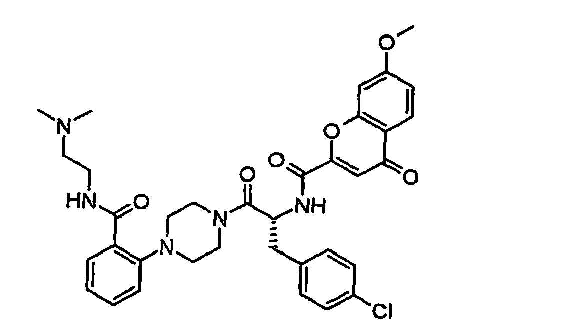

### Example 167:

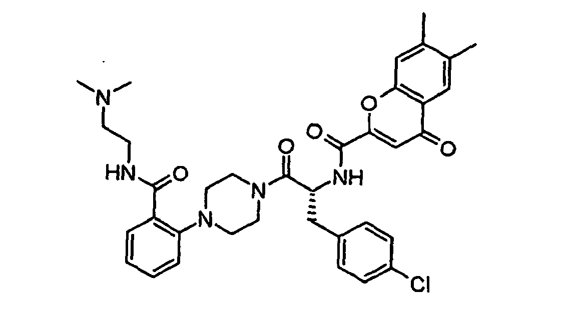

### Example 168:

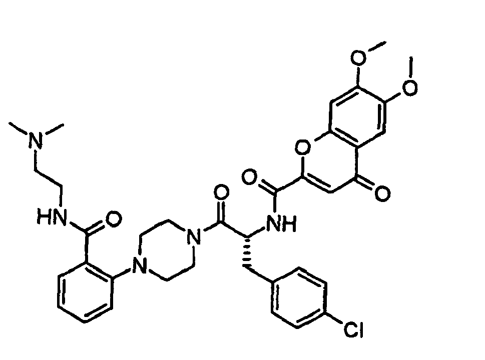

### Example 169:

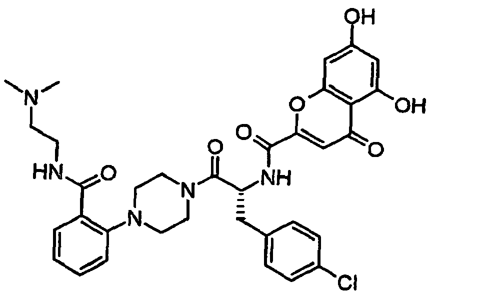

### Example 170:

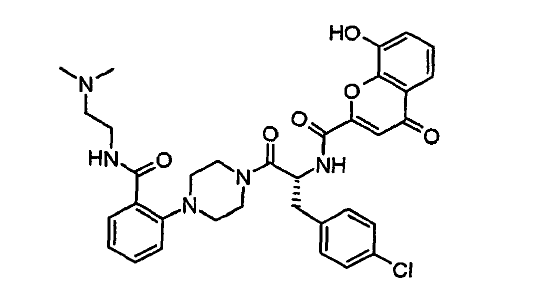

### Example 171:

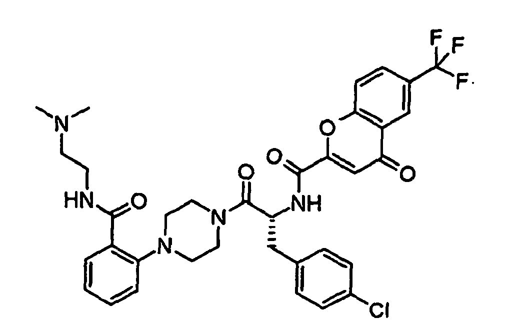

### Example 172:

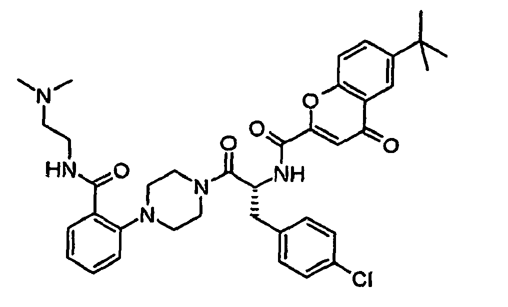

### Example 173:

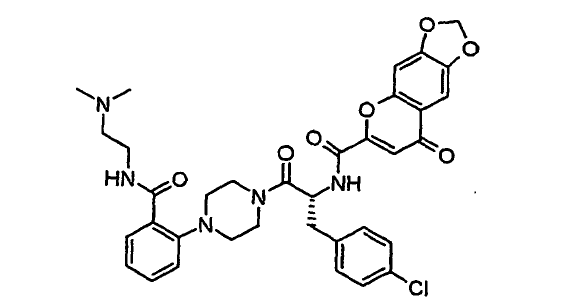

### Example 174:

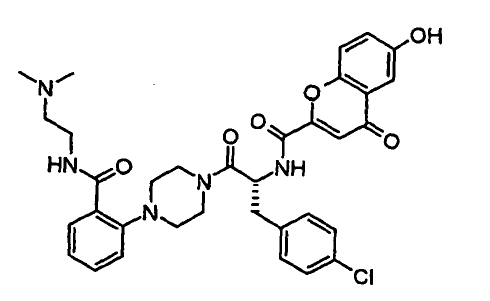

### Example 175:

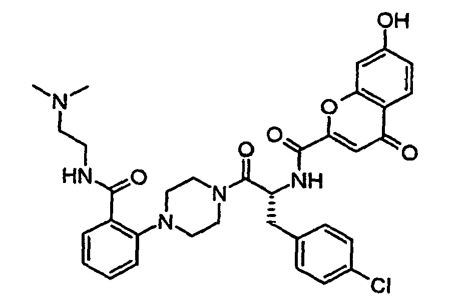

### Example 176:

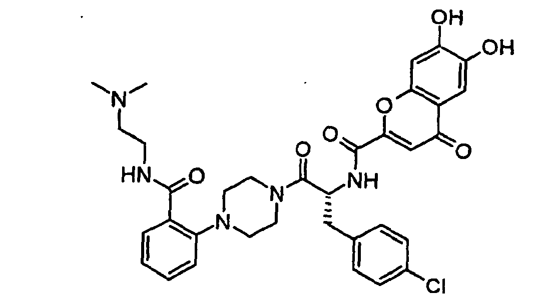

### Example 177:

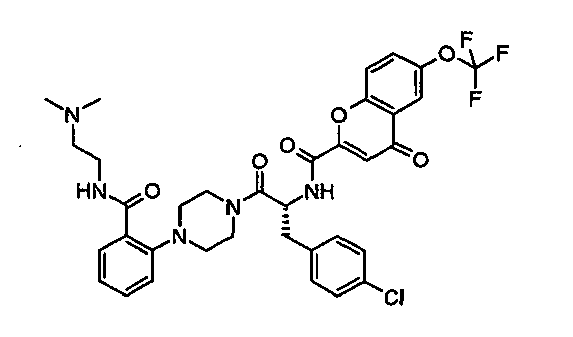

### Example 178:

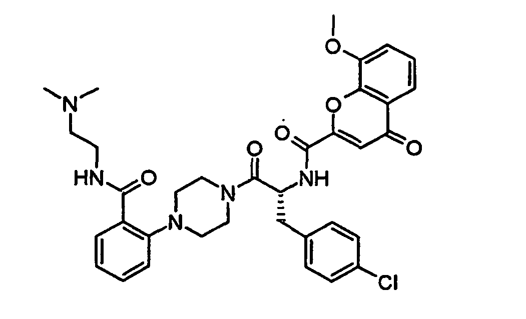

### Example 179:

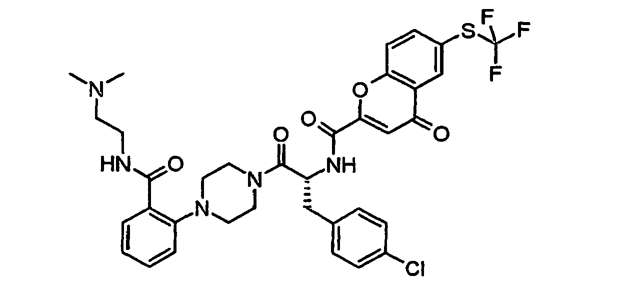

### Example 180:

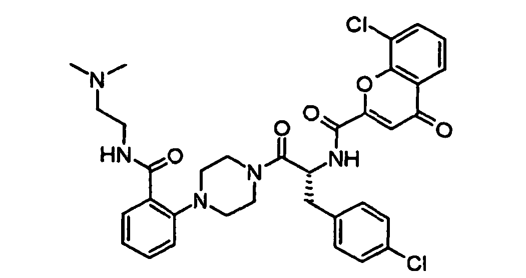

### Example 181:

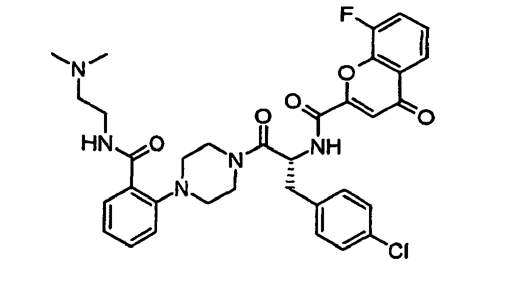

### Example 182:

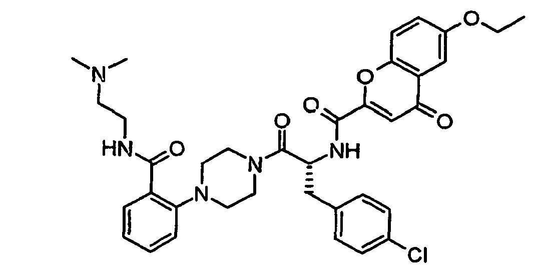

### Example 183:

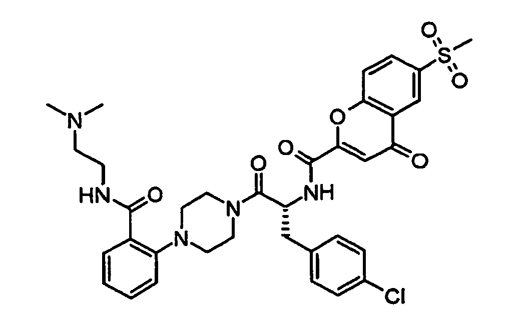

### Example 184:

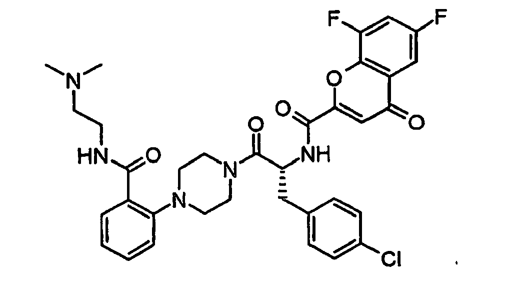

### Example 185:

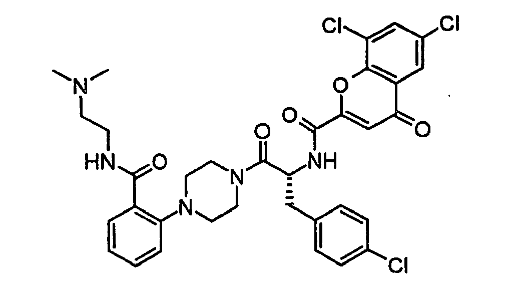

### Example 186:

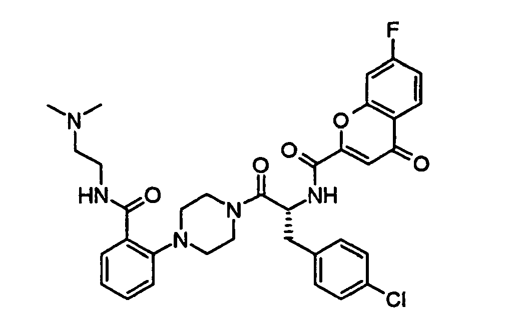

### Example 187:

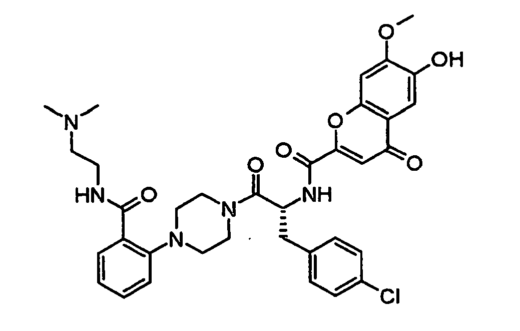

### Example 188:

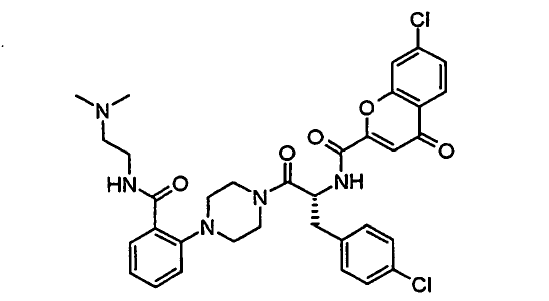

### Example 189:

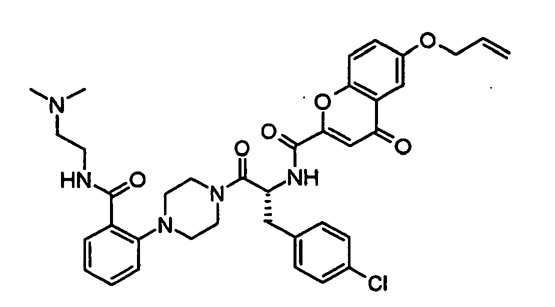

### Example 190:

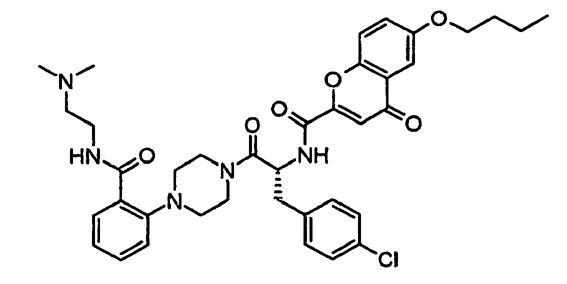

### Example 191:

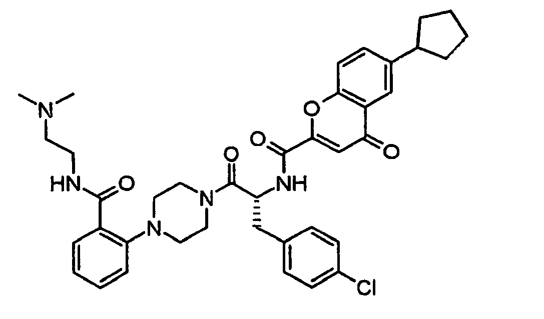

### Example 192:

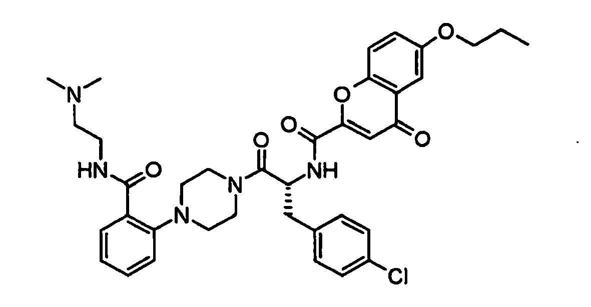

### Example 193:

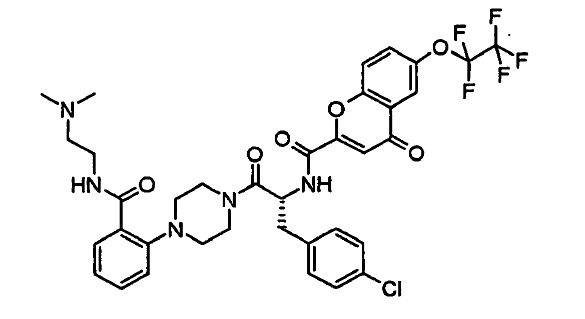

### Example 194:

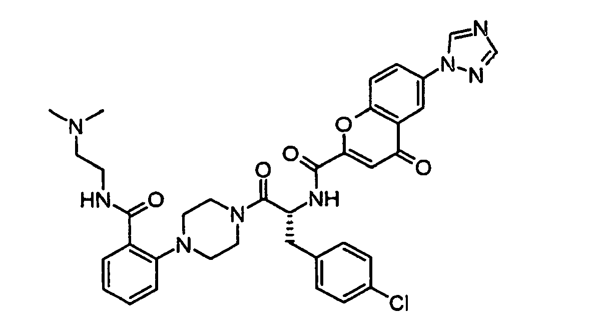

### Example 195:

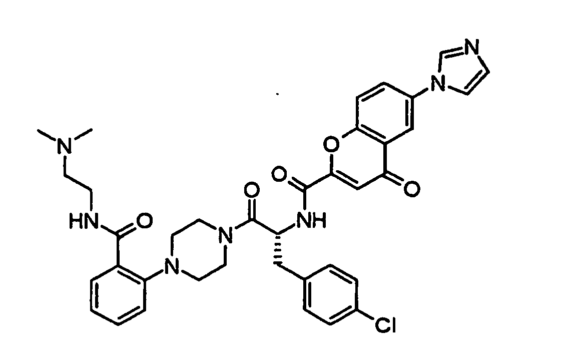

### Example 196:

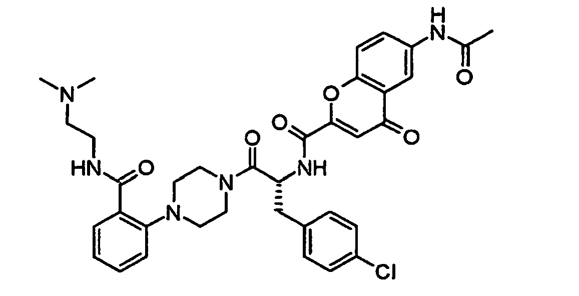

### Example 197:

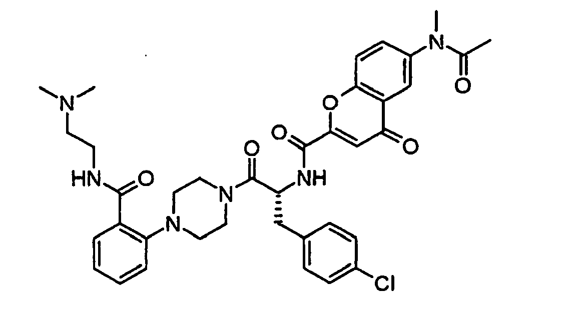

### Example 198:

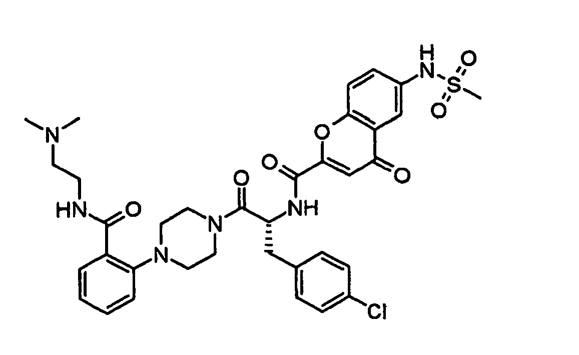

### Example 199:

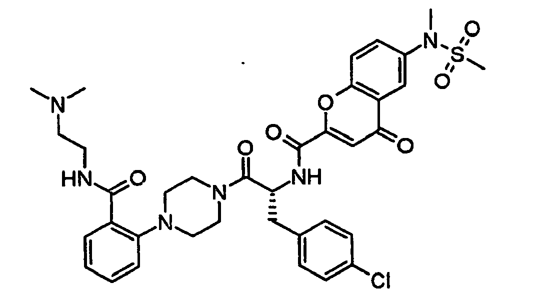

### Example 200:

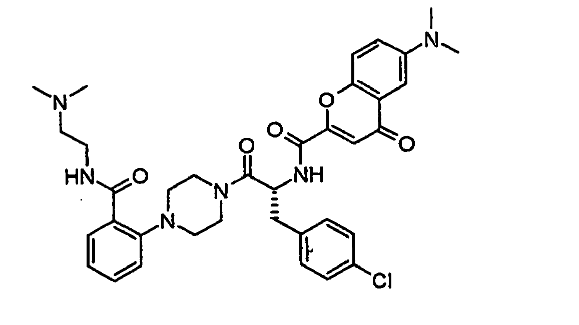

### Example 201:

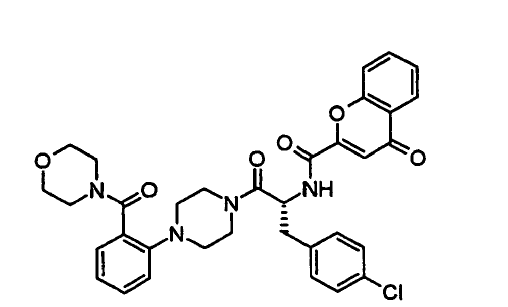

white solid
R_{f} = 0.45 (EtOAc); Mp. 196 - 206 °C.

### Example 202:

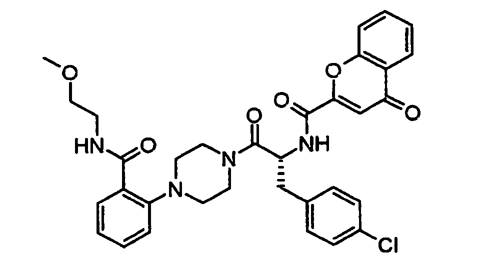

colorless glassy solid
R_{f} = 0.69 (EtOAc/ethanol 3:1); Mp. 100 - 103 °C.

### Example 203:

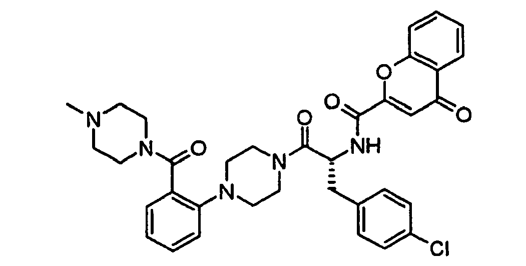

colorless glassy solid
R_{f} = 0.06 (EtOAc/ethano) 3:1); Mp. 96 - 99 °C.

### Example 204:

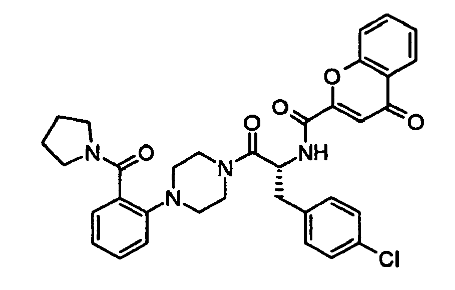

white solid
R_{f} = 0.66 (EtOAc/ethanol 3:1); Mp. 138 - 147 °C.

### Example 205:

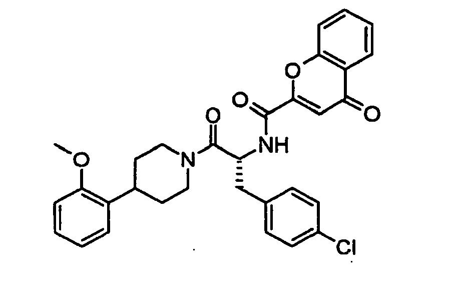

white solid
R_{f} = 0.86 (EtOAc/ethanol 3:1); Mp. 119 - 124 °C.

### Example 206:

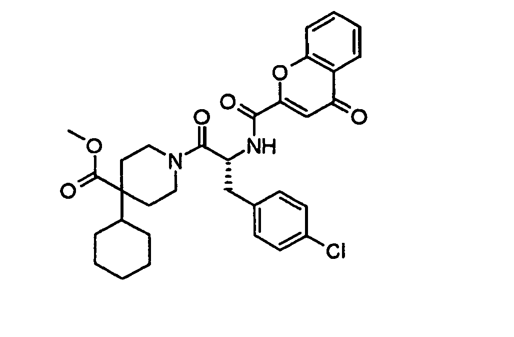

white solid
R_{f} = 0.28 (EtOAc/hexane 1:1); Mp. 152 - 155 °C.

### Example 207:

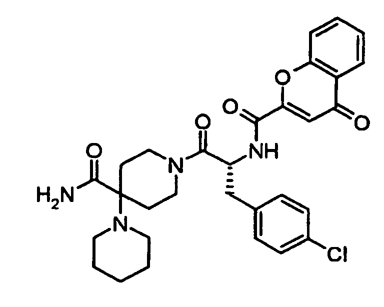

white solid
R_{f} = 0.68 (EtOAc/ethanol 3:1); Mp. 144 - 152 °C.

### Example 208:

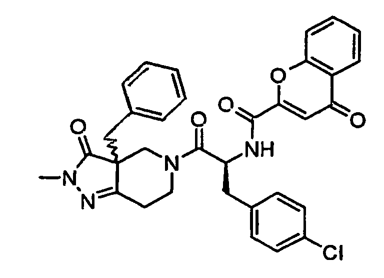

white solid
R_{f} = 0.45 (EtOAc); Mp. 134 - 146 °C.

### Example 209:

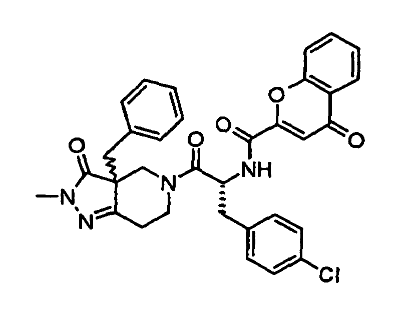

white solid
R_{f} = 0.45 (EtOAc); Mp. 137 - 143 °C.

### Example 210:

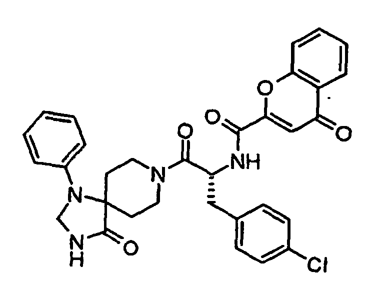

pale yellow solid
R_{f} = 0.70 (EtOAc/ethanol 3: 1); Mp. 144 - 153 °C.

### Example 211:

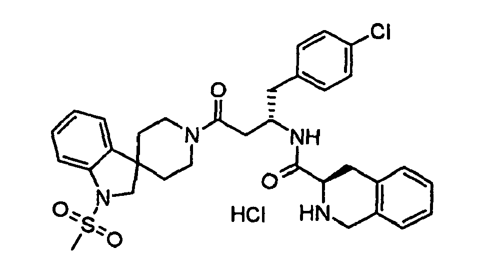

To Boc-protected intermediate 211c) (32 mg) was added hydrogen chloride, 4.0 M sol. in 1,4-dioxane (10 ml) and the solution was stirred for 90 min at room temperature. The solvent was removed under reduced pressure. The residue was dissolved in DCM and treated with diethyl ether. The precipitate was filtered off to yield the title compound as a solid.
white solid
Mp. 160 - 175 °C.

The required intermediates can be synthesized in the following way:

### Intermediate 211a):

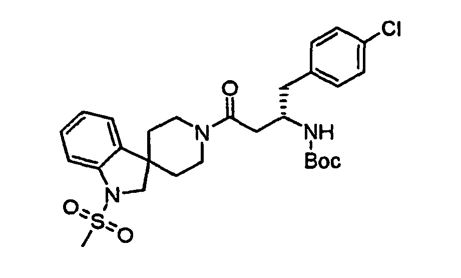

To Boc-(S)-3-amino-(4-chloro-phenyl)-butyric acid (170 mg) in DCM (6 ml) was added 1,2-dihydro-1-methanesulfonylspiro-[3H-indole-3,4'-piperidine] hydro-chloride (179 mg), N-methylmorpholine (83 µl), HOBt (140 mg) and stirred for 25 min. EDC (144 mg) was added and stirring was continued for 1 h. An additional amount of N-methylmorpholine (30 µl) was added and stirred overnight. The reaction mixture was poured into water (5 ml) and the organic phase was separated. The aqueous phase was extracted two times with DCM. The combined organic phases were washed with 0.5 N HCl and saturated sodium bicarbonate solution, dried over Na₂SO₄ and concentrated. The crude product was slurried in ethanol/ethyl acetate and filtrated to yield the desired compound after filtration.
white solid
R_{f} = 0.47 (EtOAc).

### Intermediate 211b):

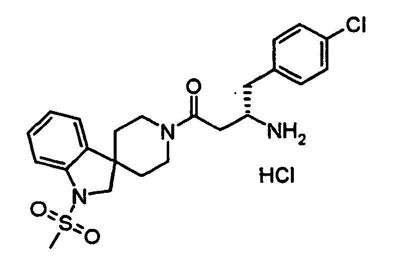

To intermediate 211a) (210 mg) in DCM (5 ml) was added TFA (1 ml) and stirred at room temperature for 2.5 h. Additional TFA (1 ml) was added and stirred for 1 h. The reaction mixture was diluted with DCM (5 ml) and carefully basified by pouring it into 10% aqueous sodium carbonate solution (10 ml). The organic layer was separated and the aqueous layer was further extracted twice with DCM. The combined organics were washed with water and brine, dried over Na₂SO₄ and concentrated to give colorless glassy solid.

The crude product was dissolved in DCM (5 ml) and app. 1 M HCl in ether (10 ml) was added. The precipitate was filtered and the residue was washed three times with ether and dried under reduced pressure to yield the title compound.
white solid
Mp. 160 - 175°C.

### Intermediate 211c):

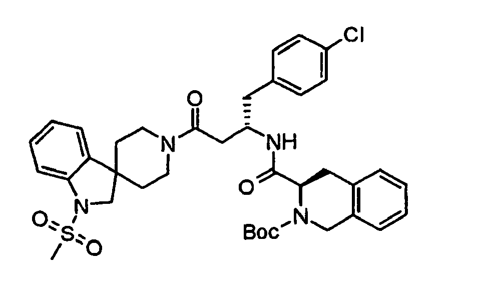

To intermediate 211b) (23 mg) and DCM (1 ml) was added (R)-Boc-1,2,3,4-tetrahydroisoquinoline-3-carboxylic acid (16 mg), HOBt (9 mg) and EDC (15 mg) and stirred overnight. The reaction mixture was poured into water (5 ml) and the organic layer was separated. The aqueous layer was extracted twice with DCM. The combined organics were washed with 0.5 N HCl (5 ml) and saturated sodium bicarbonate solution, dried over Na₂SO₄ and concentrated. The crude product was purified by column chromatography.
colorless solid
R_{f} = 0.76 (EtOAc/ethanol 3:1).

The following examples can be prepared in a similar way:

### Example 212:

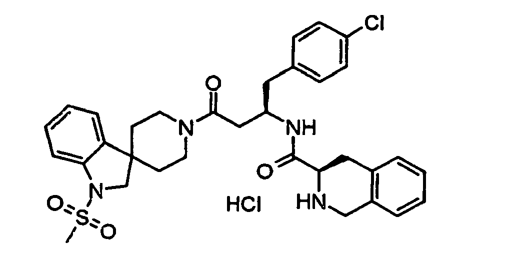

white solid
Mp. 162 - 177 °C.

### Example 213:

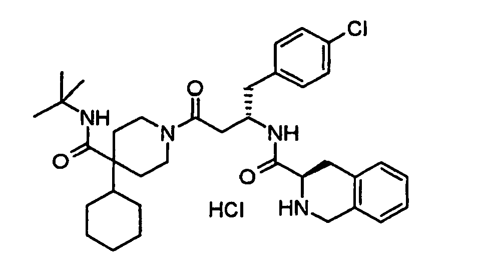

white solid
Mp. 192 - 200 °C.

### Example 214:

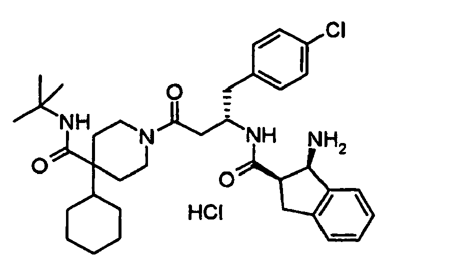

white solid
Mp. 189 - 201 °C.

### Example 215:

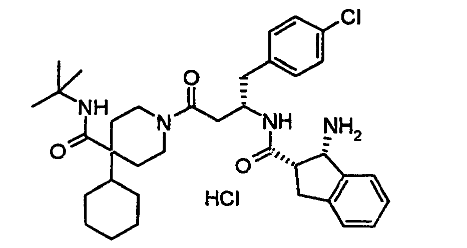

white solid
Mp. 161 - 170 °C.

### Example 216:

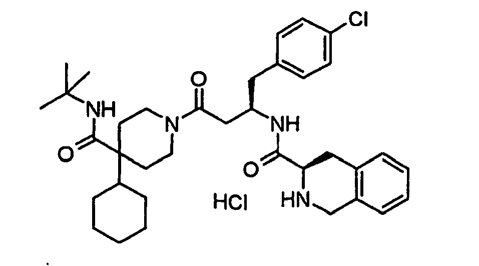

white solid
Mp. 180 - 185 °C.

### Example 217:

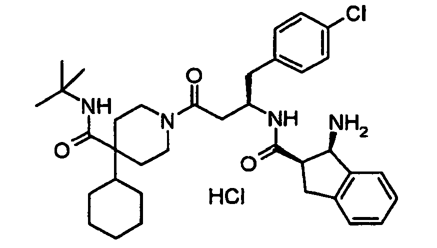

white solid
Mp. 190 - 199 °C.

### Example 218:

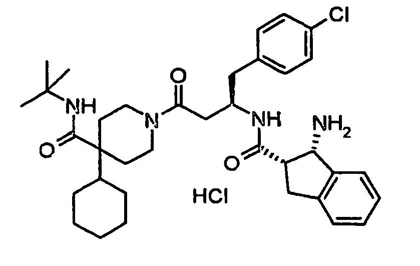

white solid
Mp. 198 - 206 °C.

### Example 219:

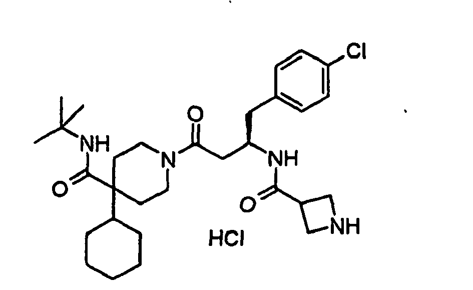

white solid
Mp. 207 - 238 °C.

### Example 220:

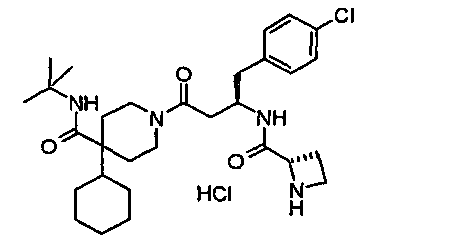

white solid
Mp. 225 - 253 °C.

### Example 221:

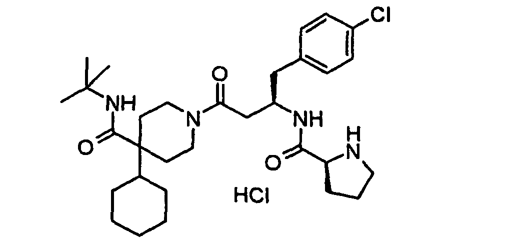

white solid
Mp. 176 - 187 °C.

### Example 222:

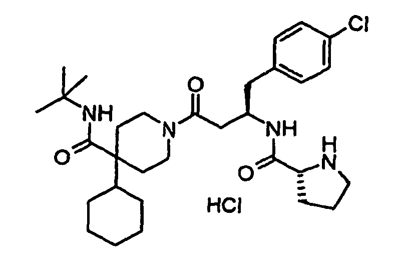

white solid
Mp. 148 °C.

### Example 223:

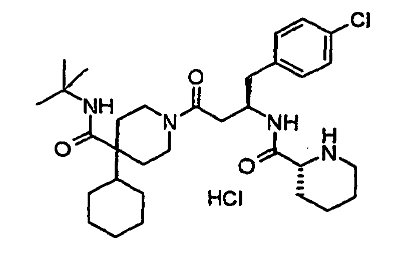

white solid
Mp. 150 - 155 °C.

### Example 224:

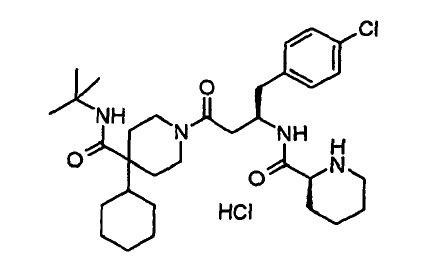

white solid
Mp. 150 - 156 °C.

### Example 225:

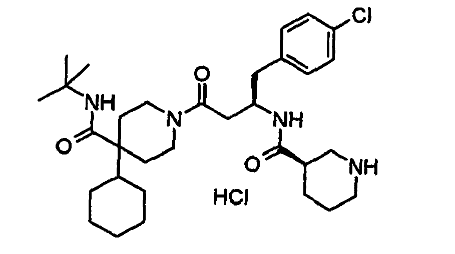

white solid
Mp. 125 - 131 °C.

### Example 226:

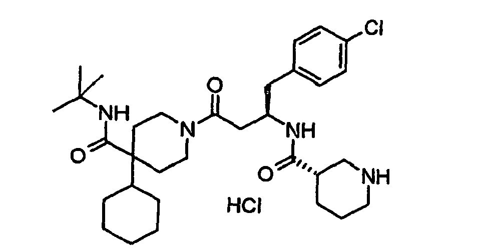

white solid
Mp. 154 - 161 °C.

### Example 227:

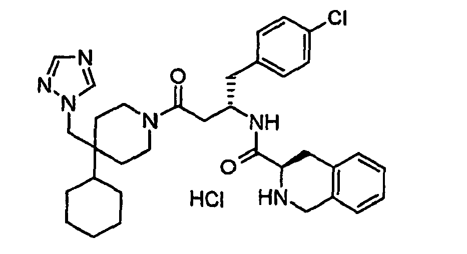

white solid
Mp. 179 - 184 °C.

### Example 228:

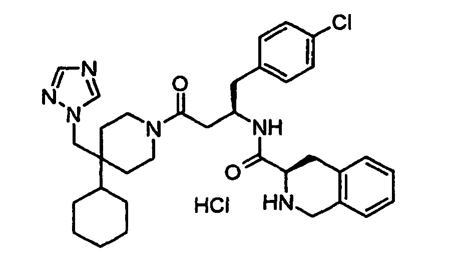

white solid
Mp. 178 - 186 °C.

### Example 229:

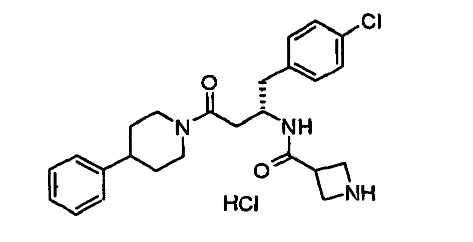

colorless glassy solid
Mp. 82 - 87 °C.

### Example 230:

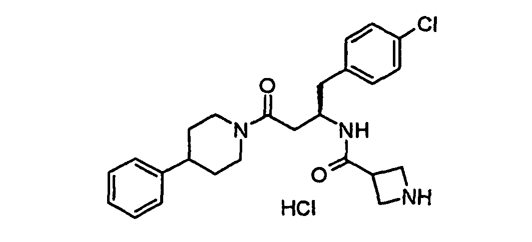

colorless glassy solid
Mp. 80 - 82 °C.

### Example 231:

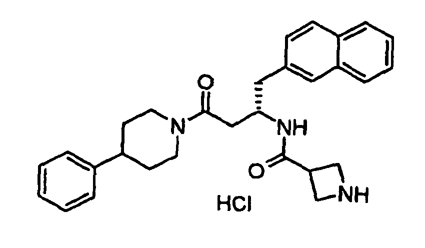

white solid
Mp. 117 - 125 °C.

### Example 234:

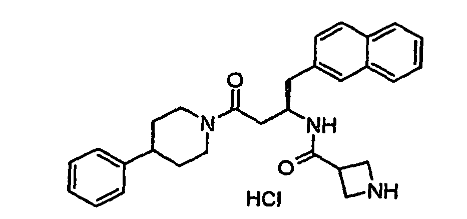

white solid
Mp. 118 - 127 °C.

### Preparation of the carboxylic acids:

### Synthesis Scheme for Carboxylic Acid 1

### Carboxylic Acid 1:

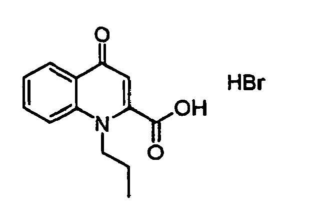

Intermediate CA1c) (0.1 g) was hydrolyzed by heating with hydrobromic acid (2 ml) and acetic acid (1.5 ml) for 3 h to give the desired compound after evaporation of the solvent.

### Intermediate CA1a):

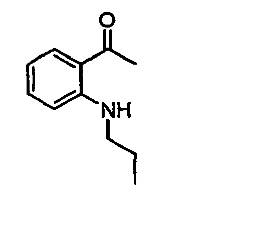

To a solution of 2'-aminoacetophenone (709 mg) in methanol (10 ml) was added propionaldehyde (580 µl) and TMOF (482 µl) and the solution was stirred overnight. The volatiles were removed under reduced pressure and the residue was redissolved in methanol (10 ml). Acetic acid (115 µl) and sodium cyanoborohydride (126 mg) were added and the reaction was stirred overnight. After basification with 1 M NaOH the volatiles were removed under reduced pressure. The residue was dissolved in ethyl acetate and washed with water and brine. The organic phase was dried over Na₂SO₄ and the solvent was removed under reduced pressure to yield the title compound.

### Intermediate CA1b):

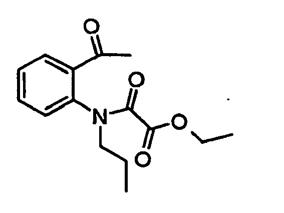

To a solution of intermediate CA1a) (681 mg) in dry THF (10 ml) was added TEA (670 µl) and the mixture was cooled to 0°C. At this temperature, ethyl oxalyl chloride (470 µl) was added dropwise. The reaction mixture was stirred for 4 h at room temperature. The volatiles were removed under reduced pressure and the residue was dissolved in ethyl acetate. The solution was washed with water and sat. NaHCO₃ and brine and dried over Na₂SO₄ to yield the desired compound.

### Intermediate CA1c):

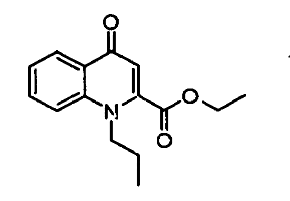

Intermediate CA1b) (555 mg) was dissolved in ethanol (10 ml). K₂CO₃ (276 mg) was added and the reaction mixture was stirred overnight. The reaction mixture was filtrated and the solvent was removed to yield the title compound.

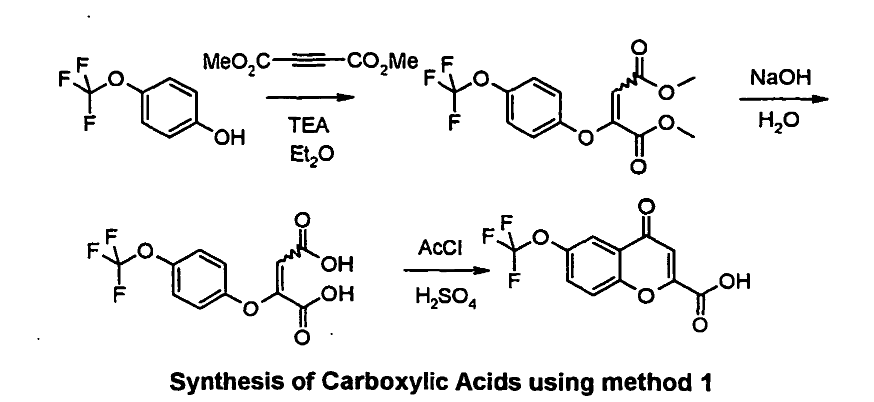

### Carboxylic acid 2:

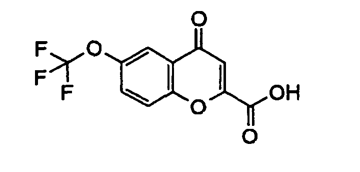

Intermediate CA2b) (5.85 g) was suspended in AcCl (110 ml) and concentrated sulfuric acid (4.40 ml) was added while stirring at RT. Then the slightly yellowish reaction mixture was heated to reflux with vigorous stirring and kept under reflux for 30 min. The reaction mixture was evaporated in vacuo to a volume of ca. 25 ml and then slowly and carefully added to well stirred H₂O (300 ml) and stirring was continued for 1 h. After brief sonication, the formed precipitate was filtered off, washed with cold H₂O (3x30 ml), and finally dried *in vacuo* at 40 °C overnight. The crude product was dissolved in a minimal amount of boiling H₂O (270 ml) and left to slowly cool to RT. Crystallization was completed at RT for 6 h, then the crystalline product was filtered off and washed with cold H₂O (3x10 ml). Finally the product was dried *in vacuo* at 40 °C overnight to yield the title compound.

### Intermediate CA2a):

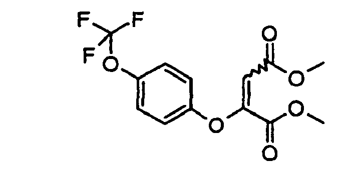

4-Trifluoromethoxyphenol (6.67 g) was dissolved in Et₂O (55 ml) and TEA (6.36 ml) was added while stirring at RT. Then dimethyl acetylendicarboxylate (5.12 ml) was added with vigorous stirring and the reaction mixture stirred at RT in the dark overnight. The reaction mixture was diluted with Et₂O (30 ml) and washed with 1 M HCl (3x65 ml), H₂O (30 ml), and brine (25 ml), dried with Na₂SO₄ and then evaporated *in vacuo.* Finally it was dried under high vacuum for 2 h to yield the desired product.

### Intermediate CA2b):

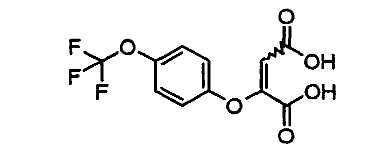

To intermediate CA2a) (9.57 g) was added a solution of NaOH (4.80 g) in water (45 ml) while stirring at RT. Then the reaction mixture was heated to reflux with vigorous stirring and kept under reflux for 3 h. The reaction mixture was extracted with Et₂O (100 ml) and then acidified to below pH 1 with conc. HCl while cooling in ice/H₂O. A white precipitate formed, which was filtered off, washed with H₂O (3x30 ml), and finally it was dried *in vacuo* at 40 °C overnight to give the desired compound.

The following chromone-2-carboxylic acids were prepared using method 1: 6-ethylchromone-2-carboxylic acid, 6-isopropylchromone-2-carboxylic acid, 6-methoxychromone-2-carboxylic acid, 6-trifluoromethylchromone-2-carboxylic acid, 6-tert.-butylchromone-2-carboxylic acid, 6-chlorochromone-2-carboxylic acid, 6-trifluoromethoxychromone-2-carboxylic acid, 8-methoxychromone-2-carboxylic acid, 6-trifluoromethylsulfanylchromone-2-carboxylic acid, 8-chlorochromone-2-carboxylic acid, 8-fluorochromone-2-carboxylic acid 7-chlorochromone-2-carboxylic acid, 6-ethoxychromone-2-carboxylic acid, 6-methanesulfonylchromone-2-carboxylic acid, 8-oxo-8H-[1,3]dioxolo[4,5-g]chromene-6-carboxylic acid, 6-allyloxy-4-hydroxy-4H-chromene-2-carboxylic acid, 6-butoxy-4-hydroxy-4H-chromene-2-carboxylic acid, 6-propoxy-4-hydroxy-4H-chromene-2-carboxylic acid, 6-cyclopentyl-4-oxo-4H-chromene-2-carboxylic acid, 6-pentafluoroethoxy-4-oxo-4H-chromene-2-carboxylic acid, 4-oxo-6-[1,2,4]triazol-1-yl-4H-chromene-2-carboxylic acid, 6-imidazol-1-yl-4-oxo-4H-chromene-2-carboxylic acid, 6-acetylamino-4-oxo-4H-chromene-2-carboxylic acid, 6-(acetyl-methyl-amino)-4-oxo-4H-chromene-2-carboxylic acid, 6-methanesulfonylamino-4-oxo-4H-chromene-2-carboxylic acid, 6-(methanesulfonyl-methyl-amino)-4-oxo-4H-chromene-2-carboxylic acid and 6-dimethylamino-4-oxo-4H-chromene-2-carboxylic acid..

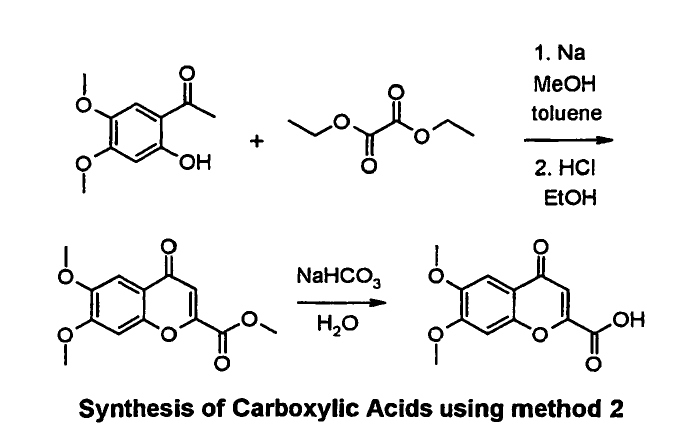

### Carboxylic acid 3:

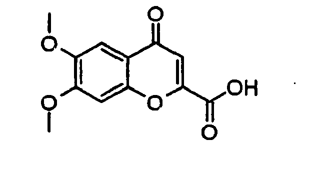

Intermediate CA3a) (2.65 g) was suspended in sat. sodium bicarbonate solution (50 ml) and heated to 80°C for 2 h. At the end of the reaction a clear solution was obtained. After cooling to room temperature the reaction mixture was acidified with HCl. The white precipitate was filtered off, washed with water and dried *in vacuo* at 40 °C overnight to give the title compound.

### Intermediate CA3a):

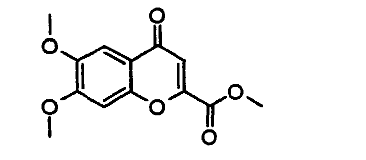

Sodium (4.0 g) was added to dry methanol (50 ml). After the conversion to the methoxide was complete the solution was cooled and a solution of 2'-hydroxy-4',5'-dimethoxyacetophenone (3.92 g) in diethyl oxalate (12 ml), methanol (50 ml) and toluene (50 ml) was added to it. The mixture was refluxed overnight. After cooling, diethyl ether (200 ml) was added. The sodium salt was filtered, washed with anhydrous ether, suspended in water and the solution acidified. The resultant precipitate was filtered and dried to yield a yellow solid. The intermediate was dissolved in ethanol (100 ml) and heated at 100°C for 15 min; concentrated HCl (2 ml) was added, and the solution stirred at 100°C for 1.5 h. Immediately after addition of the acid a precipitate was formed. After cooling to room temperature the reaction mixture was diluted with water (150 ml) and the pale yellow precipitate was filtered off and washed with water. The product was dried under reduced pressure.

The following chromone-2-carboxylic acids were prepared using method 2: 6-methoxychromone-2-carboxylic acid, 7-methoxychromone-2-carboxylic acid, 6,7-dimethylchromone-2-carboxylic acid, 6,7-dimethoxychromone-2-carboxylic acid, 6-chlorochromone-2-carboxylic acid, 6,8-difluorochromone-2-carboxylic acid, 6,8-dichlorochromone-2-carboxylic acid and 7-fluorochromone-2-carboxylic acid.

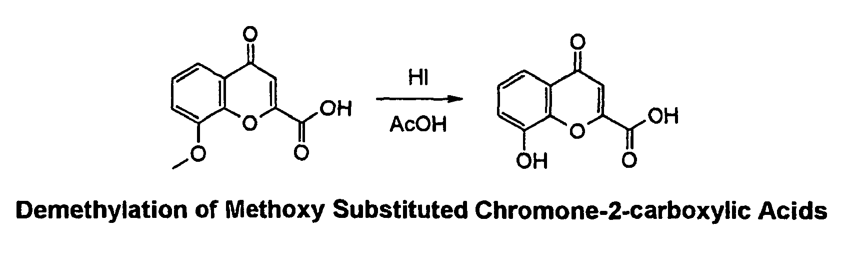

### Carboxylic acid 4:

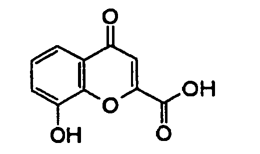

8-Methoxychromone-2-carboxylic acid (220 mg) was suspended in AcOH (2 ml) and conc. Hl (2 ml) was added while stirring at RT. Then the slightly yellowish suspension was heated to 120°C with vigorous stirring and kept at this temperature for 60 min. The warm reaction mixture was slowly and carefully added to well stirred H₂O (75 ml) and the resulting yellow solution was chilled in ice for 30 min. Crystallization was completed in the fridge for another 2 h. The formed crystalline precipitate was filtered off, washed with cold H₂O (3x3 ml), and finally dried *in vacuo* at 40 °C overnight.

The following chromone-2-carboxylic acids were prepared using the demethylation method: 6-hydroxychromone-2-carboxylic acid, 7-hydroxychromone-2-carboxylic acid, 8-hydroxychromone-2-carboxylic acid, 6,7-dihydroxychromone-2-carboxylic acid and 6-hydroxy-7-methoxychromone-2-carboxylic acid.

### BIOLOGICAL ASSAYS

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

A functional cellular assay, based on competition between unlabeled cAMP and a fixed quantity of fluorescence labeled cAMP for a limited number of binding sites on a cAMP specific antibody, is used to discriminate melanocortin receptor agonists from antagonists by fluorescence polarization.

HEK293 cells expressing one of the human melanocortin receptors are transferred to 384 well microtiter plates, an appropriate amount of cAMP antibody is added, followed by the addition of different concentrations of the test compound to effect cAMP production. Cells are lysed and a fluorescence labeled cAMP conjugate is dispensed. The plate is read on a fluorescence polarization microplate reader and the amount of cAMP produced as a response to a test compound is compared to the production of cAMP resulting from stimulation with NDP-alpha-MSH.

To define antagonistic activity of a test compound, the compound is dispensed at different concentrations to cells stimulated by an appropriate amount of NDP-α-MSH. Inhibition of cAMP production is determined by comparing the inhibition of cAMP production of the test compound to the inhibition of cAMP production by a known inhibitor tested at the same concentrations.

Biological Data for selected Examples of the Invention:

| Example | hMC4-R binding assay IC₅₀/nM | hMC4-R functional assay EC₅₀/nM | % activation functional assay |
|---|---|---|---|
| 1 | 80 | - | no activation |
| 8 | 300 | - | no activation |
| 15 | 4.4 | 700 | 40 |
| 66 | 6.8 | - | no activation |
| 110 | 790 | - | no activation |

### C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr;9(2):145-54).

Selected Examples of the present invention were active in the rat model at 3, 10 or 30 mg/kg after i.p. and p.o. administration of the test compound, respectively, using male Wistar rats (n = 4 - 6).

Example 15 at 3 mg/kg p.o. administration lead to an increase in cumulative food intake of 290% (4 hours following administration p = 0.048, n = 4), 186% (6 hours following administration, p = 0.108, n = 4) and 355% (7 hours following administration, p = 0.008, n = 4), respectively, compared to control male Wistar rats receiving vehicle only (n = 6).

Example 66 at 3 mg/kg p.o. administration lead to an increase in cumulative food intake of 410% (2 hours following administration p = 0.057, n = 4), 500% (4 hours following administration p = 0.022, n = 4), 186% (6 hours following administration, p = 0.11, n = 4) and 272% (7 hours following administration, p = 0.02, n = 4), respectively, compared to control male Wistar rats receiving vehicle only (n = 4).

Example 113 at 30 mg/kg p.o. administration lead to an increase in cumulative food intake of 192% (2 hours following administration p = 0.083, n = 4), 159% (4 hours following administration p = 0.097, n = 4), 191% (6 hours following administration, p = 0.026, n = 4) and 200% (7 hours following administration, p = 0.013, n = 4), respectively, compared to control male Wistar rats receiving vehicle only (n = 8).

Example 212 at 10 mg/kg i.p. administration lead to an increase in cumulative food intake of 708% (2 hours following administration p = 0.011, n = 6), 725% (4 hours following administration p = 0.009, n = 6), 224% (6 hours following administration, p = 0.046, n = 6) and 167% (8 hours following administration, p = 0.036, n = 6), respectively, compared to control male Wistar rats receiving vehicle only (n = 10).

### 2. Model of LPS and Tumor-Induced Cachexia

Prevention or amelioration of cachexia induced by either lipopolysaccharide (LPS) administration or by tumor growth is determined upon i.p. or p.o. administration of test compounds to rats (see e.g. Marks, D.L.; Ling, N and Cone, R.D. Cancer Res 2001 Feb 15;61 (4):1432-8).

### a) Lipopolysacchailde-induced Cachexia in Rats (acute model)

1-2 Hours prior to the onset of the dark-phase, individually housed, male Wistar rats (200 - 300 g) receive an ip or po application of test-compound or vehicle (2 ml/kg, 1 - 30 mg/kg) which is followed or preceded by an ip injection of either lipopolysaccharide (LPS) or saline (2 ml/kg, 100 µg/kg). Food intake, water intake and body weight are measured at 1 - 24 hour intervals and differences between experimental groups are evaluated.

### b) Tumour-induced Cachexia in Mice (chronic model)

Subcutaneous injection of Lewis lung carcinoma cells to male C57BL6 mice (1 million cells/100 µl/mouse) results in non-metastasizing tumor growth which in turn results in loss of lean body mass. Chronic ip or po applications of test compounds (10 ml/kg, 1 - 30 mg/kg for 7 - 21 days) are accompanied by daily measurements of food intake, water intake and body weight. Lean body mass is measured at the start, during and at the termination of the study using magnetic resonance relaxometry, and at the end of the study using a conventional chemical extraction procedure (Soxhlet's extraction). Differences between experimental groups are evaluated.

Selected Examples of the present invention were active in the rat model at 10 mg/kg after p.o. administration of the test compound using male Wistar rats (n = 5 - 6).

Example 66 at 10 mg/kg p.o. administration (in combination with LPS) lead to an increase in cumulative food intake of 148% (4 hours following administration p = 0.04, n = 5) compared to control male Wistar rats receiving LPS and vehicle only (n = 6).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm. Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

As evident from the results presented above, representative compounds of the present invention bind to the human melanocortin-4 receptor. Representative compounds of the present invention were also tested in the functional assay and found to be non-activating or activating the melanocortin-4 receptor with high EC₅₀ values and low stimulation.

For example the compound consisting of the spiroindane "A moiety", D-p-chlorophenylalanine and chromone-2-carboxylic acid as "C moiety", example 1 of the present invention, binds to the melanocortin-4 receptor with an IC₅₀ value of 80 nM and does not activate the melanocortin-4 receptor in the functional assay, whereas the compound with D-Tic as "C moiety" (W09964002, example 13) is described to bind to the melanocortin-4 receptor with Kᵢ = 10 nM (*Current Topics Med. Chem.* 2003, 3, 855-883) and to activate the melanocortin-4 receptor with an EC₅₀ = 190 nM (100% stimulation) (Bioorg. Med. Chem. Lett. 2003, 13, 3793-3796). Using our assay system the compound binds to the melanocortin-4 receptor with an IC₅₀ = 54 nM. The receptor is activated with an EC₅₀ = 1.1 µM (110% activation). Therefore example 1 is an antagonist whereas the compound with D-Tic is an agonist.

Compounds bearing an o-substituted arylpiperazine "A moiety" in combination with D-p-chlorophenylalanine as "B moiety" and D-Tic as "C moiety" are known to bind to the melanocortin-4 receptor with Kᵢ values between 24 nM and 6.6 µM (J. Med. Chem. 2004, 47, 744-755, 29 examples) and to activate melanocortin-4 receptor in the functional assay with EC₅₀ values between 14 nM and 1.3 µM (J. Med. Chem. 2004, 47, 744-755, 29 examples) and between 4 nM and 4.4 µM (Bioorg. Med. Chem. Lett. 2003, 13, 3793-3796, 23 examples). In the latter case three additional examples are reported to be weak agonists with 7- to 30-fold stimulation at 30 µM. There is no compound described which does not activate the melanocortin-4 receptor. Some of the compounds described above are claimed in patent applications WO03009850 (example 1) and WO02059108 (examples 2, 11, 15, 21, 24, 25, 26, 35, 37, 38, 40, 43, 44, 46, 52, 54, 56, 61, 74, 75, 79, 81, 153 and 154).

There is evidence in the literature that the stereochemistry of Tic in the "C moiety" does not have a big influence on the melanocortin-4 receptor affinity and activation at a concentration of 10 µM (J. Med. Chem. 2002, 45, 4589-4593, compounds 1 and 13). Both diastereomers activate the melanocortin-4 receptor, however, the D-Tic derived compound 1 shows improved functional activity. Applying this concept to compounds bearing the o-substituted arylpiperazine "A moiety" it can be concluded that the diastereomers of the compounds described in the literature cited above also act as agonists. Some of the L-Tic derivatives are described in WO03009850 (examples 56, 66, 91, 93, 117, 118 and 423).

Example 8 of the present invention binds to the melanocortin-4 receptor with an IC₅₀ = 300 nM and does not activate the melanocortin-4 receptor in the functional assay. The corresponding compound with D-Tic as "C moiety" (WO03009850, example 1 and WO02059108, example 154) is described to bind to the melanocortin-4 receptor with Kᵢ = 220 nM and to activate said receptor with an EC₅₀ = 16 nM (J. Med. Chem. 2004, 47, 744-755, compound 39). Another source describes said compound to activate the melanocortin-4 receptor with an EC₅₀ = 380 nM (100% stimulation) (Bioorg. Med. Chem. Lett. 2003, 13, 3793-3796, compound 3). In our assays the compound was found to have a melanocortin-4 receptor binding IC₅₀ = 500 nM and to activate the melanocortin-4 receptor with an EC₅₀ = 3.7 µM (96% activation). Therefore example 8 is an antagonist whereas the compound with D-Tic is an agonist.

Example 110 of the present invention binds to the melanocortin-4 receptor with an IC₅₀ = 790 nM and does not activate the melanocortin-4 receptor in the functional assay. The corresponding compound with D-Tic as "C moiety" (WO02059108, example 56) is described to bind to the melanocortin-4 receptor with Kᵢ = 210 nM and to activate said receptor with an EC₅₀ = 48 nM (J. Med. Chem. 2004, 47, 744-755, compound 43). Therefore example 110 is an antagonist whereas the compound with D-Tic is an agonist.

Compounds having a 4,4-disubstituted piperidine "A moiety" were generally found to be more potent ligands for the melanocortin-4 receptor.

Example 15 bearing the 4-cyclohexyl-4-[1,2,4]triazol-1-ylmethyl-piperidine "A moiety" binds to the melanocortin-4 receptor with an IC₅₀ = 4.4 nM. In the functional assay the compound acts as a partial agonist of said receptor with an EC₅₀ = 700 nM (40% activation). The corresponding compound with D-Tic as "C moiety" (WO0074679, example 2) is described to bind to the melanocortin-4 receptor with an IC₅₀ between 0.92 and 7 nM and to act as a full agonist of the melanocortin-4 receptor with an EC₅₀ between 2.1 and 4 nM (WO0074679, example 2; J. Med. Chem. 2002, 45, 4589-4593, compound 1; Bioorg. Med. Chem. Lett. 2003, 13, 4341-4344, compound 1 and Bioorg. Med. Chem. Lett. 2003, 13, 3793-3796, compound 27). In our assay system the compound binds to the melanocortin-4 receptor with an IC₅₀ = 10 nM and activates the receptor with an EC₅₀ = 50 nM (102% activation) when tested in the functional assay. Therefore example 15 is a very weak agonist whereas the compound with D-Tic is a full agonist.

Example 66 having the 4-cyclohexyl-piperidine-4-carboxylic acid tert-butylamide "A moiety" has a similar affinity to the melanocortin-4 receptor than example 15. The compound binds to the receptor with IC₅₀ = 6.8 nM, however, no activation of said receptor is observerd in the functional assay. Therefore example 66 is an antagonist.

As illustrated by the biological results (see above) representative compounds of the present invention are also active as antagonists when tested in vivo.

Example 15 is active in the spontaneous feeding paradigm at a dose of 3 mg/kg when administered orally. The test animals show a significantly increase in food intake. However, the corresponding compound with D-Tic as "C moiety" is reported to act as in vivo agonist in a conscious rat model of reflexogenic erections when administered i.v. or p.o. (WO0074679, example 2) and it was also evaluated for its effects on food intake (J. Med. Chem. 2002, 45, 4589-4593, compound 1). Food intake was significantly reduced on administration of the compound.

Example 66 is active in the spontaneous feeding paradigm as well as in the model of LPS-induced cachexia. In the spontaneous feeding paradigm the test animals show a significant increase in food intake at dose of 3 mg/kg p.o. and in the acute model of lipopolysaccharide-induced cachexia the test animals show a significant increase in cumulative food intake at a dose of 10 mg/kg p.o..

### Examples of a Pharmaceutical Composition

As a specific embodiment of an oral composition of a compound of the present invention, 25 mg of Example 66 is formulated with sufficient finely divided lactose to provide a total amount of 580 to 590 mg to fill a size 0 hard gelatin capsule.

As another specific embodiment of an oral composition of a compound of the present invention, 35 mg of Example 113 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, Thereforee, that the invention be limited only by the scope of the claims which follow and that such claims be interpreted as broadly as is reasonable.

## Claims

1. A compound of structural formula (I): 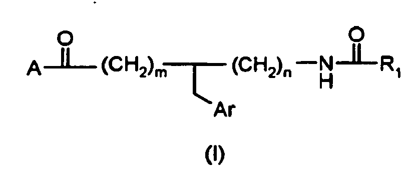 or a pharmaceutically acceptable salt or a solvate thereof, wherein
Ar is:
aryl or heteroaryl which may both be substituted by one to three substituents independently selected from the group consisting of cyano, nitro, perfluoro-C₁-C₈-alkoxy, halo, C₁-C₈-alkyl, (D)-cycloalkyl, C₁-C₈-alkoxy or halo-C₁-C₈-alkyl;
R₁ is: 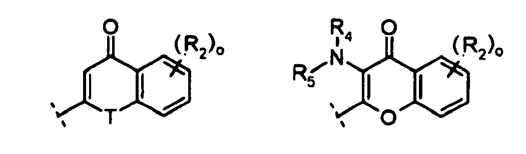
A is: 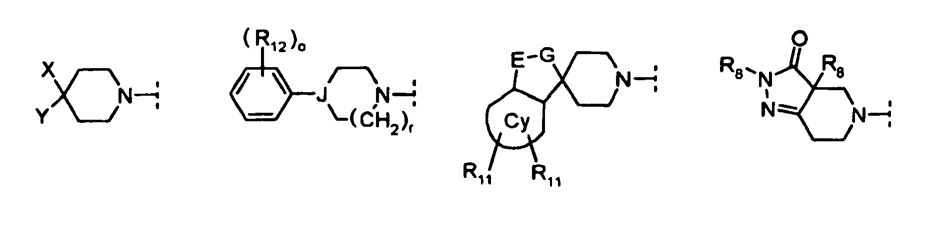
R₂ is independently:
hydrogen,
halo,
C₁-C₈-alkyl, straight chained or branched,
halo-C₁-C₈-alkyl, straight chained or branched,
hydroxy,
C₁-C₈-alkoxy, straight chained or branched,
S-C₁-C₈-alkyl, straight chained or branched,
SO₂-C₁-C₈-alkyl, straight chained or branched,
O-C₂-C₈-alkenyl, straight chained or branched,
S-C₂-C₈-alkenyl, straight chained or branched,
NR₁₄C(O)R₁₄,
NR₁₄SO₂R₁₄,
N(R₁₄)₂,
(D)-cycloalkyl,
(D)-aryl,
(D)-heteroaryl,
(D)-heterocyclyl (wherein heterocyclyl excludes a heterocyclyl containing a single nitrogen), and
wherein aryl, heteroaryl, heterocyclyl, alkyl and cycloalkyl are unsubstituted or substituted by one to three substituents selected from the group consisting of oxo, halo, C₁-C₈-alkyl, N(R₆)₂, OR₆, SR₆ and CO₂R₆,
and two adjacent R₂ may form a 4- to 7-membered ring;
R₄ and R₅ are each independently:
hydrogen,
C₁-C₈-alkyl, straight chained or branched or
(D)-cycloalkyl, or
R₄ and R₅ together with the nitrogen to which they are attached form a 5- to 8-membered ring,
wherein alkyl and cycloalkyl are unsubstituted or substituted by one to three substituents selected from R₇ and oxo;
R₆ is independently:
hydrogen,
C₁-C₈-alkyl, straight chained or branched,
C(O)-C₁-C₈-alkyl, straight chained or branched,
(D)-aryl or
(D)-cycloalkyl;
R₇ is independently:
C₁-C₈-alkyl, straight chained or branched,
(D)-aryl,
(D)-cycloalkyl,
(D)-heteroaryl,
halo,
OR₈,
NHSO₂R₈,
N(R₈)₂,
CN,
CO₂R₄,
C(R₈)(R₈)N(R₈)₂,
nitro,
SO₂N(R₈)₂,
S(O)ₚR₈,
CF₃ or
OCF₃;
R₈ is independently:
hydrogen,
C₁-C₈-alkyl, straight chained or branched,
(D)-aryl or
(D)-cycloalkyl;
R₉ is independently:
hydrogen,
C₁-C₈-alkyl, straight chained or branched,
(D)-aryl,
(D)-heteroaryl or
(D)-cycloalkyl;
R₁₀ is independently:
R₉,
(D)-heterocyclyl,
(D)-N(Y)₂,
(D)-NH-heteroaryl or
(D)-NH-heterocyclyl,
wherein aryl and heteroaryl are unsubstituted or substituted by one to three R₇, and alkyl, D, cycloalkyl and heterocyclyl are unsubstituted or substituted by one to four substituents independently selected from R₇ and oxo, or
two R₁₀ groups together with the atoms to which they are attached form a 5- to 8-membered mono- or bi-cyclic ring system;
R₁₁ is:
hydrogen,
halo,
C₁-C₈-alkyl, straight chained or branched,
C₁-C₈-alkoxy, straight chained or branched,
C≡N,
CF₃ or
OCF₃;
R₁₂ is independently:
hydrogen,
hydroxy,
cyano,
nitro,
halo,
C₁-C₈-alkyl, straight chained or branched,
C₁-C₈-alkoxy, straight chained or branched,
halo-C₁-C₈-alkyl, straight chained or branched,
(D)-C(O)R₁₄.
(D)-C(O)OR₁₄,
(D)-C(O)SR₁₄,
(D)-C(O)-heteroaryl,
(D)-C(O)-heterocyclyl,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂,
(D)-NR₁₄SO₂R₁₄,
(D)-NR₁₄SO₂N(R₁₄)₂,
(D)-NR₁₄(D)-heterocyclyl,
(D)-NR₁₄(D)-heteroaryl,
(D)-OR₁₄,
OSO₂R₁₄,
(D)-[O]_{q}(cycloalkyl),
(D)-[O]_{q}(D)aryl,
(D)-[O]_{q}(D)-heteroaryl,
(D)-[O]_{q}(D)-heterocyclyl (wherein heterocyclyl excludes a heterocyclyl containing a single nitrogen when q=1),
(D)-SR₁₄,
(D)-SOR₁₄,
(D)-SO₂R₁₄ or
(D)-SO₂N(R₁₄)₂,
wherein alkyl, alkoxy, cycloalkyl, aryl, heterocyclyl and heteroaryl are unsubstituted or substituted by one to five substituents independently selected from R₁₃;
R₁₃ is independently:
hydrogen,
halo,
oxo,
N(R₁₅)₂,
C₁-C₈-alkyl, straight chained or branched,
(D)-cycloalkyl,
halo-C₁-C₈-alkyl, straight chained or branched,
C₁-C₈-alkoxy, straight chained or branched,
heteroaryl,
hydroxy,
heterocyclyl (wherein heterocyclyl excludes a heterocyclyl containing a single
nitrogen),
phenyl,
(D)-C(O)R₁₄,
(D)-C(O)OR₁₄,
(D)-OR₁₄,
(D)-OCOR₁₄,
(D)-OCO₂R₁₄,
(D)-SR₁₄,
(D)-SOR₁₄ or
(D)-SO₂R₁₄,
wherein aryl, heteroaryl, heterocyclyl, alkyl or cycloalkyl are unsubstituted or substituted with one to three substiutents selected from the group consisting of oxo, C₁-C₈-alkyl, N(R₁₅)₂, OR₁₅, SR₁₅ and CO₂R₁₅;
R₁₄ is independently:
hydrogen,
C₁-C₈-alkyl, straight chained or branched,
halo-C₁-C₈-alkyl, straight chained or branched,
(D)-cycloalkyl,
(D)-phenyl,
(D)-naphthyl,
(D)-heteroaryl,
(D)-heterocyclyl (wherein heterocyclyl excludes a heterocyclyl containing a single nitrogen), and
wherein phenyl, naphthyl, heteroaryl, heterocyclyl, alkyl and cycloalkyl are unsubstituted or substituted with one to three substituents selected form the group consisting of oxo, halo-C₁-C₈-alkyl, N(R₁₅)₂, OR₁₅, SR₁₅ and CO₂R₁₅;
R₁₅ is independently:
hydrogen
C₁-C₈-alkyl, straight chained or branched,
C(O)C₁-C₈-alkyl, straight chained or branched,
aryl or
cycloalkyl;
X is:
C₁-C₈-alkyl, straight chained or branched,
(D)-cycloalkyl,
(D)-aryl,
(D)-heteroaryl,
(D)-heterocyclyl,
(D)-C≡N,
(D)-CON(R₉R₉),
(D)-CO₂R₉,
(D)-COR₉,
(D)-NR₉C(O)R₉,
(D)-NR₉CO₂R₉,
(D)-NR₉C(O)N(R₉)₂,
(D)-NR₉SO₂R₉,
(D)-S(O)ₚR_{9,}
(D)-SO₂N(R₉)(R₉),
(D)-OR₉,
(D)-OC(O)R₉,
(D)-OC(O)OR₉,
(D)-OC(O)N(R₉)₂,
(D)-N(R₉)(R₉) or
(D)-NR₉SO₂N(R₉)(R₉),
wherein aryl and heteroaryl are unsubstituted or substituted with one to three groups selected from R₇ and
alkyl, D, cycloalkyl and heterocyclyl are unsubstituted or substituted with one to four groups independently selected from R₇ and oxo;
Y is:
hydrogen,
C₁-C₈-alkyl, straight chained or branched,
(D)-cycloalkyl,
(D)-aryl,
(D)-heterocyclyl or
(D)-heteroaryl,
wherein aryl and heteroaryl are unsubstituted or substituted with one to three groups selected from R₇ and
alkyl, D and cycloalkyl are unsubstituted or substituted with one to three groups independently selected from R₇ and oxo;
Cy is benzene, pyridine or cyclohexane;
D is a bond or C₁-C₈-alkylene, straight chained or branched;
E is CHCO₂Y, CHC(O)N(Y)₂, NSO₂R₁₀, CHN(Y)COR₁₀, CHN(Y)SO₂R₁₀, CHCH₂OY or CHCH₂heteroaryl;
G is D, CH-alkyl, O, C=O or SO₂, with the proviso that when G is O, the ring atom E is carbon;
J is N or CH;
T is O or NR₄;
n is 0 - 2;
m is 0 - 2;
o is 0 - 3;
p is 0 - 2;
q is 0 or 1;
r is 1 or 2 and
wherein
aryl is an aromatic mono- or polycyclic moiety with 6 to 20 carbon atoms, heteroaryl is an aromatic moiety having 6 to 20 carbon atoms with at least one heterocycle;
heterocyclyl is a saturated, unsaturated or aromatic ring containing at least one heteroatom selected from O, N and/or S and 1 to 6 carbon atoms; and
cycloalkyl is an alkyl ring having 3 to 8 carbon atoms.

2. The compound of claim 1, wherein
Ar is:
aryl which may be substituted with one to three substituents independently selected from the group consisting of cyano, nitro, perfluoroalkoxy, halo, C₁-C₈-alkyl, straight chained or branched, (D)-cycloalkyl, C₁-C₈-alkoxy, straight chained or branched, and/or halo-C₁-C₈-alkyl, straight chained or branched;
R₁ is: 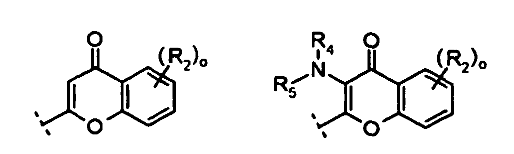
A is: 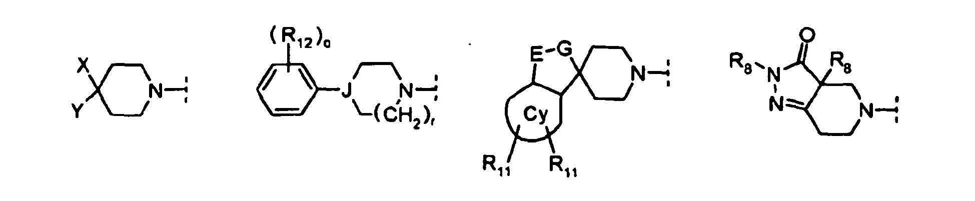
R₂ is independently:
hydrogen,
hydroxy,
halo,
C₁-C₈-alkyl, straight chained or branched,
C₁-C₈-alkoxy, straight chained or branched,
S-C₁-C₈-alkyl, straight chained or branched,
SO₂-C₁-C₈-alkyl, straight chained or branched,
O-C₂-C₈-alkenyl, straight chained or branched,
S-C₂-C₈-alkenyl, straight chained or branched,
halo-C₁-C₈-alkyl, straight chained or branched or
(D)-cycloalkyl;
R₄ and R₅ are each independently:
hydrogen,
C₁-C₈-alkyl, straight chained or branched or
cycloalkyl, or
R₄ and R₅ together with the nitrogen to which they are attached form a 5- to 7-membered ring which may contain an additional heteroatom selected from
O, S and NR₆;
R₆ is independently:
hydrogen,
C₁-C₈-alkyl, straight chained or branched,
C(O)-C₁-C₈-alkyl, straight chained or branched,
(D)-aryl or
(D)-cycloalkyl;
R₈ is independently:
hydrogen,
C₁-C₈-alkyl, straight chained or branched, or
(D)-aryl;
R₉ is independently:
hydrogen,
C₁-C₈-alkyl, straight chained or branched, or
(D)-cycloalkyl;
R₁₀ is R₉;
R₁₁ is:
hydrogen,
halo,
C₁-C₈-alkyl, straight chained or branched,
C₁-C₈-alkoxy, straight chained or branched, or
C≡N;
R₁₂ is independently:
hydrogen,
hydroxy,
cyano,
nitro,
halo,
C₁-C₈-alkyl, straight chained or branched,
C₁-C₈-alkoxy, straight chained or branched,
Halo-C₁-C₈-alkyl, straight chained or branched,
(D)-C(O)-heterocyclyl,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂,
(D)-NR₁₄SO₂R₁₄ or
(D)-NR₁₄SO₂N(R₁₄)₂;
R₁₄ is independently:
hydrogen,
halo,
C₁-C₈-alkyl, straight chained or branched,
(D)-cycloalkyl,
C₁-C₈-alkox, straight chained or branched or
phenyl;
X is:
C₁-C₈-alkyl, straight chained or branched,
(D)-cycloalkyl,
(D)-aryl,
(D)-heteroaryl,
(D)-heterocyclyl,
(D)-NHC(O)R₉,
(D)-CO₂R₉ or
(D)-CON(R₉R₉);
Y is:
hydrogen,
C₁-C₈-alkyl, straight chained or branched,
(D)-cycloalkyl,
(D)-aryl,
(D)-heterocyclyl or
(D)-heteroaryl;
Cy is benzene or pyridine;
D is a bond or C₁ - C₄-alkylene;
E is NSO₂R₁₀, CHN(Y)COR₁₀ or CHN(Y)SO₂R₁₀;
G is D or CH-alkyl;
J is N or CH;
n is 0 or 1;
m is 0 or 1;
o is 0, 1 or 2;
r is 1.

3. The compound of claim 1 or 2, wherein
Ar is:
phenyl or naphthyl which may be substituted with one or two substituents independently selected from the group consisting of halo, C₁-C₈-alkyl, C₁-C₈-alkoxy and/or halo-C₁-C₈-alkyl;
R₁ is: 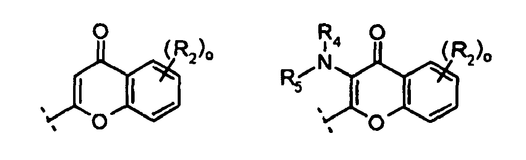
A is: 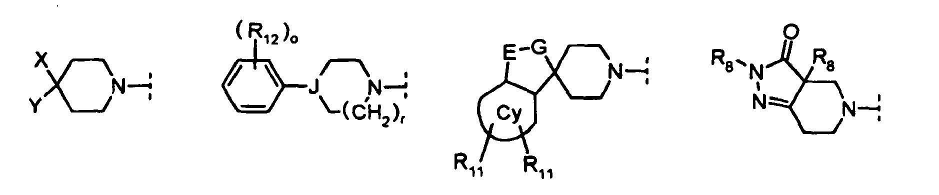
R₂ is independently:
hydrogen,
hydroxy,
C₁-C₈-alkoxy, straight chained or branched,
S-C₁-C₈-alkyl, straight chained or branched,
SO₂-C₁-C₈-alkyl, straight chained or branched,
O-C₂-C₈-alkenyl, straight chained or branched,
S-C₂-C₈-alkenyl, straight chained or branched,
halo or
C₁-C₈-alkyl, straight chained or branched;
R₄ and R₅ are each independently:
hydrogen or
C₁-C₈-alkyl, straight chained or branched, or
R₄ and R₅ together with the nitrogen to which they are attached form a 5- to 6-membered ring optionally containing an additional oxygen atom;
R₆ is hydrogen;
R₈ is independently:
C₁-C₈-alkyl, straight chained or branched or
(D)-aryl;
R₉ is C₁-C₈-alkyl, straight chained or branched;
R₁₀ is R₉;
R₁₁ is:
hydrogen,
halo and
C₁ - C₄-alkyl;
R₁₂ is independently:
cyano,
nitro,
halo,
C₁-C₈-alkyl, straight chained or branched,
(D)-C(O)-heterocyclyl.
(D)-N(R₁₄)₂,
(D)-NR₁₄COR₁₄,
(D)-NR₁₄CON(R₁₄)₂,
(D)-NR₁₄C(O)OR₁₄ or
(D)-NR₁₄SO₂R₁₄;
R₁₄ is independently:
hydrogen,
halo,
C₁-C₈-alkyl, straight chained or branched,
C₁-C₈-alkoxy, straight chained or branched, or phenyl;
X is:
C₁-C₈-alkyl, straight chained or branched,
(D)-cycloalkyl,
(D)-heterocyclyl,
(D)-NHC(O)R₉ or
(D)-CON(R₉R₉);
Y is:
hydrogen,
C₁-C₈-alkyl, straight chained or branched,
(D)-cycloalkyl or
(D)-heterocyclyl;
Cy is benzene;
D is a bond or CH₂;
E is NSO₂R₁₀;
GisD;
J is N or CH;
n is 0;
m is 0;
o is 0 or 1;
p is 0, 1 or 2;
q is 0 or 1;
r is 1.

4. The compound of any of claims 1 to 3 for use as a medicament.

5. Use of the compound of any of claims 1 to 3 for the preparation of a medicament for the treatment or prevention of disorders, diseases or conditions responsive to the modulation of the melanocortin-4 receptor in a mammal, where modulation means activation in the case of MC4-R agonists or inactivation in the case of MC4-R antagonists.

6. Use of MC4-R antagonists according to claims 5 for the preparation of a medicament for the treatment or prevention of cancer cachexia.

7. Use of MC4-R antagonists according to claims 5 for the preparation of a medicament for the treatment or prevention of muscle wasting.

8. Use of MC4-R antagonists according to claims 5 for the preparation of a medicament for the treatment or prevention of anorexia.

9. Use of MC4-R antagonists according to claims 5 for the preparation of a medicament for the treatment or prevention of anxiety and/or depression.

10. Use of MC4-R agonists according to claims 5 for the preparation of a medicament for the treatment or prevention of obesity.

11. Use of MC4-R agonists according to claims 5 for the preparation of a medicament for the treatment or prevention of diabetes mellitus.

12. Use of MC4-R agonists according to claims 5 for the preparation of a medicament for the treatment or prevention of male or female sexual dysfunction.

13. Use of MC4-R agonists according to claims 5 for the preparation of a medicament for the treatment or prevention of erectile dysfunction.

14. A pharmaceutical composition which comprises a compound of any of claims 1 to 3 and a pharmaceutically acceptable carrier.

## Patentansprüche

1. Eine Verbindung der Strukturformel (I): 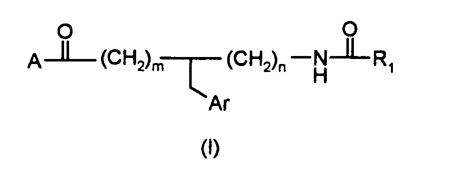 oder ein pharmazeutisch akzeptables Salz oder ein Solvat davon, worin
Ar bedeutet:
Aryl oder Heteroaryl, welche beide durch ein bis drei Substituenten, unabhängig voneinander ausgewählt aus der Gruppe bestehend aus Cyano, Nitro, Perfluor-C₁-C₈-alkoxy, Halogen, C₁-C₈-Alkyl, (D)-Cycloalkyl, C₁-C₈-Alkoxy oder Halogen-C₁-C₈-alkyl, substituiert sein können;
R₁ bedeutet: 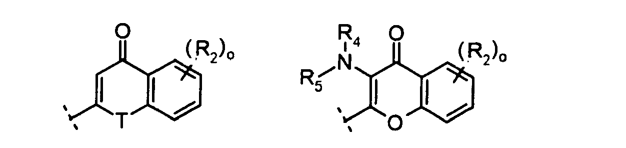
A bedeutet: 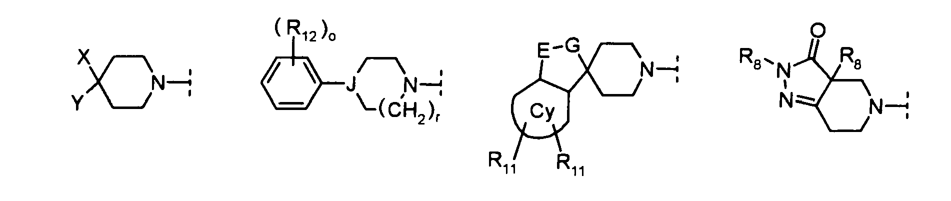
R₂ bedeutet unabhängig:
Wasserstoff,
Halogen,
C₁-C₈-Alkyl, geradkettig oder verzweigt,
Halogen-C₁-C₈-alkyl, geradkettig oder verzweigt,
Hydroxy,
C₁-C₈-Alkoxy, geradkettig oder verzweigt,
S-C₁-C₈-Alkyl, geradkettig oder verzweigt,
SO₂-C₁-C₈-Alkyl, geradkettig oder verzweigt,
O-C₂-C₈-Alkenyl, geradkettig oder verzweigt,
S-C₂-C₈-Alkenyl, geradkettig oder verzweigt,
NR₁₄C(O)R₁₄,
NR₁₄SO₂R₁₄,
N(R₁₄)₂,
(D)-Cycloalkyl,
(D)-Aryl,
(D)-Heteroaryl,
(D)-Heterocyclyl (worin Heterocyclyl ein Heterocyclyl enthaltend ein einzelnes Stickstoff ausschließt), und
worin Aryl, Heteroaryl, Heterocyclyl, Alkyl und Cycloalkyl unsubstituiert oder durch ein bis drei Substituenten, ausgewählt aus der Gruppe bestehend aus Oxo, Halogen, C₁-C₈-Alkyl, N(R₆)₂, OR₆, SR₆ und CO₂R₆, substituiert sind,
und zwei benachbarte R₂ einen 4- bis 7-gliedrigen Ring bilden können;
R₄ und R₅ jeweils unabhängig bedeuten:
Wasserstoff,
C₁-C₈-Alkyl, geradkettig oder verzweigt oder
(D)-Cycloalkyl oder
R₄ und R₅ zusammen mit dem Stickstoff, an welchem sie befestigt sind, einen 5- bis 8-gliedrigen Ring bilden,
worin Alkyl und Cycloalkyl unsubstituiert oder durch ein bis drei Substituenten, ausgewählt aus R₇ und Oxo, substituiert sind;
R₆ bedeutet unabhängig:
Wasserstoff,
C₁-C₈-Alkyl, geradkettig oder verzweigt,
C(O)-C₁-C₈-Alkyl, geradkettig oder verzweigt,
(D)-Aryl oder
(D)-Cycloalkyl;
R₇ bedeutet unabhängig:
C₁-C₈-Alkyl, geradkettig oder verzweigt,
(D)-Aryl,
(D)-Cycloalkyl,
(D)-Heteroaryl,
Halogen,
OR₈,
NHSO₂R₈,
N(R₈)₂,
CN,
CO₂R₄,
C(R₈)(R₈)N(R₈)₂,
Nitro,
SO₂N(R₈)₂,
S(O)ₚR₈,
CF₃ oder
OCF₃;
R₈ bedeutet unabhängig:
Wasserstoff,
C₁-C₈-Alkyl, geradkettig oder verzweigt,
(D)-Aryl oder
(D)-Cycloalkyl;
R₉ bedeutet unabhängig:
Wasserstoff,
C₁-C₈-Alkyl, geradkettig oder verzweigt,
(D)-Aryl,
(D)-Heteroaryl oder
(D)-Cycloalkyl;
R₁₀ bedeutet unabhängig:
R₉,
(D)-Heterocyclyl,
(D)-N(Y)₂,
(D)-NH-Heteroaryl oder
(D)-NH-Heterocyclyl,
worin Aryl und Heteroaryl unsubstituiert oder durch ein bis drei R₇ substituiert sind und Alkyl, D, Cycloalkyl und Heterocyclyl unsubstituiert oder durch ein bis vier Substituenten, unabhängig ausgewählt aus R₇ und Oxo, substituiert sind, oder
zwei R₁₀-Gruppen bilden zusammen mit den Atomen, an welchen sie befestigt sind, ein 5- bis 8-gliedriges mono- oder bicyclisches Ringsystem;
R₁₁ bedeutet:
Wasserstoff,
Halogen,
C₁-C₈-Alkyl, geradkettig oder verzweigt,
C₁-C₈-Alkoxy, geradkettig oder verzweigt,
C≡N,
CF₃ oder
OCF₃;
R₁₂ bedeutet unabhängig:
Wasserstoff,
Hydroxy,
Cyano,
Nitro,
Halogen,
C₁-C₈-Alkyl, geradkettig oder verzweigt,
C₁-C₈-Alkoxy, geradkettig oder verzweigt,
Halogen-C₁-C₈-alkyl, geradkettig oder verzweigt,
(D)-C(O)R₁₄,
(D)-C(O)OR₁₄,
(D)-C(O)SR₁₄,
(D)-C(O)-Heteroaryl,
(D)-C(O)-Heterocyclyl,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂,
(D)-NR₁₄SO₂R₁₄,
(D)-NR₁₄SO₂N(R₁₄)₂,
(D)-NR₁₄(D)-Heterocyclyl,
(D)-NR₁₄(D)-Heteroaryl,
(D)-OR₁₄,
OSO₂R₁₄,
(D)-[O]_{q}(Cycloalkyl),
(D)-[O]_{q}(D)Aryl,
(D)-[O]_{q}(D)-Heteroaryl,
(D)-[O]q(D)-Heterocyclyl (worin Heterocyclyl ein Heterocyclyl enthaltend ein einzelnes Stickstoff ausschließt, wenn q = 1),
(D)-SR₁₄,
(D)-SOR₁₄,
(D)-SO₂R₁₄ oder
(D)-SO₂N(R₁₄)₂,
worin Alkyl, Alkoxy, Cycloalkyl, Aryl, Heterocyclyl und Heteroaryl unsubstituiert oder durch ein bis fünf Substituenten, unabhängig voneinander ausgewählt aus R₁₃, substituiert sind;
R₁₃ bedeutet unabhängig:
Wasserstoff,
Halogen,
Oxo,
N(R₁₅)₂,
C₁-C₈-Alkyl, geradkettig oder verzweigt,
(D)-Cycloalkyl,
Halogen-C₁-C₈-alkyl, geradkettig oder verzweigt,
C₁-C₈-Alkoxy, geradkettig oder verzweigt,
Heteroaryl,
Hydroxy,
Heterocyclyl (worin Heterocyclyl ein Heterocyclyl enthaltend ein einzelnes Stickstoff ausschließt)
Phenyl,
(D)-C(O)R₁₄,
(D)-C(O)OR₁₄,
(D)-OR₁₄,
(D)-OCOR₁₄,
(D)-OCO₂R₁₄,
(D)-SR₁₄,
(D)-SOR₁₄, oder
(D)-SO₂R₁₄,
worin Aryl, Heteroaryl, Heterocyclyl, Alkyl oder Cycloalkyl unsubstituiert oder durch ein bis drei Substituenten, ausgewählt aus der Gruppe bestehend aus Oxo, C₁-C₈-Alkyl, N(R₁₅)₂, OR₁₅, SR₁₅ und CO₂R₁₅, substituiert sind;
R₁₄ bedeutet unabhängig:
Wasserstoff,
C₁-C₈-Alkyl, geradkettig oder verzweigt,
Halogen-C₁-C₈-alkyl, geradkettig oder verzweigt,
(D)-Cycloalkyl,
(D)-Phenyl,
(D)-Naphthyl,
(D)-Heteroaryl
(D)-Heterocyclyl (worin Heterocyclyl ein Heterocyclyl enthaltend ein einzelnes Stickstoff ausschließt), und
worin Phenyl, Naphthyl, Heteroaryl, Heterocyclyl, Alkyl und Cycloalkyl unsubstituiert oder mit ein bis drei Substituenten, ausgewählt aus der Gruppe bestehend aus Oxo, Halogen-C₁-C₈-Alkyl, N(R₁₅)₂, OR₁₅, SR₁₅ und CO₂R₁₅, substituiert sind;
R₁₅ bedeutet unabhängig:
Wasserstoff
C₁-C₈-Alkyl, geradkettig oder verzweigt,
C(O)C₁-C₈-Alkyl, geradkettig oder verzweigt,
Aryl oder
Cycloalkyl;
X bedeutet:
C₁-C₈-Alkyl, geradkettig oder verzweigt,
(D)-Cycloalkyl,
(D)-Aryl,
(D)-Heteroaryl,
(D)-Heterocyclyl,
(D)-C≡N,
(D)-CON(R₉R₉),
(D)-CO₂R₉,
(D)-COR₉,
(D)-NR₉C(O)R₉,
(D)-NR₉CO₂R₉,
(D)-NR₉C(O)N(R₉)₂,
(D)-NR₉SO₂R₉,
(D)-S(O)ₚR₉,
(D)-SO₂N(R₉)(R₉),
(D)-OR₉,
(D)-OC(O)R₉,
(D)-OC(O)OR₉,
(D)-OC(O)N(R₉)₂,
(D)-N(R₉)(R₉) oder
(D)-NR₉SO₂N(R₉)(R₉),
worin Aryl und Heteroaryl unsubstituiert oder mit einer bis drei Gruppen, ausgewählt aus R₇, substituiert sind und
Alkyl, D, Cycloalkyl und Heterocyclyl unsubstituiert oder mit einer bis vier Gruppen, unabhängig ausgewählt aus R₇ und Oxo, substituiert sind;
Y bedeutet:
Wasserstoff,
C₁-C₈-Alkyl, geradkettig oder verzweigt,
(D)-Cycloalkyl,
(D)-Aryl,
(D)-Heterocyclyl oder
(D)-Heteroaryl,
worin Aryl und Heteroaryl unsubstituiert oder mit einer bis drei Gruppen, ausgewählt aus R₇, substituiert sind und
Alkyl, D und Cycloalkyl unsubstituiert oder mit einer bis drei Gruppen, ausgewählt aus R₇ und Oxo, substituiert sind;
Cy bedeutet Benzol, Pyridin oder Cyclohexan;
D bedeutet eine Bindung oder C₁-C₈-Alkylen, geradkettig oder verzweigt;
E bedeutet CHCO₂Y, CHC(O)N(Y)₂, NSO₂R₁₀, CHN(Y)COR₁₀, CHN(Y)SO₂R₁₀, CHCH₂OY oder CHCH₂Heteroaryl;
G bedeutet D, CH-Alkyl, O, C=O oder S0₂, mit der Maßgabe, dass wenn G O (Sauerstoff) bedeutet, das Ringatom E Kohlenstoff ist;
J bedeutet N oder CH;
T bedeutet O oder NR₄;
n bedeutet 0 - 2;
m bedeutet 0 - 2;
o bedeutet 0 - 3;
p bedeutet 0 - 2;
q bedeutet 0 oder 1;
r bedeutet 1 oder 2 und
worin Aryl ein aromatischer mono- oder polycyclischer Baustein mit 6 bis 20 Kohlenstoffatomen ist;
Heteroaryl ein aromatischer Baustein mit 6 bis 20 Kohlenstoffatomen mit mindestens einem Heterocyclus ist;
Heterocyclyl ein gesättigter, ungesättigter oder aromatischer Ring enthaltend wenigstens ein Heteroatom, ausgewählt aus O, N und/oder S, und 1 bis 6 Kohlenstoffatome ist; und
Cycloalkyl ein Alkylring mit 3 bis 8 Kohlenstoffatomen ist.

2. Verbindung gemäß Anspruch 1, worin
Ar bedeutet:
Aryl, welches mit einem bis drei Substituenten unabhängig voneinander ausgewählt aus der Gruppe bestehend aus Cyano, Nitro, Perfluoralkoxy, Halogen, C₁-C₈-Alkyl, geradkettig oder verzweigt, (D)-Cycloalkyl, C₁-C₈-Alkoxy, geradkettig oder verzweigt, und/oder Halogen-C₁-C₈-alkyl, geradkettig oder verzweigt, substituiert sein kann;
R₁ bedeutet: 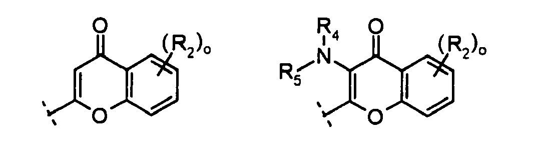
A bedeutet: 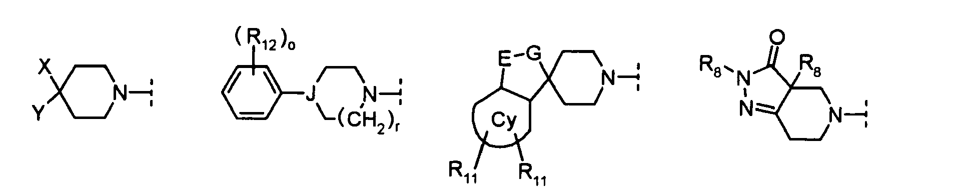
R₂ bedeutet unabhängig:
Wasserstoff,
Hydroxy,
Halogen,
C₁-C₈-Alkyl, geradkettig oder verzweigt,
C₁-C₈-Alkoxy, geradkettig oder verzweigt,
S-C₁-C₈-Alkyl, geradkettig oder verzweigt,
SO₂-C₁-C₈-Alkyl, geradkettig oder verzweigt,
O-C₂-C₈-Alkenyl, geradkettig oder verzweigt,
S-C₂-C₈-Alkenyl, geradkettig oder verzweigt,
Halogen-C₁-C₈-alkyl, geradkettig oder verzweigt, oder
(D)-Cycloalkyl;
R₄ und R₅ bedeuten jeweils unabhängig:
Wasserstoff,
C₁-C₈-Alkyl, geradkettig oder verzweigt, oder
Cycloalkyl, oder
R₄ und R₅ zusammen mit dem Stickstoff, an welchem sie befestigt sind, bilden einen 5- bis 7-gliedrigen Ring, welcher ein zusätzliches Heteroatom, ausgewählt aus O, S und NR₆, enthalten kann;
R₆ bedeutet unabhängig:
Wasserstoff,
C₁-C₈-Alkyl, geradkettig oder verzweigt,
C(O)-C₁-C₈-Alkyl, geradkettig oder verzweigt,
(D)-Aryl oder
(D)-Cycloalkyl;
R₈ bedeutet unabhängig:
Wasserstoff,
C₁-C₈-Alkyl, geradkettig oder verzweigt, oder
(D)-Aryl;
R₉ bedeutet unabhängig:
Wasserstoff,
C₁-C₈-Alkyl, geradkettig oder verzweigt, oder
(D)-Cycloalkyl;
R₁₀ bedeutet R₉;
R₁₁ bedeutet:
Wasserstoff,
Halogen,
C₁-C₈-Alkyl, geradkettig oder verzweigt,
C₁-C₈-Alkoxy, geradkettig oder verzweigt, oder
C≡N;
R₁₂ bedeutet unabhängig:
Wasserstoff,
Hydroxy,
Cyano,
Nitro,
Halogen,
C₁-C₈-Alkyl, geradkettig oder verzweigt,
C₁-C₈-Alkoxy, geradkettig oder verzweigt,
Halogen-C₁-C₈-alkyl, geradkettig oder verzweigt,
(D)-C(O)-Heterocyclyl,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂
(D)-NR₁₄SO₂R₁₄ oder
(D)-NR₁₄SO₂N(R₁₄)₂;
R₁₄ bedeutet unabhängig:
Wasserstoff,
Halogen,
C₁-C₈-Alkyl, geradkettig oder verzweigt,
(D)-Cycloalkyl,
C₁-C₈-Alkoxy, geradkettig oder verzweigt, oder
Phenyl;
X bedeutet:
C₁-C₈-Alkyl, geradkettig oder verzweigt,
(D)-Cycloalkyl,
(D)-Aryl,
(D)-Heteroaryl,
(D)-Heterocyclyl,
(D)-NHC(O)R₉,
(D)-CO₂R₉ oder
(D)-CON(R₉R₉);
Y bedeutet:
Wasserstoff,
C₁-C₈-Alkyl, geradkettig oder verzweigt,
(D)-Cycloalkyl,
(D)-Aryl,
(D)-Heterocyclyl, oder
(D)-Heteroaryl;
Cy bedeutet Benzol oder Pyridin;
D bedeutet eine Bindung oder C₁-C₄-Alkylen;
E bedeutet NSO₂R₁₀, CHN(Y)COR₁₀ oder CHN(Y)SO₂R₁₀;
G bedeutet D oder CH-Alkyl;
J bedeutet N oder CH;
n bedeutet 0 oder 1;
m bedeutet 0 oder 1;
o bedeutet 0, 1 oder 2;
r bedeutet 1.

3. Verbindung gemäß Anspruch 1 oder 2, worin
Ar bedeutet:
Phenyl oder Naphthyl, welche mit einem oder zwei Substituenten, unabhängig ausgewählt aus der Gruppe bestehend aus Halogen, C₁-C₈-Alkyl, C₁-C₈-Alkoxy und/oder Halogen-C₁-C₈-alkyl, substituiert sein können;
R₁ bedeutet: 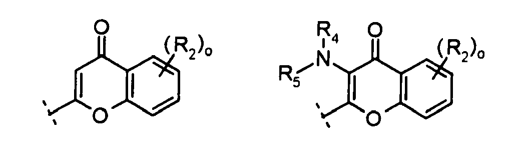
A bedeutet: 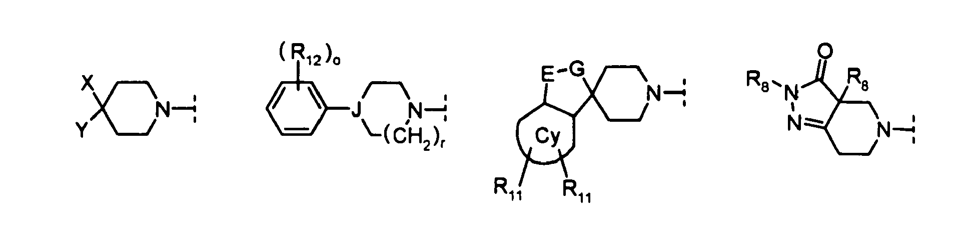
R₂ bedeutet unabhängig:
Wasserstoff,
Hydroxy,
C₁-C₈-Alkoxy, geradkettig oder verzweigt,
S-C₁-C₈-Alkyl, geradkettig oder verzweigt,
SO₂-C₁-C₈-Alkyl, geradkettig oder verzweigt,
O-C₂-C₈-Alkenyl, geradkettig oder verzweigt,
S-C₂-C₈-Alkenyl, geradkettig oder verzweigt,
Halogen oder
C₁-C₈-Alkyl, geradkettig oder verzweigt,
R₄ und R₅ bedeuten jeweils unabhängig:
Wasserstoff oder
C₁-C₈-Alkyl, geradkettig oder verzweigt, oder
R₄ und R₅ bilden zusammen mit dem Stickstoff, an welchem sie befestigt sind, einen 5- bis 6-gliedrigen Ring, der optional ein zusätzliches Sauerstoffatom enthalten kann;
R₆ bedeutet Wasserstoff;
R₈ bedeutet unabhängig:
C₁-C₈-Alkyl, geradkettig oder verzweigt, oder
(D)-Aryl;
R₉ bedeutet C₁-C₈-Alkyl, geradkettig oder verzweigt;
R₁₀ bedeutet R₉;
R₁₁ bedeutet:
Wasserstoff,
Halogen und
C₁-C₄-Alkyl;
R₁₂ bedeutet unabhängig:
Cyano,
Nitro,
Halogen,
C₁-C₈-Alkyl, geradkettig oder verzweigt,
(D)-C(O)-Heterocyclyl,
(D)-N(R₁₄)₂,
(D)-NR₁₄COR₁₄,
(D)-NR₁₄CON(R₁₄)₂,
(D)-NR₁₄C(O)OR₁₄, oder
(D)-NR₁₄SO₂R₁₄;
R₁₄ bedeutet unabhängig:
Wasserstoff,
Halogen,
C₁-C₈-Alkyl, geradkettig oder verzweigt,
C₁-C₈-Alkoxy, geradkettig oder verzweigt, oder
Phenyl;
X bedeutet:
C₁-C₈-Alkyl, geradkettig oder verzweigt,
(D)-Cycloalkyl,
(D)-Heterocyclyl,
(D)-NHC(O)R₉, oder
(D)-CON(R₉R₉);
Y bedeutet:
Wasserstoff,
C₁-C₈-Alkyl, geradkettig oder verzweigt,
(D)-Cycloalkyl, oder
(D)-Heterocyclyl;
Cy bedeutet Benzol;
D bedeutet eine Bindung oder CH₂;
E bedeutet NSO₂R₁₀;
G bedeutet D;
J bedeutet N oder CH;
n bedeutet 0;
m bedeutet 0;
o bedeutet 0 oder 1;
p bedeutet 0, 1 oder 2;
q bedeutet 0 oder 1;
r bedeutet 1.

4. Verbindung gemäß einem der Ansprüche 1 bis 3 zur Verwendung als ein Arzneimittel.

5. Verwendung der Verbindung gemäß einem der Ansprüche 1 bis 3 für die Herstellung eines Arzneimittels zur Behandlung oder Vorbeugung von Störungen, Krankheiten oder Bedingungen, die auf die Modulierung des Melanocortin-4-Rezeptors in einem Säuger reagieren, wobei Modulierung Aktivierung im Falle von MC4-R Agonisten bedeutet oder Inaktivierung im Falle von MC4-R Antagonisten bedeutet.

6. Verwendung von MC4-R Antagonisten gemäß Anspruch 5 für die Herstellung eines Arzneimittels zur Behandlung oder Vorbeugung von Krebs-Kachexie.

7. Verwendung von MC4-R Antagonisten gemäß Anspruch 5 für die Herstellung eines Arzneimittels zur Behandlung oder Vorbeugung von Muskelschwund.

8. Verwendung von MC4-R Antagonisten gemäß Anspruch 5 für die Herstellung eines Arzneimittels zur Behandlung oder Vorbeugung von Anorexie.

9. Verwendung von MC4-R Antagonisten gemäß Anspruch 5 für die Herstellung eines Arzneimittels zur Behandlung oder Vorbeugung von Angstzuständen und/oder Depressionen.

10. Verwendung von MC4-R Agonisten gemäß Anspruch 5 für die Herstellung eines Arzneimittels zur Behandlung oder Vorbeugung von Fettleibigkeit.

11. Verwendung von MC4-R Agonisten gemäß Anspruch 5 für die Herstellung eines Arzneimittels zur Behandlung oder Vorbeugung von Diabetes mellitus.

12. Verwendung von MC4-R Agonisten gemäß Anspruch 5 für die Herstellung eines Arzneimittels zur Behandlung oder Vorbeugung von männlicher oder weiblicher sexueller Dysfunktion.

13. Verwendung von MC4-R Agonisten gemäß Anspruch 5 für die Herstellung eines Arzneimittels zur Behandlung oder Vorbeugung von erektiler Dysfunktion.

14. Eine pharmazeutische Zusammensetzung, welche eine Verbindung gemäß einem der Ansprüche 1 bis 3 und einen pharmazeutisch akzeptablen Träger umfasst.

## Revendications

1. Composé de formule structurale (I) : 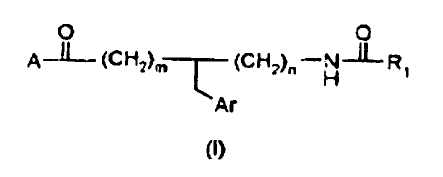 ou un sel pharmaceutiquement acceptable ou un solvate de celui-ci, dans lequel
Ar est :
un aryle ou un hétéroaryle qui peuvent tous deux être substitués par un à trois substituants indépendamment choisis dans le groupe constitué par un cyano, nitro, perfluoroalcoxy en C₁-C₈, halo, alkyle en C₁-C₈, (D)-cycloalkyle, alcoxy en C₁-C₈ ou haloalkyle en C₁-C₈ ;
R₁ est : 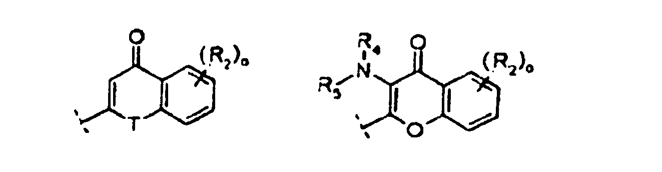
A est : 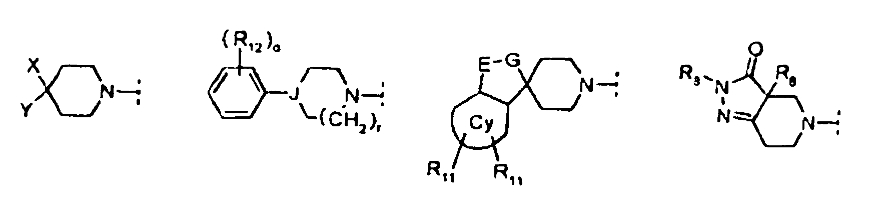
R₂ est indépendamment :
un hydrogène,
un halo,
un alkyle en C₁-C₈, à chaîne linéaire ou ramifié,
un haloalkyle en C₁-C₈, à chaîne linéaire ou ramifié,
un hydroxy
un alcoxy en C₁-C₈, à chaîne linéaire ou ramifié,
un S-alkyle en C₁-C₈, à chaîne linéaire ou ramifié, un SO₂-alkyle en C₁-C₈, à chaîne linéaire ou ramifié,
un O-alcényle en C₂-C₈, à chaîne linéaire ou ramifié,
un S-alcényle en C₂-C₈, à chaîne linéaire ou ramifié,
NR₁₄C(O)R₁₄,
NR₁₄SO₂R₁₄,
N(R₁₄)₂,
un (D)-cycloalkyle,
un (D)-aryle,
un (D)-hétéroaryle,
un (D)-hétérocyclyle (où hétérocyclyle exclut un hétérocyclyle contenant un seul azote), et
dans lequel aryle, hétéroaryle, hétérocyclyle, alkyle et cycloalkyle sont non substitués ou substitués par un à trois substituants choisis dans le groupe constitué par un oxo, halo, alkyle en C₁-C₈, N(R₆)₂, OR₆, SR₆ et CO₂R₆,
et deux R₂ adjacents peuvent former un anneau de 4 à 7 chaînons ;
R₄ et R₅ sont chacun indépendamment :
un hydrogène,
un alkyle en C₁-C₈, à chaîne linéaire ou ramifié ou
un (D)-cycloalkyle, ou
R₄ et R₅ conjointement avec l'azote auquel ils sont liés forment un anneau de 5 à 8 chaînons,
dans lesquels alkyle et cycloalkyle sont non substitués ou substitués par un à trois substituants choisis parmi R₇ et oxo ;
R₆ est indépendamment :
un hydrogène,
un alkyle en C₁-C₈, à chaîne linéaire ou ramifié,
un C(O)-alkyle en C₁-C₈, à chaîne linéaire ou ramifié,
un (D)-aryle ou
un (D)-cycloalkyle;
R₇ est indépendamment:
un alkyle en C₁-C₈, à chaîne linéaire ou ramifié,
un (D)-aryle,
un (D)-cycloalkyle,
un (D)-hétéroaryle,
un halo,
OR₈,
NHSO₂R₈,
N(R₈)₂,
CN,
CO₂R₄,
C(R₈)(R₈)N(R₈)₂,
un nitro,
SO₂N(R₈)₂,
S(O)ₚR₈,
CF₃ ou
OCF₃ ;
R₈ est indépendamment :
un hydrogène,
un alkyle en C₁-C₈, à chaîne linéaire ou ramifié,
un (D)-aryle ou
un (D)-cycloalkyle ;
R₉ est indépendamment :
un hydrogène,
un alkyle en C₁-C₈, à chaîne linéaire ou ramifié,
un (D)-aryle,
un (D)-hétéroaryle ou
un (D)-cycloalkyle ;
R₁₀ est indépendamment :
R₉,
un (D)-hétérocyclyle,
un (D)-N(Y)₂,
un (D)-NH-hétéroaryle ou
un (D)-NH-hétérocyclyle,
dans lequel aryle et hétéroaryle sont non substitués ou substitués par un à trois R₇, et alkyle, D, cycloalkyle et hétérocyclyle sont non substitués ou substitués par un à quatre substituants indépendamment choisis parmi R₇ et oxo, ou deux groupes R₁₀ conjointement avec les atomes auxquels ils sont liés forment un système d'anneau mono- ou bicyclique de 5 à 8 chaînons ;
R₁₁ est :
un hydrogène,
un halo,
un alkyle en C₁-C₈, à chaîne linéaire ou ramifié,
un alcoxy en C₁-C₈, à chaîne linéaire ou ramifié,
C≡N,
CF₃ ou
OCF₃ ;
R₁₂ est indépendamment :
un hydrogène,
un hydroxy,
un cyano,
un nitro,
un halo,
un alkyle en C₁-C₈, à chaîne linéaire ou ramifié,
un alcoxy en C₁-C₈, à chaîne linéaire ou ramifié,
un haloalkyle en C₁-C₈, à chaîne linéaire ou ramifié,
(D)-C(O)R₁₄,
(D)-C(O)OR₁₄,
(D)-C(O)SR₁₄,
un (D)-C(O)-hétéroaryle,
un (D)-C(O)-hétérocyclyle,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂,
(D)-NR₁₄SO₂R₁₄,
(D)-NR₁₄SO₂N(R₁₄)₂,
un (D)-NR₁₄(D)-hétérocyclyle,
un (D)-NR₁₄(D)-hétéroaryle,
(D)-OR₁₄,
OSO₂R₁₄,
un (D)-[O]_{q}(cycloalkyle),
un (D)-[O]_{q}(D)aryle,
un (D)-[O]_{q}(D)-hétéroaryle,
un (D)-[O]_{q}(D)-hétérocyclyle (où l'hétérocyclyle exclut un hétérocyclyle contenant un seul azote lorsque q = 1),
(D)-SR₁₄,
(D)-SOR₁₄,
(D)-SO₂R₁₄ ou
(D)-SO₂N(R₁₄)₂,
dans lequel alkyle, alcoxy, cycloalkyle, aryle, hétérocyclyle et hétéroaryle sont non substitués ou substitués par un à cinq substituants indépendamment choisis parmi R₁₃;
R₁₃ est indépendamment:
un hydrogène,
un halo,
un oxo,
N(R₁₅)₂,
un alkyle en C₁-C₈, à chaîne linéaire ou ramifié,
un (D)-cycloalkyle,
un haloalkyle en C₁-C₈, à chaîne linéaire ou ramifié,
un alcoxy en C₁-C₈, à chaîne linéaire ou ramifié,
un hétéroaryle,
un hydroxy,
un hétérocyclyle (où l'hétérocyclyle exclut un hétérocyclyle contenant un seul azote),
un phényle,
(D)-C(O)R₁₄,
(D)-C(O)OR₁₄,
(D)-OR₁₄,
(D)-OCOR₁₄,
(D)-OCO₂R₁₄,
(D)-SR₁₄,
(D)-SOR₁₄ ou
(D)-SO₂R₁₄,
dans lequel aryle, hétéroaryle, hétérocyclyle, alkyle ou cycloalkyle sont non substitués ou substitués par un à trois substituants choisis dans le groupe constitué par un oxo, alkyle en C₁-C₈, N(R₁₅)₂, OR₁₅, SR₁₅ et CO₂R₁₅ ;
R₁₄ est indépendamment :
un hydrogène,
un alkyle en C₁-C₈, à chaîne linéaire ou ramifié,
un haloalkyle en C₁-C₈, à chaîne linéaire ou ramifié,
un (D)-cycloalkyle,
un (D)-phényle,
un (D)-naphtyle,
un (D)-hétéroaryle,
un (D)-hétérocyclyle (où l'hétérocyclyle exclut un hétérocyclyle contenant un seul azote), et
dans lequel phényle, naphtyle, hétéroaryle, hétérocyclyle, alkyle et cycloalkyle sont non substitués ou substitués par un à trois substituants choisis dans le groupe constitué par un oxo, haloalkyle en C₁-C₈, N(R₁₅)₂, OR₁₅, SR₁₅ et CO₂R₁₅ ;
R₁₅ est indépendamment :
un hydrogène,
un alkyle en C₁-C₈, à chaîne linéaire ou ramifié,
C(O)-alkyle en C₁-C₈, à chaîne linéaire ou ramifié, un aryle ou
un cycloalkyle ;
X est :
un alkyle en C₁-C₈, à chaîne linéaire ou ramifié,
un (D)-cycloalkyle,
un (D)-aryle,
un (D)-hétéroaryle,
un (D)-hétérocyclyle,
(D)-C≡N,
(D)-CON(R₉R₉),
(D)-CO₂R₉,
(D)-COR₉,
(D)-NR₉C(O)R₉,
(D)-NR₉CO₂R₉,
(D)-NR₉C(O)N(R₉)₂,
(D)-NR₉SO₂R₉,
(D)-S(O)ₚR₉,
(D)-SO₂N(R₉)(R₉),
(D)-OR₉,
(D)-OC(O)R₉,
(D)-OC(O)OR₉,
(D)-OC(O)N(R₉)₂,
(D)-N(R₉)(R₉) ou
(D)-NR₉SO₂N(R₉)(R₉),
dans lequel aryle et hétéroaryle sont non substitués ou substitués par un à trois groupes choisis parmi R₇ et
alkyle, D, cycloalkyle et hétérocyclyle sont non substitués ou substitués par un à quatre groupes indépendamment choisis parmi R₇ et oxo ;
Y est:
un hydrogène,
un alkyle en C₁-C₈, à chaîne linéaire ou ramifié,
un (D)-cycloalkyle,
un (D)-aryle,
un (D)-hétérocyclyle ou
un (D)-hétéroaryle,
dans lequel aryle et hétéroaryle sont non substitués ou substitués par un à trois groupes choisis parmi R₇ et
alkyle, D et cycloalkyle sont non substitués ou substitués par un à trois groupes indépendamment choisis parmi R₇ et oxo ;
Cy est un benzène, pyridine ou cyclohexane ;
D est une liaison ou un alkylène en C₁-C₈, à chaîne linéaire ou ramifié,
E est CHCO₂Y, CHC(O)N(Y)₂, NSO₂R₁₀, CHN(Y)COR₁₀, CHN(Y)SO₂R₁₀, CHCH₂OY ou CHCH₂hétéroaryle ;
G est D, CH-alkyle, O, C=O ou SO₂, à condition que lorsque G est 0, l'atome E de l'anneau est un carbone ;
J est N ou CH ;
T est O ou NR₄ ;
n est 0-2 ;
m est 0-2 ;
o est 0-3 ;
p est 0-2 ;
q est 0 ou 1 ;
r est 1 ou 2 et
dans lequel
un aryle est un groupe caractéristique mono- ou polycyclique aromatique ayant 6 à 20 atomes de carbone,
un hétéroaryle est un groupe caractéristique aromatique ayant 6 à 20 atomes de carbone ayant au moins un hétérocycle ;
un hétérocyclyle est un anneau saturé, insaturé ou aromatique contenant au moins un hétéroatome choisi parmi O, N et/ou S et 1 à 6 atomes de carbone ; et
un cycloalkyle est un anneau alkyle ayant 3 à 8 atomes de carbone.

2. Composé selon la revendication 1, dans lequel
Ar est :
un aryle qui peut être substitué par un à trois substituants indépendamment choisis dans le groupe constitué par un cyano, nitro, perfluoroalcoxy, halo, alkyle en C₁-C₈, à chaîne linéaire ou ramifié, (D)-cycloalkyle, alcoxy en C₁-C₈, à chaîne linéaire ou ramifié, et/ou haloalkyle en C₁-C₈, à chaîne linéaire ou ramifié ;
R₁ est : 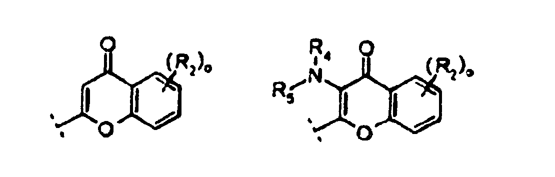
A est: 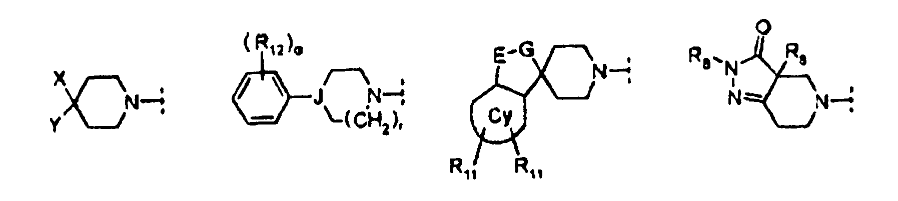
R₂ est indépendamment :
un hydrogène,
un hydroxy,
un halo,
un alkyle en C₁-C₈, à chaîne linéaire ou ramifié,
un alcoxy en C₁-C₈, à chaîne linéaire ou ramifié,
un S-alkyle en C₁-C₈, à chaîne linéaire ou ramifié,
un SO₂-alkyle en C₁-C₈, à chaîne linéaire ou ramifié,
un O-alcényle en C₂-C₈, à chaîne linéaire ou ramifié,
un S-alcényle en C₂-C₈, à chaîne linéaire ou ramifié,
un haloalkyle en C₁₋₈, à chaîne linéaire ou ramifié ou
un (D)-cycloalkyle ;
R₄ et R₅ sont chacun indépendamment :
un hydrogène,
un alkyle en C₁-C₈, à chaîne linéaire ou ramifié ou
un cycloalkyle, ou
R₄ et R₅ conjointement avec l'azote auquel ils sont liés forment un anneau de 5 à 7 chaînons qui peut contenir un hétéroatome supplémentaire choisi parmi O, S et NR₆ ;
R₆ est indépendamment :
un hydrogène,
un alkyle en C₁-C₈, à chaîne linéaire ou ramifié,
un C(O)-alkyle en C₁-C₈, à chaîne linéaire ou ramifié,
un (D)-aryle ou
un (D)-cycloalkyle ;
R₈ est indépendamment:
un hydrogène,
un alkyle en C₁-C₈, à chaîne linéaire ou ramifié,
un (D)-aryle ;
R₉ est indépendamment :
un hydrogène,
un alkyle en C₁-C₈, à chaîne linéaire ou ramifié, ou
un (D)-cycloalkyle ;
R₁₀ est R₉;
R₁₁ est:
un hydrogène,
un halo,
un alkyle en C₁-C₈, à chaîne linéaire ou ramifié,
un alcoxy en C₁-C₈, à chaîne linéaire ou ramifié, ou
C≡N ;
R₁₂ est indépendamment :
un hydrogène,
un hydroxy,
un cyano,
un nitro,
un halo,
un alkyle en C₁-C₈, à chaîne linéaire ou ramifié,
un alcoxy en C₁-C₈, à chaîne linéaire ou ramifié,
un haloalkyle en C₁-C₈, à chaîne linéaire ou ramifié,
un (D)-C(O)-hétérocyclyle,
(D)-C(O)N(R₁₄)₂,
(D)-N(R₁₄)₂,
(D)-NR₁₄COR₁₄,
(D)-NR₁₄CON(R₁₄)₂,
(D)-NR₁₄C(O)OR₁₄,
(D)-NR₁₄C(R₁₄)=N(R₁₄),
(D)-NR₁₄C(=NR₁₄)N(R₁₄)₂,
(D)-NR₁₄SO₂R₁₄ ou
(D)-NR₁₄SO₂N(R₁₄)₂ ;
R₁₄ est indépendamment :
un hydrogène,
un halo,
un alkyle en C₁-C₈, à chaîne linéaire ou ramifié,
un (D)-cycloalkyle,
un alcoxy en C₁-C₈, à chaîne linéaire ou ramifié ou
un phényle ;
X est:
un alkyle en C₁-C₈, à chaîne linéaire ou ramifié,
un (D)-cycloalkyle,
un (D)-aryle,
un (D)-hétéroaryle,
un (D)-hétérocyclyle,
(D)-NHC(O)R₉,
(D)-CO₂R₉ ou
(D)-CON(R₉R₉) ;
Y est :
un hydrogène,
un alkyle en C₁-C₈, à chaîne linéaire ou ramifié,
un (D)-cycloalkyle,
un (D)-aryle,
un (D)-hétérocyclyle ou
un (D)-hétéroaryle ;
Cy est un benzène ou pyridine ;
D est une liaison ou un alkylène en C₁-C₄ ;
E est NSO₂R₁₀, CHN(Y)COR₁₀ ou CHN(Y)SO₂R₁₀ ;
G est D ou CH-alkyle ;
J est N ou CH ;
n est 0 ou 1 ;
m est 0 ou 1 ;
o est 0, 1 ou 2 ;
r est 1.

3. Composé selon la revendication 1 ou 2, dans lequel
Ar est :
un phényle ou naphtyle qui peut être substitué par un ou deux substituants indépendamment choisis dans le groupe constitué par halo, alkyle en C₁-C₈, alcoxy en C₁-C₈ et/ou haloalkyle en C₁-C₈ ;
R₁ est : 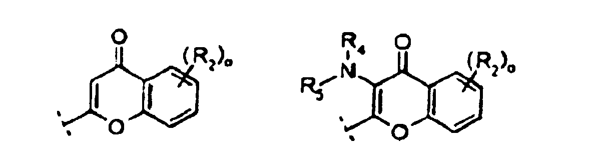
A est : 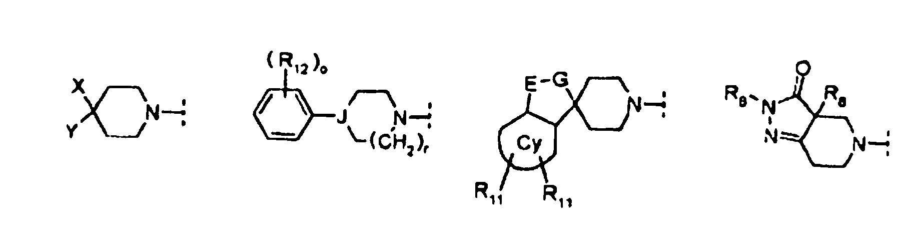
R₂ est indépendamment :
un hydrogène,
un hydroxy,
un alcoxy en C₁-C₈, à chaîne linéaire ou ramifié,
un S-alkyle en C₁-C₈, à chaîne linéaire ou ramifié,
un SO₂-alkyle en C₁-C₈, à chaîne linéaire ou ramifié,
un O-alcényle en C₂-C₈, à chaîne linéaire ou ramifié,
un S-alcényle en C₂-C₈, à chaîne linéaire ou ramifié,
un halo ou
un alkyle en C₁-C₈, à chaîne linéaire ou ramifié ;
R₄ et R₅ sont chacun indépendamment :
un hydrogène ou
un alkyle en C₁-C₈, à chaîne linéaire ou ramifié, ou
R₄ et R₅ conjointement avec l'azote auquel ils sont liés forment un anneau de 5 à 6 chaînons contenant facultativement un atome d'oxygène supplémentaire ;
R₆ est un hydrogène ;
R₈ est indépendamment :
un alkyle en C₁-C₈, à chaîne linéaire ou ramifié ou
un (D)-aryle ;
R₉ est un alkyle en C₁-C₈, à chaîne linéaire ou ramifié ;
R₁₀ est R₉;
R₁₁ est:
un hydrogène,
un halo et
un alkyle en C₁-C₄;
R₁₂ est indépendamment :
un cyano,
un nitro,
un halo,
un alkyle en C₁-C₈, à chaîne linéaire ou ramifié,
un (D)-C(O)-hétérocyclyle,
(D)-N(R₁₄)₂,
(D)-NR₁₄COR₁₄,
(D)-NR₁₄CON(R₁₄)₂,
(D)-NR₁₄C(O)OR₁₄ ou
(D)-NR₁₄SO₂R₁₄ ;
R₁₄ est indépendamment :
un hydrogène,
un halo,
un alkyle en C₁-C₈, à chaîne linéaire ou ramifié,
un alcoxy en C₁-C₈, à chaîne linéaire ou ramifié ou
un phényle ;
X est :
un alkyle en C₁-C₈, à chaîne linéaire ou ramifié,
un (D)-cycloalkyle,
un (D)-hétérocyclyle,
(D)-NHC(O)R₉ ou
(D)-CON(R₉R₉) ;
Y est :
un hydrogène,
un alkyle en C₁-C₈, à chaîne linéaire ou ramifié,
un (D)-cycloalkyle ou
un (D)-hétérocyclyle ;
Cy est un benzène ;
D est une liaison ou CH₂ ;
E est NSO₂R₁₀ ;
G est D ;
J est N ou CH ;
n est 0 ;
m est 0 ;
o est 0 ou 1 ;
p est 0, 1 ou 2 ;
q est 0 ou 1 ;
r est 1.

4. Composé selon l'une quelconque des revendications 1 à 3 pour une utilisation en tant que médicament.

5. Utilisation du composé selon l'une quelconque des revendications 1 à 3 pour la préparation d'un médicament pour le traitement ou la prévention de troubles, maladies ou conditions sensibles à la modulation du récepteur de la mélanocortine-4 chez un mammifère, où la modulation désigne l'activation dans le cas d'agonistes du MC4-R ou l'inactivation dans le cas d'antagonistes du MC4-R.

6. Utilisation d'antagonistes du MC4-R selon la revendication 5 pour la préparation d'un médicament pour le traitement ou la prévention de la cachexie cancéreuse.

7. Utilisation d'antagonistes du MC4-R selon la revendication 5 pour la préparation d'un médicament pour le traitement ou la prévention de l'émaciation musculaire.

8. Utilisation d'antagonistes du MC4-R selon la revendication 5 pour la préparation d'un médicament pour le traitement ou la prévention de l'anorexie.

9. Utilisation d'antagonistes du MC4-R selon la revendication 5 pour la préparation d'un médicament pour le traitement ou la prévention de l'anxiété et/ou de la dépression.

10. Utilisation d'agonistes du MC4-R selon la revendication 5 pour la préparation d'un médicament pour le traitement ou la prévention de l'obésité.

11. Utilisation d'agonistes du MC4-R selon la revendication 5 pour la préparation d'un médicament pour le traitement ou la prévention du diabète sucré.

12. Utilisation d'agonistes du MC4-R selon la revendication 5 pour la préparation d'un médicament pour le traitement ou la prévention du dysfonctionnement sexuel chez l'homme ou la femme.

13. Utilisation d'agonistes du MC4-R selon la revendication 5 pour la préparation d'un médicament pour le traitement ou la prévention du dysfonctionnement érectile.

14. Composition pharmaceutique qui comprend un composé selon l'une quelconque des revendications 1 à 3 et un véhicule pharmaceutiquement acceptable.
